# EUROPEAN PATENT SPECIFICATION

(11) **EP 2 082 065 B1**
(45) Date of publication and mention of the grant of the patent: **12.03.2014**
(21) Application number: 08769927.8
(22) Date of filing: 30.05.2008
(51) Int. Cl.: C12Q 1/68

(54) **MULTIGENE PROGNOSTIC ASSAY FOR LUNG CANCER**
PROGNOSTISCHES MULTIGEN-TESTVERFAHREN FÜR LUNGENKREBS
TEST PRONOSTIQUE MULTIGÉNIQUE POUR LE CANCER DU POUMON

(30) Priority: 01.06.2007 US 941550 P
(43) Date of publication of application: 29.07.2009
(60) Divisional application: 14153513.8
(73) Proprietor: The Regents of the University of California, Oakland, CA 94607-5200 (US)
(72) Inventor: JABLONS, David M., San Francisco, California 94107 (US); RAZ, Dan J., San Francisco, California 94131 (US)
(74) Representative: Campbell, Patrick John Henry
(86) International application number: PCT/US2008/065409
(87) International publication number: WO 2008/151072

(56) References cited:
- WO-A2-2006/110581
- WO-A2-2006/110581
- US-A1- 2004 170 977
- US-A1- 2007 105 133
- RAPONI MITCH ET AL: "Gene expression signatures for predicting prognosis of squamous cell and adenocarcinomas of the lung." CANCER RESEARCH, vol. 66, no. 15, 1 August 2006 (2006-08-01), pages 7466-7472, XP002607461 ISSN: 0008-5472 -& RAPONI MITCH ET AL: "Supplementary Information: Gene expression signatures for predicting prognosis of squamous cell and adenocarcinomas of the lung." CANCER RESEARCH, vol. 66, no. 15, 2 August 2006 (2006-08-02), page 17PP, XP002610888
- SAFAR A MAZIN ET AL: "Methylation profiling of archived non-small cell lung cancer: a promising prognostic system." CLINICAL CANCER RESEARCH : AN OFFICIAL JOURNAL OF THE AMERICAN ASSOCIATION FOR CANCER RESEARCH, vol. 11, no. 12, 15 June 2005 (2005-06-15), pages 4400-4405, XP002607462 ISSN: 1078-0432
- CHEN HSUAN-YU ET AL: "A five-gene signature and clinical outcome in non-small-cell lung cancer" NEW ENGLAND JOURNAL OF MEDICINE, MASSACHUSETTS MEDICAL SOCIETY, BOSTON, MA, US LNKD- DOI:10.1056/NEJMOA060096, vol. 356, no. 1, 4 January 2007 (2007-01-04), pages 11-20, XP009086044 ISSN: 1533-4406
- TAZI N ET AL: "[Choroidal metastasis revealing pulmonary adenocarcinoma]" LA REVUE DE MÉDECINE INTERNE / FONDÉE ... PAR LA SOCIÉTÉ NATIONALE FRANCAISE DE MÉDECINE INTERNE SEP 2006 LNKD- PUBMED:16872723, vol. 27, no. 9, September 2006 (2006-09), pages 699-701, XP002607463 ISSN: 0248-8663
- "Affymetrix Genechip bHuman Genome U133 plus 2.0 Array" GEO, 7 November 2003 (2003-11-07), XP002343693
- YI E S ET AL: "High c-erbB-3 protein expression is associated with shorter survival in advanced non-small cell lung carcinomas." MODERN PATHOLOGY : AN OFFICIAL JOURNAL OF THE UNITED STATES AND CANADIAN ACADEMY OF PATHOLOGY, INC, vol. 10, no. 2, February 1997 (1997-02), pages 142-148, XP009140617 ISSN: 0893-3952

## Description

### BACKGROUND OF THE INVENTION

The likelihood of long-term mortality for patients with lung cancer is poorly defined by clinical stage and histopathological findings. Our hypothesis was that a multigene quantitative polymerase chain reaction (PCR) assay can predict risk of mortality among patients with lung cancer.

### BRIEF SUMMARY OF THE INVENTION

In one aspect, the present invention provides a method of providing a prognosis for lung cancer in a subject, the method comprising the steps of:
(a) contacting a biological sample from the subject with reagents that each specifically bind to one member of a panel of mRNA biomarkers comprising the eight biomarkers ctnnb1, wnt3a, tp53, kras, erbb3, muc1, erbb2, and dusp6, and
(b) calculating a model score for said subject using the expression levels of the eight biomarkers and model coefficients, thereby identifying said subject as high-risk or low-risk based on the model score.

In another aspect, the present invention provides a method of providing a prognosis of a subject having a lung cancer, the method comprising quantifying in a biological sample derived from said subject the mRNA expression levels of at least four biomarkers comprising rnd3, wnt3a. crbb3, and lck; and calculating a model score for said subject using said expression levels and model coefficients, thereby identifying said subject as high-risk or low-risk based on the model score.

In one embodiment, the reagent is a nucleic acid. In another embodiment, the reagent is an oligonucleotide. In another embodiment, the reagent is an PCR primer set.

In one embodiment, the sample is from a surgically resected tumor. In another embodiment, the sample is from a lung tissue or lung tumor biopsy.

Disclosed herein is a kit comprising reagents that specifically bind to a panel of biomarkers comprising ctnnb1, wnt3a, tp53, kras, erbb3, muc1, erbb2, and dusp. The reagent may be a PCR primer set.

Disclosed herein is a method of determining the prognosis of a subject having a lung cancer by quantifying in a biological sample the expression levels of at least two (e.g., at least three or at least four) genes selected from a group of: Rnd3, wnt3a, erbb3, lck, sh3bgr, fut3, il11, cdc6, cdk2ap1, bag1, emx2, six3, and brca1. In this aspect, the biological sample (e.g., a tumor biopsy, a lung biopsy, and a blood sample) is derived from the subject and the expression levels are indicative of the prognosis.

In any of the forgoing aspects, the expression levels can be mRNA expression levels or protein levels, or combination of both. The invention features determining mRNA expression levels through, for example, quantitative rtPCR.

Disclosed herein is a method of determining the prognosis of a subject having a lung cancer by measuring in a biological sample the methylation levels of at least two (e.g., at least three or at least four) genes selected from a group consisting of: Rnd3, wnt3a, erbb3, lck, sh3bgr, fut3, il11, cdc6, cdk2ap1, bag1, emx2, six3, and brca1. In this aspect, the biological sample (e.g., a tumor biopsy, a lung biopsy, and a blood sample) is derived from the subject and the methylation levels are indicative of the prognosis.

In any of the forgoing aspects, the lung cancer can be lung adencarcinoma, e.g., stage I, stage II, stage III, or stage IV.

Also, in any of the forgoing aspects, the prognosis provides a high or low risk assessment for long term mortality.

### BRIEF DESCRIPTION OF THE DRAWINGS

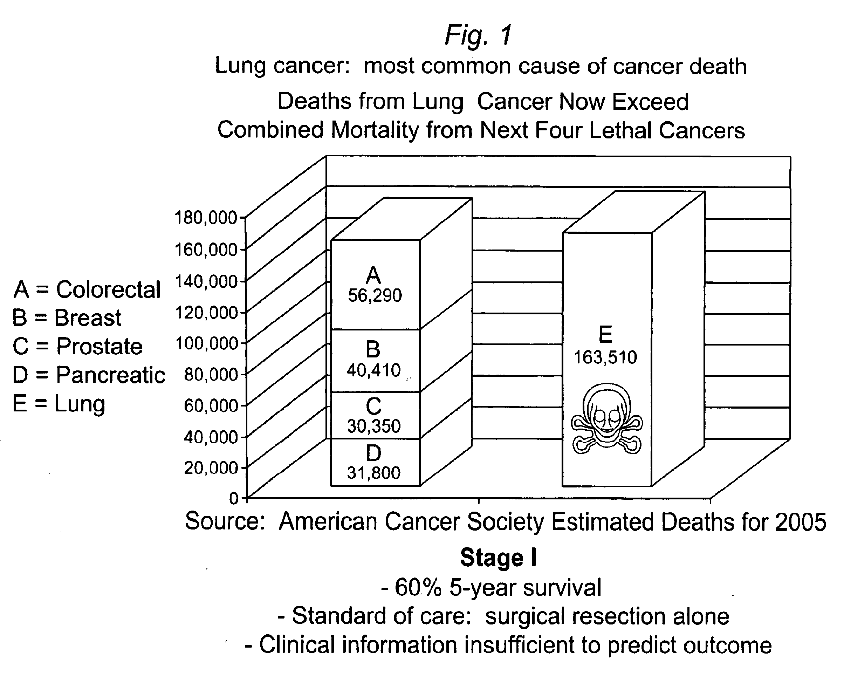

Figure 1 shows that lung cancer is the most common cause of cancer death and shows that for stage 1 cancers, the prognosis for long term mortality (five year survival) is approximately 60%.

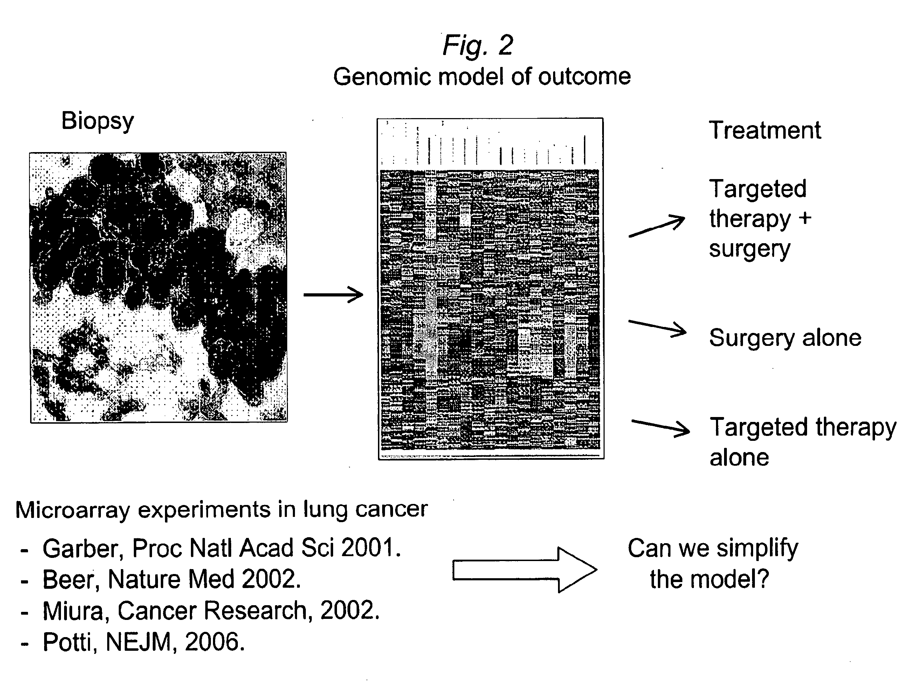

Figure 2 shows genomic models of prognosis.

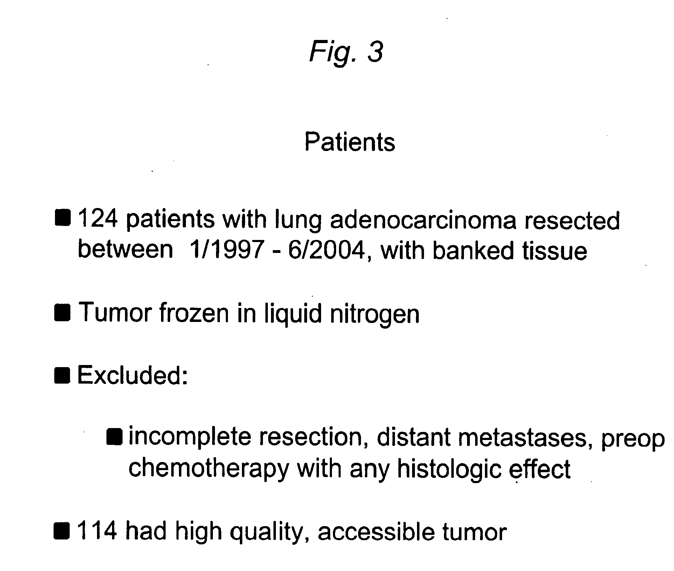

Figure 3 describes the samples used for RT PCT analysis.

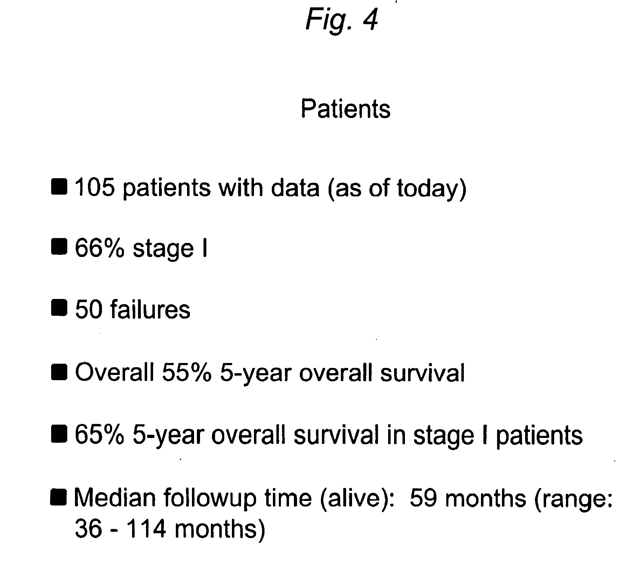

Figure 4 describes the patient population followed for prognosis.

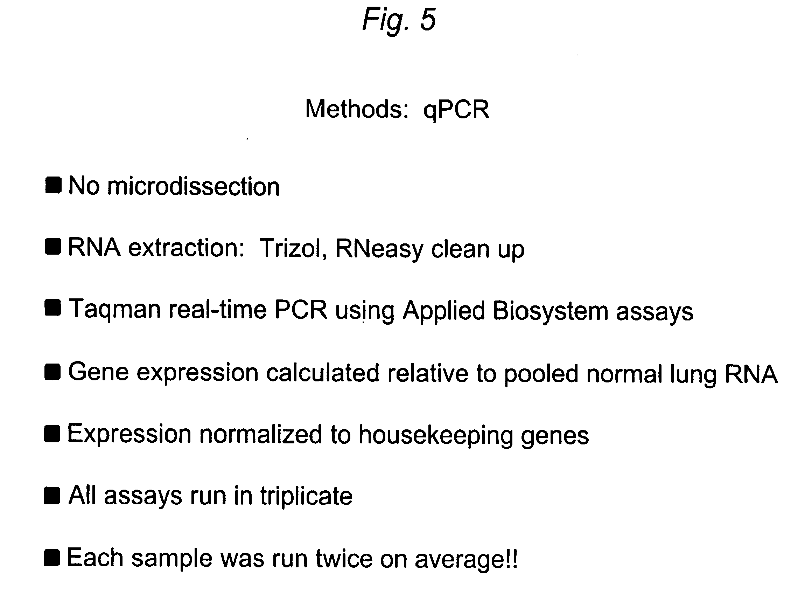

Figure 5 describes methods for quantitative (RT) PCR.

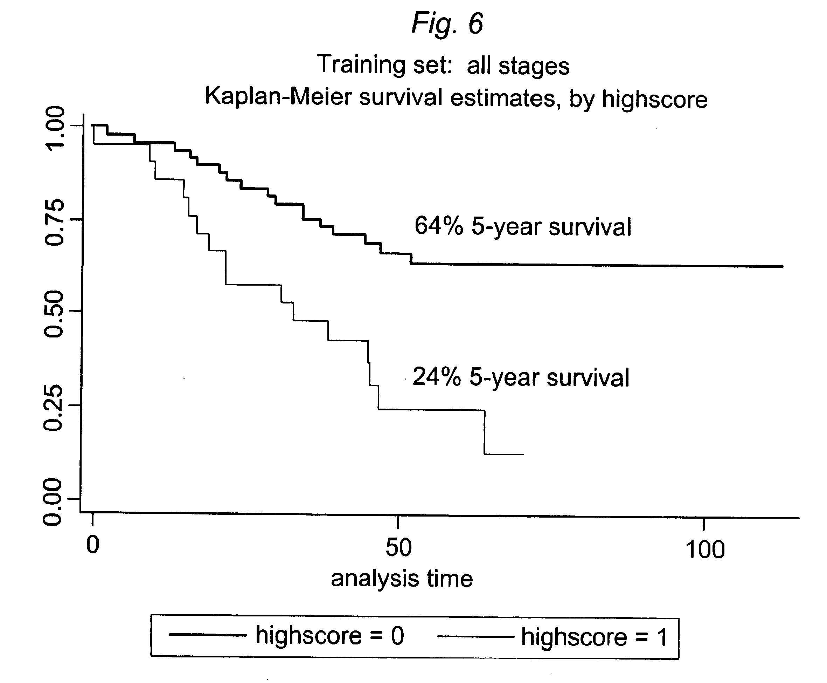

Figure 6 describes results for the training set of markers for all lung adenocarcinomas.

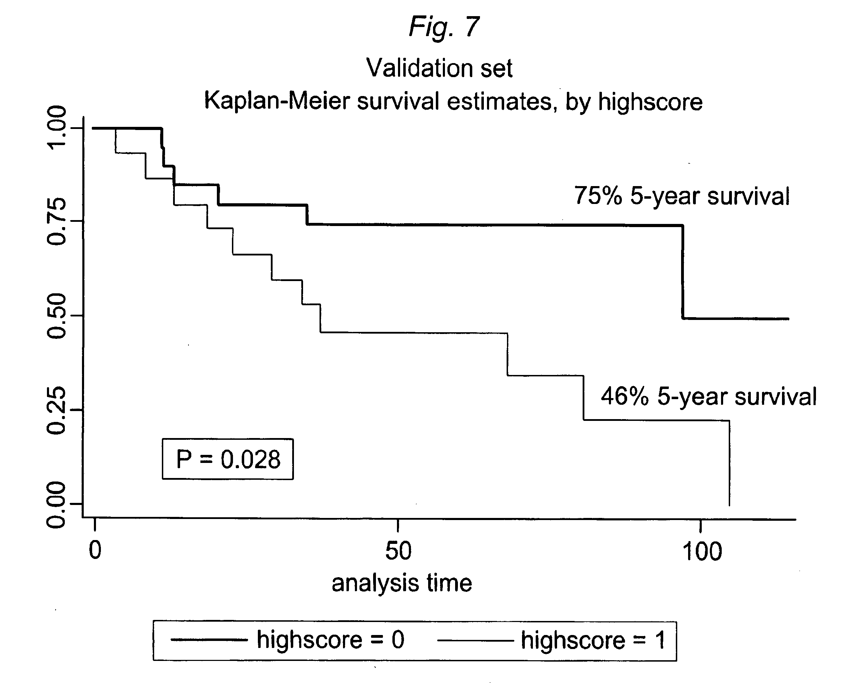

Figure 7 describes results for the validation set of markers for all lung adenocarcinomas.

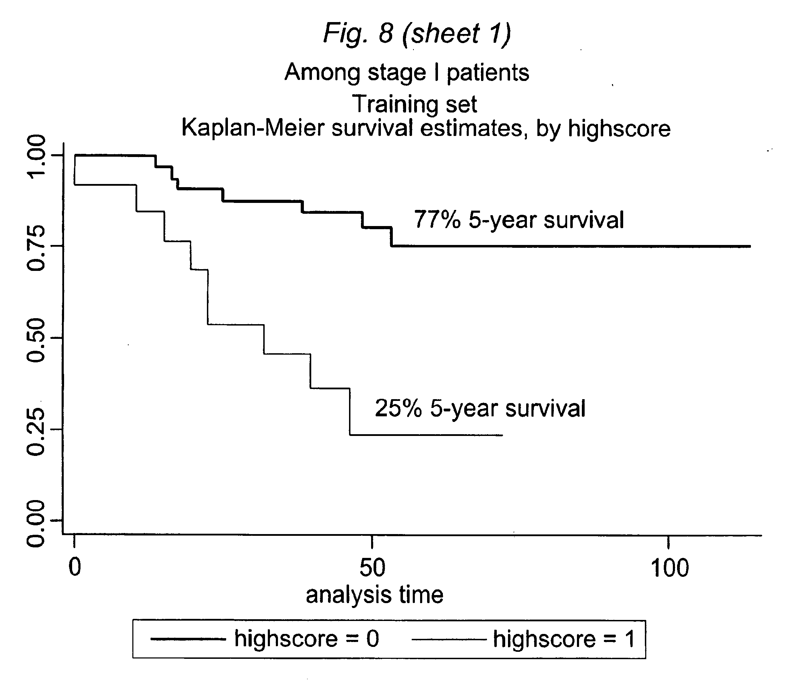

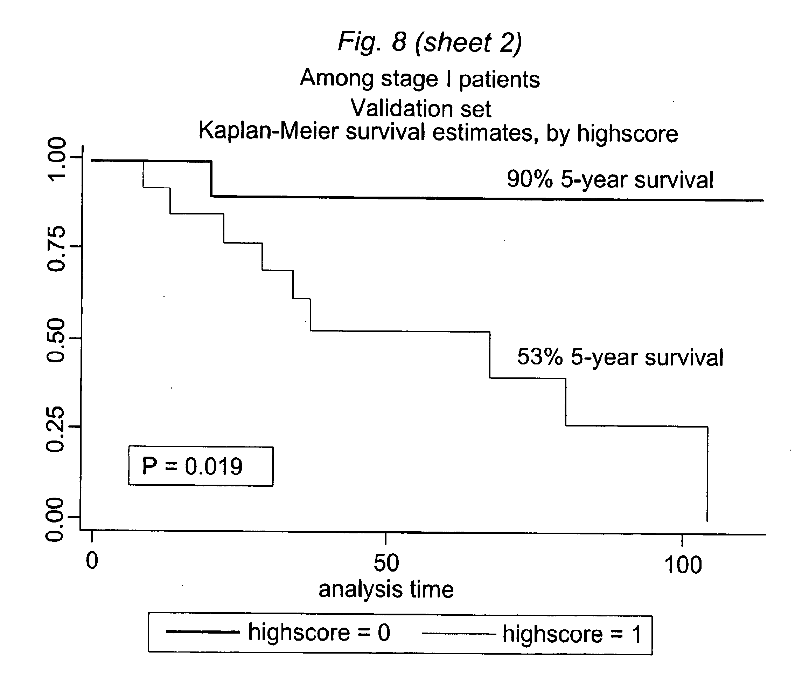

Figure 8 describes results for the validation and training set of markers for stage 1 lung adenocarcinoma.

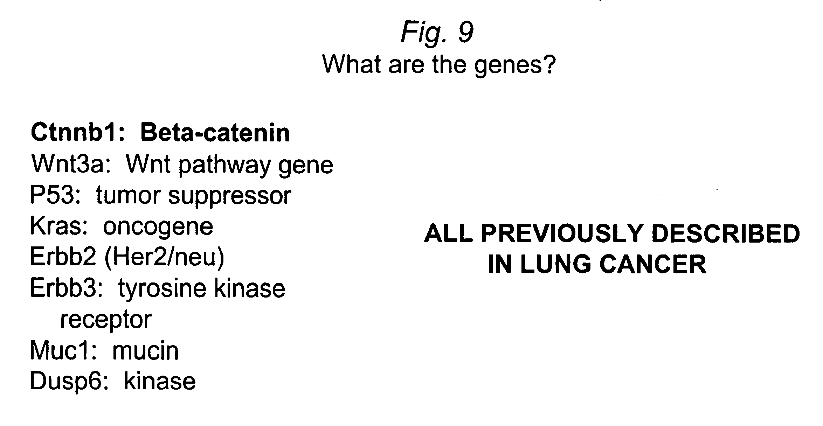

Figure 9 lists the eight genes used in the RT-PCR multigene prognostic assay for stage 1 lung adenocarcinoma.

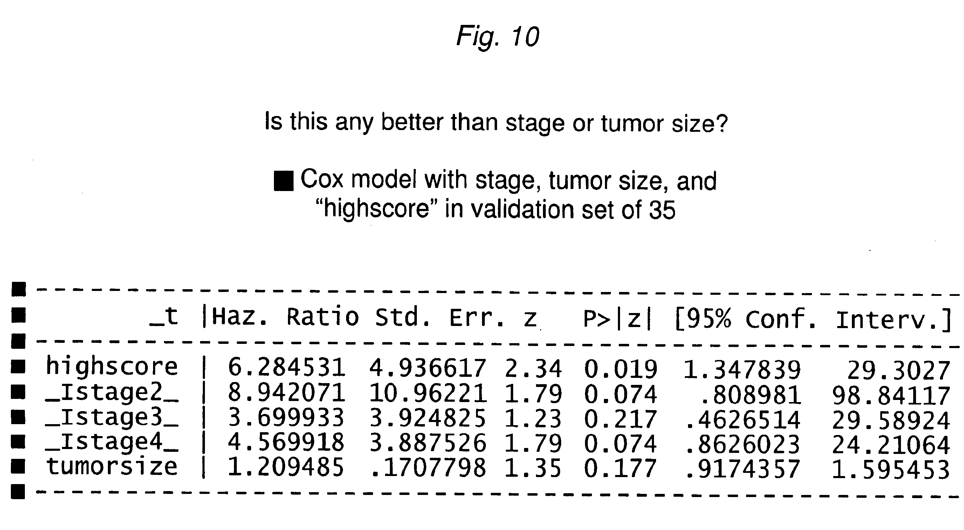

Figure 10 shows results for a Cox model with stage, tumor size and high score for the validation set.
Figure 11a is a Kaplan-Meier curves comparing overall survival among patients with low- and high-risk (all stages) using the 4-gene prognostic model.
Figure 11b is a Kaplan-Meier curves comparing disease-free survival among patients with low- and high-risk scores (all stages).
Figure 12a is a Kaplan-Meier curves comparing overall survival among stage I patients with low- and high-risk scores using the 4-gene prognostic model.
Figure 12b is a Kaplan-Meier curves comparing disease-free survival among stage I patients with low- and high-risk scores.
Figure 13 is a Kaplan-Meier curves comparing stage I patients with low- and high-risk scores using the 5-gene model published by Chen and colleagues.

### DETAILED DESCRIPTION OF THE INVENTION

### INTRODUCTION

Disclosed herein is the identification of expression profiles of certain groups of genes which allows accurate prognosis of long term mortality in early stage lung cancer. We identified 65 genes that were previously identified as prognostic for long-term mortality in early stage lung cancer in 3 published microarray studies and 2 PCR-based studies. RNA was extracted from 124 fresh-frozen tumor samples from consecutive patients with completely resected lung adenocarcinoma with at least 3 years of clinical follow-up. 80 samples were randomly assigned to a test group and the remainder assigned to a validation group. Real-time PCR of the 65 identified genes were run on the test set using Taq-man assays. A prediction model was created using a proportional hazards model of normalized gene expression levels using backwards model selection. A model score was calculated for each patient using model coefficients and individual gene expression levels. Patients were defined as high-risk if the model score was greater than the median score.

Adequate real-time PCR profiles were identified in all 80 patients. Eighteen genes were included in the final model. The proportion of patients identified as high-risk and low-risk was 52% and 48% percent, respectively. The Kaplan-Meier estimated five-year survival in the low-risk group was 82% and 5% in the high-risk group (P<0.001, log-rank test). Median survival was 22 months in the high-risk group and was not reached in the low-risk group. In multivariate survival analysis, the prognostic score predicted survival independent of tumor stage and size (P<0.001). Prognostic score predicted mortality better than clinical stage, based on model log-likelihood values (P<0.001). For clinical staging, see, e.g., Mountain, Clifton F; Herman I Libshitz, Kay E Hermes. A Handbook for Staging, Imaging, and Lymph Node Classification. Charles P Young Company, and Mountain, CF (1997). "Revisions in the international system for staging lung cancer". Chest 111: 1710-1717.

Disclosed herein is the quantification of expression of the following genes as a prognostic indicator or mortality due to lung cancers: rnd3, wnt3a, erbb3, lck, sh3bgr, fut3, il11, cdc6, cdk2ap1, bag1, emx2, six3, and brca1 (e.g., wnt3a, rnd3, lck, and erbb3) (See Example 2 below).

The eight membered multi-gene RT-PCR assay (ctnnb1, wnt3a, tp53, kras, erbb3, muc1, erbb2, and dusp6), the 13 membered multi-gene RT-PCR assay (md3, wnt3a, erbb3, lck, sh3bgr, fut3, il11, cdc6, cdk2ap1, bag1, emx2, six3, and brca1), or any multi-gene RT-PCR assay that includes wnt3a, rnd3, lck, and erbb3, aid in predicting long-term mortality among patients with lung cancer. The assays of the present invention are defined in the attached claims.

Disclosed herein is a multigene diagnostic kit, composed of the markers described herein that can be used to provide a prognosis for lung cancer patients.

### DEFINITIONS

"Lung cancer" refers generally to two main types of lung cancer categorized by the size and appearance of the malignant cells: non-small cell (80%) and small-cell (roughly 20%) lung cancer. "Non-small cell lung cancer"(NSCLC) includes squamous cell carcinoma, accounting for approximately 29% of lung cancers. Lung adenocarcinoma is the most common subtype of NSCLC, accounting for approximately 32% of lung cancers. A subtype of lung adenocarcinoma, the bronchioloalveolar carcinoma, is more common in female never-smokers. Large cell carcinoma accounts for approximately 9% of lung cancers. "Small cell lung cancer" includes SCLC (also called "oat cell carcinoma"), a less common form of lung cancer. Other types of lung cancer include carcinoid, adenoid cystic carcinoma, cylindroma, and mucoepidermoid carcinoma. In one embodiment, lung cancers are staged according to stages I-IV, with I being an early stage and IV being the most advanced.

"Prognosis" refers, e.g., to overall survival, long term mortality, and disease free survival. In one embodiment, long term mortality refers to survival 5 years after diagnosis of lung cancer. In one embodiment, the prognosis for long term mortality is "high risk," e.g., high risk of mortality, or "low risk," e.g., low risk of mortality. The stage of cancer and the prognosis may be used to tailor a patients therapy to provide a better outcome, e.g., targeted therapy and surgery, surgery alone, or targeted therapy alone.

Other forms of cancer include carcinomas, sarcomas, adenocarcinomas, lymphomas, leukemias, etc., including solid and lymphoid cancers, head and neck cancer, e.g., oral cavity, pharyngeal and tongue cancer, kidney, breast, kidney, bladder, colon, ovarian, prostate, pancreas, stomach, brain, head and neck, skin, uterine, testicular, esophagus, and liver cancer, including hepatocarcinoma, lymphoma, including non-Hodgkin's lymphomas (e.g., Burkitt's, Small Cell, and Large Cell lymphomas) and Hodgkin's lymphoma, leukemia, and multiple myeloma.

The term "marker" refers to a molecule (typically protein, nucleic acid, carbohydrate, or lipid) that is expressed in the cell, expressed on the surface of a cancer cell or secreted by a cancer cell in comparison to a non-cancer cell, and which is useful for the diagnosis of cancer, for providing a prognosis, and for preferential targeting of a pharmacological agent to the cancer cell. Oftentimes, such markers are molecules that are overexpressed in a lung cancer or other cancer cell in comparison to a non-cancer cell, for instance, 1-fold overexpression, 2-fold overexpression, 3-fold overexpression or more in comparison to a normal cell. Further, a marker can be a molecule that is inappropriately synthesized in the cancer cell, for instance, a molecule that contains deletions, additions or mutations in comparison to the molecule expressed on a normal cell. Alternatively, such biomarkers are molecules that are underexpressed in a cancer cell in comparison to a non-cancer cell, for instance, 1-fold underexpression, 2-fold underexpression, 3-fold underexpression, or more. Further, a marker can be a molecule that is inappropriately synthesized in cancer, for instance, a molecule that contains deletions, additions or mutations in comparison to the molecule expressed on a normal cell.

It will be understood by the skilled artisan that markers may be used in combination with other markers or tests for any of the uses, e.g., prediction, diagnosis, or prognosis of cancer, disclosed herein.

"Biological sample" includes sections of tissues such as biopsy and autopsy samples, and frozen sections taken for histologic purposes. Such samples include blood and blood fractions or products (e.g., serum, platelets, red blood cells, and the like), sputum, bronchoalveolar lavage, cultured cells, e.g., primary cultures, explants, and transformed cells, stool, urine, etc. A biological sample is typically obtained from a eukaryotic organism, most preferably a mammal such as a primate e.g., chimpanzee or human; cow; dog; cat; a rodent, e.g., guinea pig, rat, Mouse; rabbit; or a bird; reptile; or fish.

A "biopsy" refers to the process of removing a tissue sample for diagnostic or prognostic evaluation, and to the tissue specimen itself. Any biopsy technique known in the art can be applied to the prognostic methods of the present invention. The biopsy technique applied will depend on the tissue type to be evaluated (e.g., lung *etc.*), the size and type of the tumor, among other factors. Representative biopsy techniques include, but are not limited to, excisional biopsy, incisional biopsy, needle biopsy, surgical biopsy, and bone marrow biopsy. An "excisional biopsy" refers to the removal of an entire tumor mass with a small margin of normal tissue surrounding it. An "incisional biopsy" refers to the removal of a wedge of tissue that includes a cross-sectional diameter of the tumor. A diagnosis or prognosis made by endoscopy or fluoroscopy can require a "core-needle biopsy", or a "fine-needle aspiration biopsy" which generally obtains a suspension of cells from within a target tissue. Biopsy techniques are discussed, for example, in Harrison's Principles of Internal Medicine, Kasper, et al., eds., 16th ed., 2005, Chapter 70, and throughout Part V.

The terms "overexpress," "overexpression" or "overexpressed" interchangeably refer to a protein or nucleic acid (RNA) that is transcribed or translated at a detectably greater level, usually in a cancer cell, in comparison to a normal cell. The term includes overexpression due to transcription, post transcriptional processing, translation, post-translational processing, cellular localization (e.g., organelle, cytoplasm, nucleus, cell surface), and RNA and protein stability, as compared to a normal cell. Overexpression can be detected using conventional techniques for detecting mRNA (i.e., RT-PCR, PCR, hybridization) or proteins (i.e., ELISA, immunohistochemical techniques). Overexpression can be 10%, 20%, 30%, 40%, 50%, 60%, 70%, 80%, 90% or more in comparison to a normal cell. In certain instances, overexpression is 1-fold, 2-fold, 3-fold, 4-fold or more higher levels of transcription or translation in comparison to a normal cell.

The terms "underexpress," "underexpression" or "underexpressed" or "downregulated" interchangeably refer to a protein or nucleic acid that is transcribed or translated at a detectably lower level in a cancer cell, in comparison to a normal cell. The term includes underexpression due to transcription, post transcriptional processing, translation, post-translational processing, cellular localization (e.g., organelle, cytoplasm, nucleus, cell surface), and RNA and protein stability, as compared to a control. Underexpression can be detected using conventional techniques for detecting mRNA (*i.e.,* RT-PCR, PCR, hybridization) or proteins (*i.e.,* ELISA, immunohistochemical techniques). Underexpression can be 10%, 20%, 30%, 40%, 50%, 60%, 70%, 80%, 90% or less in comparison to a control. In certain instances, underexpression is 1-fold, 2-fold, 3-fold, 4-fold or more lower levels of transcription or translation in comparison to a control.

The term "differentially expressed" or "differentially regulated" refers generally to a protein or nucleic acid that is overexpressed (upregulated) or underexpressed (downregulated) in one sample compared to at least one other sample, generally in a cancer patient, in comparison to a patient without cancer.

"Therapeutic treatment" and "cancer therapies" refers to chemotherapy, hormonal therapy, radiotherapy, immunotherapy, and biologic (targeted) therapy.

By "therapeutically effective amount or dose" or "sufficient amount or dose" herein is meant a dose that produces effects for which it is administered. The exact dose will depend on the purpose of the treatment, and will be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

The terms "identical" or percent "identity," in the context of two or more nucleic acids or polypeptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (*see, e.g.,* NCBI web site ncbi.nlm.nih.gov/BLAST or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length. The biomarkers described herein can be detected with probes that have, e.g., more than 70% identity over a specified region, or more than 80% identity, or more than 90% identity to the reference sequence provided by the accession number, up to 100% identity.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1987-2005, Wiley Interscience)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins disclosed herein. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, Proc. Natl. Acad. Sci. USA 89:10915 (1989)) alignments (B) of 50, expectation (E) of 10, M=5, N=-4, and a comparison of both strands.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)). The term nucleic acid is used interchangeably with gene, cDNA, mRNA, oligonucleotide, and polynucleotide.

A particular nucleic acid sequence also implicitly encompasses "splice variants" and nucleic acid sequences encoding truncated forms of a protein. Similarly, a particular protein encoded by a nucleic acid implicitly encompasses any protein encoded by a splice variant or truncated form of that nucleic acid. "Splice variants," as the name suggests, are products of alternative splicing of a gene. After transcription, an initial nucleic acid transcript may be spliced such that different (alternate) nucleic acid splice products encode different polypeptides. Mechanisms for the production of splice variants vary, but include alternate splicing of exons. Alternate polypeptides derived from the same nucleic acid by read-through transcription are also encompassed by this definition. Any products of a splicing reaction, including recombinant forms of the splice products, are included in this definition. Nucleic acids can be truncated at the 5' end or at the 3' end. Polypeptides can be truncated at the N-terminal end or the C-terminal end. Truncated versions of nucleic acid or polypeptide sequences can be naturally occurring or recombinantly created.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M). *See, e.g.,* Creighton, Proteins (1984).

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (e.g., as commonly used in an ELISA), biotin, digoxigenin, or haptens and proteins which can be made detectable, e.g., by incorporating a radiolabel into the peptide or used to detect antibodies specifically reactive with the peptide.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The phrase "stringent hybridization conditions" refers to conditions under which a probe will hybridize to its target subsequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization. Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 65°C.

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37°C, and a wash in 1X SSC at 45°C. A positive hybridization is at least twice background. Those of ordinary skill will readily recognize that alternative hybridization and wash conditions can be utilized to provide conditions of similar stringency. Additional guidelines for determining hybridization parameters are provided in numerous reference, e.g., and Current Protocols in Molecular Biology, ed. Ausubel*, et al., supra.*

For PCR, a temperature of about 36°C is typical for low stringency amplification, although annealing temperatures may vary between about 32°C and 48°C depending on primer length. For high stringency PCR amplification, a temperature of about 62°C is typical, although high stringency annealing temperatures can range from about 50°C to about 65°C, depending on the primer length and specificity. Typical cycle conditions for both high and low stringency amplifications include a denaturation phase of 90°C - 95°C for 30 sec - 2 min., an annealing phase lasting 30 sec. - 2 min., and an extension phase of about 72°C for 1 - 2 min. Protocols and guidelines for low and high stringency amplification reactions are provided, e.g., in Innis et al. (1990) PCR Protocols, A Guide to Methods and Applications, Academic Press, Inc. N.Y.).

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding. Antibodies can be polyclonal or monoclonal, derived from serum, a hybridoma or recombinantly cloned, and can also be chimeric, primatized, or humanized.

An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, e.g., as intact immunoglobulins or as a number of well-characterized fragments produced by digestion with various peptidases. Thus, for example, pepsin digests an antibody below the disulfide linkages in the hinge region to produce F(ab)'₂, a dimer of Fab which itself is a light chain joined to V_{H}-C_{H}l by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (*see Fundamental Immunology* (Paul ed., 3d ed. 1993). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies (e.g., single chain Fv) or those identified using phage display libraries (*see, e.g.,* McCafferty et al., Nature 348:552-554 (1990)).

In one embodiment, the antibody is conjugated to an "effector" moiety. The effector moiety can be any number of molecules, including labeling moieties such as radioactive labels or fluorescent labels, or can be a therapeutic moiety. In one aspect the antibody modulates the activity of the protein.

The nucleic acids of the differentially expressed genes disclosed herein or their encoded polypeptides refer to all forms of nucleic acids (e.g., gene, pre-mRNA, mRNA) or proteins, their polymorphic variants, alleles, mutants, and interspecies homologs that (as applicable to nucleic acid or protein): (1) have an amino acid sequence that has greater than about 60% amino acid sequence identity, 65%, 70%, 75%, 80%, 85%, 90%, preferably 91%, 92%, 93%, 94%, 95%, 96%, 97%, 98% or 99% or greater amino acid sequence identity, preferably over a region of at least about 25, 50, 100, 200, 500, 1000, or more amino acids, to a polypeptide encoded by a referenced nucleic acid or an amino acid sequence described herein; (2) specifically bind to antibodies, *e.g.,* polyclonal antibodies, raised against an immunogen comprising a referenced amino acid sequence, immunogenic fragments thereof, and conservatively modified variants thereof; (3) specifically hybridize under stringent hybridization conditions to a nucleic acid encoding a referenced amino acid sequence, and conservatively modified variants thereof; (4) have a nucleic acid sequence that has greater than about 95%, preferably greater than about 96%, 97%, 98%, 99%, or higher nucleotide sequence identity, preferably over a region of at least about 25, 50, 100, 200, 500, 1000, or more nucleotides, to a reference nucleic acid sequence. A polynucleotide or polypeptide sequence is typically from a mammal including, but not limited to, primate, e.g., human; rodent, e.g., rat, mouse, hamster; cow, pig, horse, sheep, or any mammal. The nucleic acids of the invention include both naturally occurring or recombinant molecules. Truncated and alternatively spliced forms of these antigens are included in the definition.

The phrase "specifically (or selectively) binds" when referring to a protein, nucleic acid, antibody, or small molecule compound refers to a binding reaction that is determinative of the presence of the protein or nucleic acid, such as the differentially expressed genes of the present invention, often in a heterogeneous population of proteins or nucleic acids and other biologics. In the case of antibodies, under designated immunoassay conditions, a specified antibody may bind to a particular protein at least two times the background and more typically more than 10 to 100 times background. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (*see, e.g.,* Harlow & Lane, Antibodies, A Laboratory Manual (1988) for a description of immunoassay formats and conditions that can be used to determine specific immunoreactivity).

The phrase "functional effects" in the context of assays for testing compounds that modulate a marker protein includes the determination of a parameter that is indirectly or directly under the influence of a biomarker of the invention, e.g., a chemical or phenotypic. A functional effect therefore includes ligand binding activity, transcriptional activation or repression, the ability of cells to proliferate, the ability to migrate, among others. "Functional effects" include *in vitro, in vivo,* and *ex vivo* activities.

By "determining the functional effect" is meant assaying for a compound that increases or decreases a parameter that is indirectly or directly under the influence of a biomarker of the invention, e.g., measuring physical and chemical or phenotypic effects. Such functional effects can be measured by any means known to those skilled in the art, e.g., changes in spectroscopic characteristics (e.g., fluorescence, absorbance, refractive index); hydrodynamic (e.g., shape), chromatographic; or solubility properties for the protein; ligand binding assays, e.g., binding to antibodies; measuring inducible markers or transcriptional activation of the marker; measuring changes in enzymatic activity; the ability to increase or decrease cellular proliferation, apoptosis, cell cycle arrest, measuring changes in cell surface markers. The functional effects can be evaluated by many means known to those skilled in the art, e.g., microscopy for quantitative or qualitative measures of alterations in morphological features, measurement of changes in RNA or protein levels for other genes expressed in placental tissue, measurement of RNA stability, identification of downstream or reporter gene expression (CAT, luciferase, *β*-gal, GFP and the like), e.g., via chemiluminescence, fluorescence, colorimetric reactions, antibody binding, inducible markers, etc.

"Inhibitors," "activators," and "modulators" of the markers are used to refer to activating, inhibitory, or modulating molecules identified using *in vitro and in vivo* assays of cancer biomarkers. Inhibitors are compounds that, e.g., bind to, partially or totally block activity, decrease, prevent, delay activation, inactivate, desensitize, or down regulate the activity or expression of cancer biomarkers. "Activators" are compounds that increase, open, activate, facilitate, enhance activation, sensitize, agonize, or up regulate activity of cancer biomarkers, e.g., agonists. Inhibitors, activators, or modulators also include genetically modified versions of cancer biomarkers, e.g., versions with altered activity, as well as naturally occurring and synthetic ligands, antagonists, agonists, antibodies, peptides, cyclic peptides, nucleic acids, antisense molecules, ribozymes, RNAi and siRNA molecules, small organic molecules and the like. Such assays for inhibitors and activators include, e.g., expressing cancer biomarkers in *vitro,* in cells, or cell extracts, applying putative modulator compounds, and then determining the functional effects on activity, as described above.

Samples or assays comprising cancer biomarkers that are treated with a potential activator, inhibitor, or modulator are compared to control samples without the inhibitor, activator, or modulator to examine the extent of inhibition. Control samples (untreated with inhibitors) are assigned a relative protein activity value of 100%. Inhibition of cancer biomarkers is achieved when the activity value relative to the control is about 80%, preferably 50%, more preferably 25-0%. Activation of cancer biomarkers is achieved when the activity value relative to the control (untreated with activators) is 110%, more preferably 150%, more preferably 200-500% (i.e., two to five fold higher relative to the control), more preferably 1000-3000% higher.

The term "test compound" or "drug candidate" or "modulator" or grammatical equivalents as used herein describes any molecule, either naturally occurring or synthetic, e.g., protein, oligopeptide (e.g., from about 5 to about 25 amino acids in length, preferably from about 10 to 20 or 12 to 18 amino acids in length, preferably 12, 15, or 18 amino acids in length), small organic molecule, polysaccharide, peptide, circular peptide, lipid, fatty acid, siRNA, polynucleotide, oligonucleotide, etc., to be tested for the capacity to directly or indirectly modulate cancer biomarkers. The test compound can be in the form of a library of test compounds, such as a combinatorial or randomized library that provides a sufficient range of diversity. Test compounds are optionally linked to a fusion partner, e.g., targeting compounds, rescue compounds, dimerization compounds, stabilizing compounds, addressable compounds, and other functional moieties. Conventionally, new chemical entities with useful properties are generated by identifying a test compound (called a "lead compound") with some desirable property or activity, e.g., inhibiting activity, creating variants of the lead compound, and evaluating the property and activity of those variant compounds. Often, high throughput screening (HTS) methods are employed for such an analysis.

A "small organic molecule" refers to an organic molecule, either naturally occurring or synthetic, that has a molecular weight of more than about 50 daltons and less than about 2500 daltons, preferably less than about 2000 daltons, preferably between about 100 to about 1000 daltons, more preferably between about 200 to about 500 daltons.

### PROGNOSTIC METHODS

The present invention provides methods of predicting or providing prognosis for lung cancer by detecting the expression of a panel of markers differentially expressed in the cancer. Examples of such panels would include all of the genes from ctnnb1, wnt3a, tp53, kras, erbb3, muc1, erbb2, and dusp6, or all of the genes wnt3a, rnd3, lck, and erbb3. Prediction and prognosis involve determining the level of a panel of lung cancer biomarker polynucleotide or the corresponding polypeptides in a patient or patient sample and then comparing the level to a baseline or range. Typically, the baseline value is representative of levels of the polynucleotide or nucleic acid in a healthy person not suffering from, or destined to develop, lung cancer, as measured using a biological sample such as a lung biopsy or a sample of a bodily fluid. Variation of levels of a polynucleotide or corresponding polypeptides of the invention from the baseline range (either up or down) indicates that the patient has an increased risk of long term mortality.

In a preferred embodiment, real time or quantitative PCR is used to examine expression of the eight biomarkers in the panel using RNA from a biological sample such as tumor tissue. No microdissection is required. RNA extraction can be performed by any method know to those of skill in the art, e.g., using Trizol and RNeasy. Real time PCR can be performed by any method known to those of skill in the art, e.g., Taqman real time PCR using Applied Biosystem assays. Gene expression is calculated relative to pooled normal lung RNA, and expression is normalized to housekeeping genes. Suitable oligonucleotide primers are selected by those of skill in the art. In one embodiment, the assay is used for stage I, stage II, stage III, or stage IV cancers. In one embodiment, the tissue sample is from a surgically resected tumor.

In one embodiment, RNA biomarkers are examined using nucleic acid binding molecules such as probes, oligonucleotides, oligonucleotide arrays, and primers to detect differential RNA expression in patient samples. In one embodiment, RT-PCR is used according to standard methods known in the art. In another embodiment, quantitative PCR assays such as Taqman^{®} assays available from, e.g., Applied Biosystems, can be used to detect nucleic acids and variants thereof. In other embodiments, nucleic acid microarrays can be used to detect nucleic acids. Analysis of nucleic acids can be achieved using routine techniques such as northern analysis, or any other methods based on hybridization to a nucleic acid sequence that is complementary to a portion of the marker coding sequence (e.g., slot blot hybridization) are also within the scope of the present invention. Reagents that bind to selected nucleic acid biomarkers can be prepared according to methods known to those of skill in the art or purchased commercially.

Applicable PCR amplification techniques are described in, e.g., Ausubel *et al.* and Innis *et al., supra.* General nucleic acid hybridization methods are described in Anderson, "Nucleic Acid Hybridization," BIOS Scientific Publishers, 1999. Amplification or hybridization of a plurality of nucleic acid sequences (e.g., genomic DNA, mRNA or cDNA) can also be performed from mRNA or cDNA sequences arranged in a microarray. Microarray methods are generally described in Hardiman, "Microarrays Methods and Applications: Nuts & Bolts," DNA Press, 2003; and Baldi et al., "DNA Microarrays and Gene Expression: From Experiments to Data Analysis and Modeling," Cambridge University Press, 2002.

Analysis of nucleic acid markers can be performed using techniques known in the art including, without limitation, sequence analysis, and electrophoretic analysis. Non-limiting examples of sequence analysis include Maxam-Gilbert sequencing, Sanger sequencing, capillary array DNA sequencing, thermal cycle sequencing (Sears et al., Biotechniques, 13:626-633 (1992)), solid-phase sequencing (Zimmerman et al., Methods Mol. Cell Biol., 3:39-42 (1992)), sequencing with mass spectrometry such as matrix-assisted laser desorption/ionization time-of-flight mass spectrometry (MALDI-TOF/MS; Fu et al., Nat. Biotechnol., 16:381-384 (1998)), and sequencing by hybridization. Chee et al., Science, 274:610-614 (1996); Drmanac et al., Science, 260:1649-1652 (1993); Drmanac et al., Nat. Biotechnol., 16:54-58 (1998). Non-limiting examples of electrophoretic analysis include slab gel electrophoresis such as agarose or polyacrylamide gel electrophoresis, capillary electrophoresis, and denaturing gradient gel electrophoresis.

In another aspect of the present disclosure, antibody reagents can be used in assays to detect expression levels of protein biomarkers in patient samples using any of a number of immunoassays known to those skilled in the art. Immunoassay techniques and protocols are generally described in Price and Newman, "Principles and Practice of Immunoassay," 2nd Edition, Grove's Dictionaries, 1997; and Gosling, "Immunoassays: A Practical Approach," Oxford University Press, 2000. A variety of immunoassay techniques, including competitive and non-competitive immunoassays, can be used. *See, e.g.,* Self et al., Curr. Opin. Biotechnol., 7:60-65 (1996). The term immunoassay encompasses techniques including, without limitation, enzyme immunoassays (EIA) such as enzyme multiplied immunoassay technique (EMIT), enzyme-linked immunosorbent assay (ELISA), IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); and chemiluminescence assays (CL). If desired, such immunoassays can be automated. Immunoassays can also be used in conjunction with laser induced fluorescence. *See, e.g.,* Schmalzing et al., Electrophoresis, 18:2184-93 (1997); Bao, J. Chromatogr. B. Biomed. Sci., 699:463-80 (1997). Liposome immunoassays, such as flow-injection liposome immunoassays and liposome immunosensors, are also suitable for use in the present invention. *See, e.g.,* Rongen et al., J. Immunol. Methods, 204:105-133 (1997). In addition, nephelometry assays, in which the formation of protein/antibody complexes results in increased light scatter that is converted to a peak rate signal as a function of the marker concentration, are suitable. Nephelometry assays are commercially available from Beckman Coulter (Brea, CA; Kit #449430) and can be performed using a Behring Nephelometer Analyzer (Fink et al., J. Clin. Chem. Clin. Biochem., 27:261-276 (1989)).

A detectable moiety can be used in the assays described herein (direct or indirect detection). A wide variety of detectable moieties can be used, with the choice of label depending on the sensitivity required, ease of conjugation with the antibody, stability requirements, and available instrumentation and disposal provisions. Suitable detectable moieties include, but are not limited to, radionuclides, fluorescent dyes (e.g., fluorescein, fluorescein isothiocyanate (FITC), Oregon Green™, rhodamine, Texas red, tetrarhodimine isothiocynate (TRITC), Cy3, Cy5, *etc.*)*,* fluorescent markers (e.g., green fluorescent protein (GFP), phycoerythrin, *etc.*)*,* autoquenched fluorescent compounds that are activated by tumor-associated proteases, enzymes (e.g., luciferase, horseradish peroxidase, alkaline phosphatase, *etc.*), nanoparticles, biotin, digoxigenin, metals, and the like.

A chemiluminescence assay using a chemiluminescent antibody specific for the nucleic acid is suitable for sensitive, non-radioactive detection of protein levels. An antibody labeled with fluorochrome is also suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), *β*-galactosidase, urease, and the like. A horseradish-peroxidase detection system can be used, for example, with the chromogenic substrate tetramethylbenzidine (TMB), which yields a soluble product in the presence of hydrogen peroxide that is detectable at 450 nm. An alkaline phosphatase detection system can be used with the chromogenic substrate p-nitrophenyl phosphate, for example, which yields a soluble product readily detectable at 405 nm. Similarly, a *β*-galactosidase detection system can be used with the chromogenic substrate o-nitrophenyl-*β*-D-galactopyranoside (ONPG), which yields a soluble product detectable at 410 nm. An urease detection system can be used with a substrate such as urea-bromocresol purple (Sigma Immunochemicals; St. Louis, MO).

A signal from the direct or indirect label can be analyzed, for example, using a spectrophotometer to detect color from a chromogenic substrate; a radiation counter to detect radiation such as a gamma counter for detection of ¹²⁵I; or a fluorometer to detect fluorescence in the presence of light of a certain wavelength. For detection of enzyme-linked antibodies, a quantitative analysis can be made using a spectrophotometer such as an EMAX Microplate Reader (Molecular Devices; Menlo Park, CA) in accordance with the manufacturer's instructions. If desired, the assays of the present invention can be automated or performed robotically, and the signal from multiple samples can be detected simultaneously.

The antibodies can be immobilized onto a variety of solid supports, such as magnetic or chromatographic matrix particles, the surface of an assay plate (e.g., microtiter wells), pieces of a solid substrate material or membrane (e.g., plastic, nylon, paper), and the like. An assay strip can be prepared by coating the antibody or a plurality of antibodies in an array on a solid support. This strip can then be dipped into the test sample and processed quickly through washes and detection steps to generate a measurable signal, such as a colored spot.

Useful physical formats comprise surfaces having a plurality of discrete, addressable locations for the detection of a plurality of different markers. Such formats include microarrays and certain capillary devices. *See, e.g.,* Ng et al., J. Cell Mol. Med., 6:329-340 (2002); U.S. Pat. No. 6,019,944. In these aspects, each discrete surface location may comprise antibodies to immobilize one or more markers for detection at each location. Surfaces may alternatively comprise one or more discrete particles (e.g., microparticles or nanoparticles) immobilized at discrete locations of a surface, where the microparticles comprise antibodies to immobilize one or more markers for detection.

Analysis can be carried out in a variety of physical formats. For example, the use of microtiter plates or automation could be used to facilitate the processing of large numbers of test samples. Alternatively, single sample formats could be developed to facilitate diagnosis or prognosis in a timely fashion.

Alternatively, the nucleic acid probes of the invention can be applied to sections of patient biopsies immobilized on microscope slides. The resulting in situ hybridization pattern can be visualized using any one of a variety of light or fluorescent microscopic methods known in the art.

In another format, the various markers of the invention also provide reagents for in vivo imaging such as, for instance, the imaging of labeled regents that detect the nucleic acids of the biomarkers of the invention. For in vivo imaging purposes, reagents that detect the presence of proteins encoded by cancer biomarkers, such as antibodies, may be labeled using an appropriate marker, such as a fluorescent marker.

### REPORTS

In another aspect, the disclosure features a report indicating a prognosis of a subject with cancer. The report can, for example, be in electronic or paper form. The report can include basic patient information, including a subject identifier (e.g., the subject's name, a social security number, a medical insurance number, or a randomly generated number), physical characteristics of the subject (e.g., age, weight, or sex), the requesting physician's name, the date the prognosis was generated, and the date of sample collection. The reported prognosis can relate to likelihood of survival for a certain period of time, likelihood of response to certain treatments within a certain period of time (e.g., chemotherapeutic or surgical treatments), and/or likelihood of reoccurrence of cancer. The reported prognosis can be in the form of a percentage chance of survival for a certain period of time, percentage chance of favorable response to treatment (favorable response can be defined, e.g., tumor shrinkage or slowing of tumor growth), or reoccurrence over a defined period of time (e.g., 20% chance of survival over a five year period). The reported prognosis can alternatively be in the form of a calculated score. A greater or lower score, for example, can be indicative of a favorable prognosis. In another aspect, the reported prognosis can be a general description of the likelihood of survival, response to treatment, or reoccurrence over a period of time (e.g., very likely, likely, or unlikely to survive for five years). In another aspect, the reported prognosis can be in the form of a graph. In addition to the gene expression levels, the reported prognosis may also take into account additional characteristics of the subject (e.g., age, stage of cancer, gender, previous treatment, fitness, cardiovascular health, and mental health).

In addition to a prognosis, the report can optionally include raw data concerning the expression level at least two genes selected from Rnd3, wnt3a, erbb3, lck, sh3bgr, fut3, il11, cdc6, cdk2ap1, bag1, emx2, six3, and brca1.

### EXAMPLES

The following examples are offered to illustrate, but not to limit the claimed invention.

### Example 1. CTNNB1, WNT3A, TP53, KRAS, ERBB3, MUC1, ERBB2, AND DUSP6

The present invention analyzed data on 105 patients with surgically resected lung adenocarcinoma who had at least 3 years of follow up and had high quality tissue accessible. Real-time PCR was run on 65 genes and housekeeping genes (18S and POL2RA, see also Table 1). PCR output (in cycle thresholds, CT) was normalized to commercially available pooled normal lung RNA and 18S as the housekeeping gene. The resulting values (as shown in Table 2) are proportion of expression relative to the normal lung RNA. In Table 2, the genes are arranged in rows, and the genes are abbreviated. The number before each gene (e.g. 7-FZD) is a number we used to keep track of genes and has no other bearing. These values were then log transformed (the second set of values in Table 2).

The analysis was done as follows. The samples (patients) were divided into 2 sets: a training set (n=70) and a validation set (n=35) randomly. Cox modeling was performed on each gene individually in the training set, and a liberal P value threshold of 0.2 for survival was used to select genes. We then did backwards selection of all these genes together on the training set, that is dropping genes one-by-one based on P value criteria of 0.2, and stopping when dropping that gene had a meaningful change in one or more of the remaining gene's coefficients. The final model contained the following eight genes: ctnnb1, wnt3a, tp53, kras, erbb3, muc1, erbb2, and dusp6. Individual sample expression values were then plugged into the model to arrive at a score for each sample. We chose to set the cutpoint for a high score as the top tertile of scores. Kaplan-Meier curves were generated for all samples and also for stage I patients only. This was repeated in the validation set. A cox model was run including tumor size and tumor stage and a high-score predicted a higher risk of death after adjusting for these other variables.

### Example 2. WNT3A, RND3, LCK, and ERBB3

Ideally, a prognostic tool should provide accurate risk stratification, should be clinically feasible to employ in day-to-day practice, and should be cost effective. Such an assay would be of particular benefit to patients with surgically resected stage I NSCLC. The current standard of care for stage I NSCLC is lobectomy and mediastinal lymph node dissection, without adjuvant chemotherapy. Better identification of good prognosis patient subsets might allow lesser surgical procedures to be employed with equal survival potential. Conversely, stage I subsets with a poor prognosis could be selected for inclusion into clinical trials testing novel approaches and new therapeutic agents. Considering the current limitations of chemotherapy in stage I disease, a bioassay that is both prognostic and predictive of chemotherapy benefit would be especially beneficial. Lastly, stage I NSCLC is likely to be of increasing importance in the future. While approximately twenty percent of patients currently diagnosed with NSCLC are stage I, this proportion probably will grow due to the recent advent of lung cancer screening by computerized tomography.

We have found that the expression of Wnt3a, rnd3, lck, and erbb3 is prognostic of survival in lung cancer patients. Clinical and histologic characteristics of patients studied are listed in **Table 3.** We first analyzed the entire data set, and subsequently restricted the analysis to stage I patients. Cross-validation, after forced adjustment for age, tumor size, and stage, supported a model containing 4 genes, selected in the following order: Wnt3a, rnd3, lck, and erbb3. Overall survival (OS) was analyzed utilizing the risk score for the model utilizing these four genes (with L1 shrunken coefficients). After adjusting for patient age, disease stage, and tumor size, the hazard ratio (HR) for the risk score as a continuous variable was 6.7 (95% CI: 1.6-28.9, p=0.005), as listed in **Table 4.**

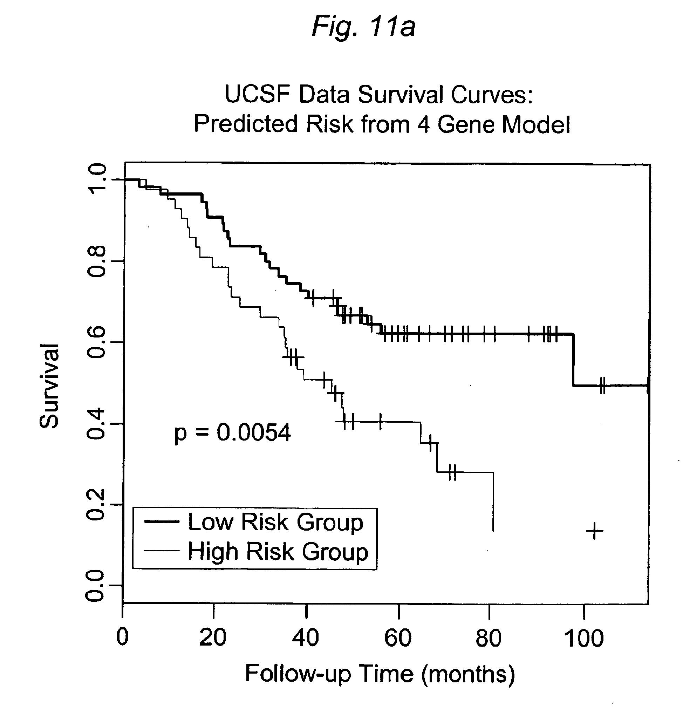

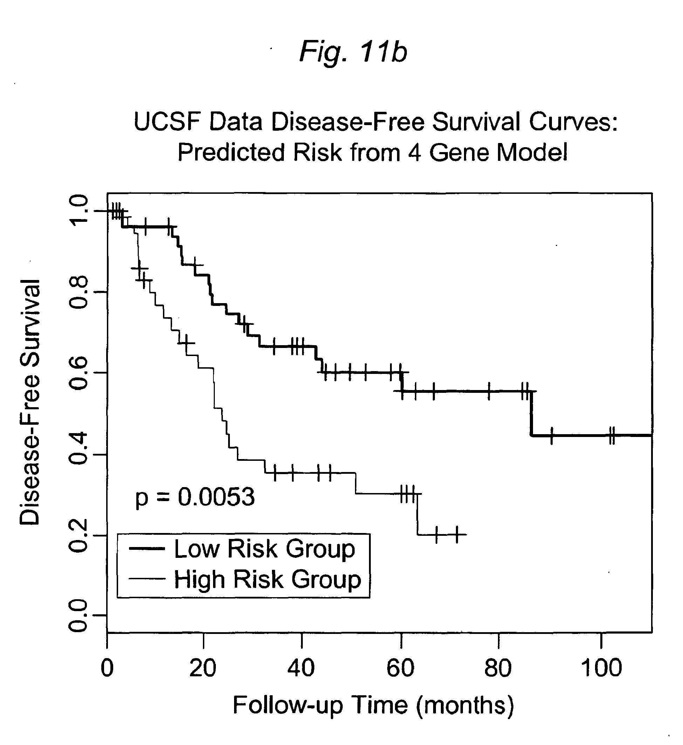

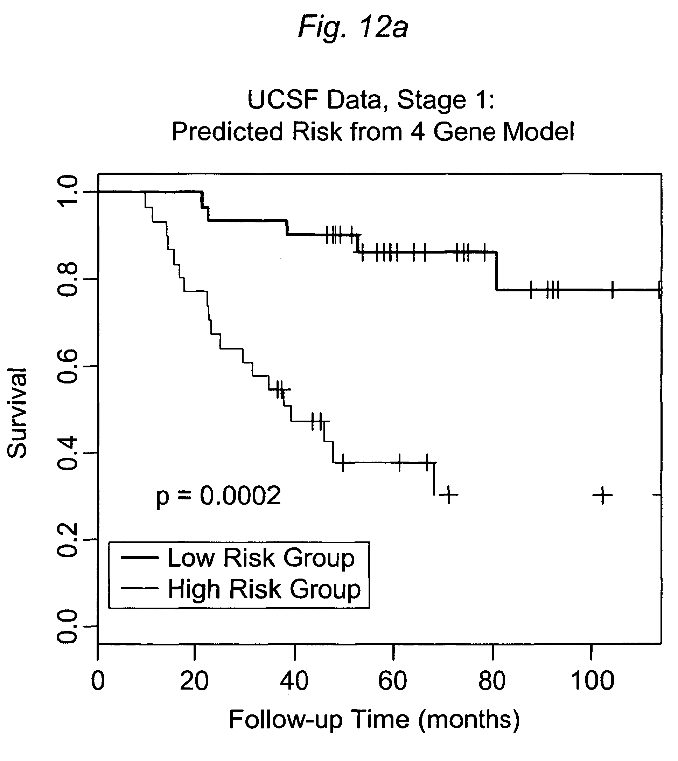

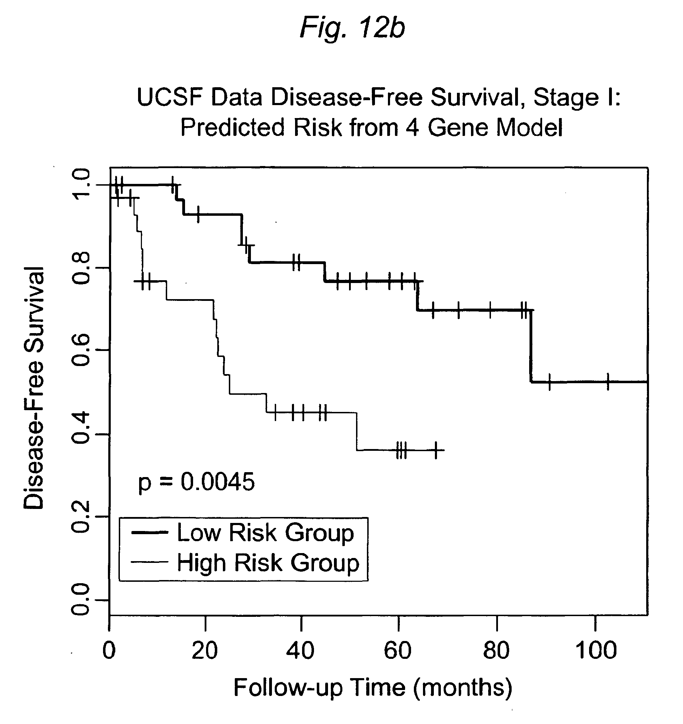

Survival curves for the entire study cohort based on dichotomized risk scores from the 4 gene model are presented in **Figure 11a** **and** **11b****.** Corresponding survival curves for dichotomized risk scores among 70 stage I patients are presented in **Figure 12a** **and** **12b****.** 5-year OS was 56% for all patients and 65% for stage I patients. Among all patients, 5-year OS was 62% among low-risk patients and 41 % among high-risk patients (p=0.0054). 5-year DFS was 60% among low-risk patients and 28% among high-risk patients (p=0.0053). Among stage I patients, 5-year OS was 87% among low-risk patients and 38% among high-risk patients (p=0.0002), and DFS was 77% among low-risk patients and 35% among high-risk patients (p=0.0045).

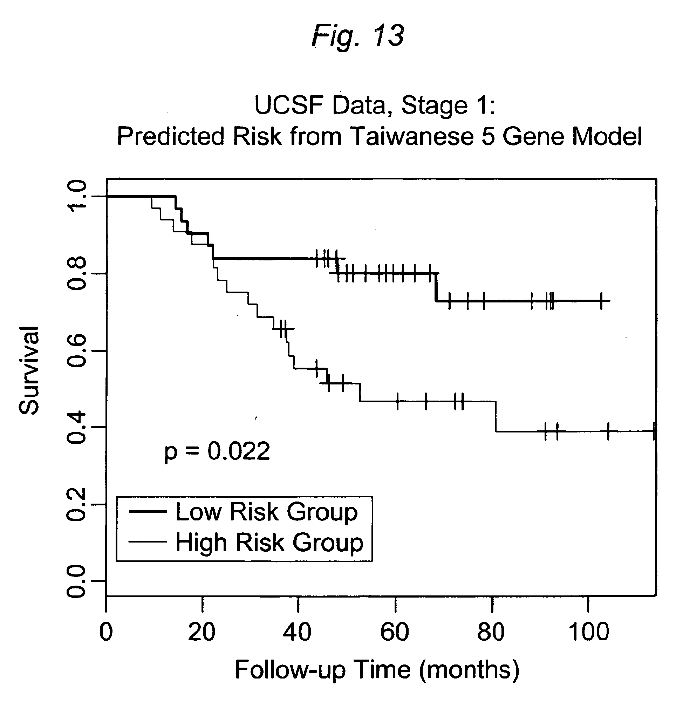

Applying the 5-gene model of Chen and colleagues to our study population, the HR adjusted for age, stage, and tumor size was 2.4 (95% CI: 1.3-4.3, p=0.0047) 13. (Chen et al. N Eng1 J Med 356:11-20 (2007). Among all patients, 5-year OS was 65% among low-risk and 43% among high risk patients (p=0.045). Among stage I patients, 5-year OS was 80% among low-risk and 47% among high risk patients (p=0.022), **Figure 13****.**

We identified a risk score based on the gene expression of 4-genes by quantitative PCR that is prognostic of long-term survival in patients with completely surgically resected lung adenocarcinoma. This gene expression-based score predicted survival (adjusted HR 6.7) and disease recurrence better than clinical stage and tumor size. Our model best predicted risk of death among patients with stage I disease. In our cross-validated cohort, low- and high-risk scores were associated with 5-year overall survival of 87% and 38% among stage I patients.

We utilized cross-validation both for model selection and for model validation. Rather than splitting data into test and validation sets, cross-validation utilizes repeated data-splitting to prevent model over-fitting and to generate accurate estimates of model coefficients. Cross-validation allows for the generation of validated model coefficients, but utilizes data more efficiently than simple data-splitting.

This prognostic model has important clinical implications for diagnosis and providing a prognosis for lung cancer, e.g., as a tool to complement clinical staging in risk-stratifying patients with stage I lung adenocarcinoma. The current standard of care for patients with stage I NSCLC is surgical resection alone, typically lobectomy. Conversely, post-resection patients at low risk for recurrence may be best served by observation alone, and may even be candidates for less radical lung resection.

In our patient population, the prognostic value of our model compares favorably to that of Chen and colleagues (Id.). There are several possible explanations for this finding. First, candidate genes for our model were selected based on previously published genome-wide expression microarray studies, whereas genes in the Chen model were selected from a more limited custom expression array platform. Additionally, a relatively large set of genes were assessed by RT-PCR before finalizing our gene model. Moreover, our patient follow-up time was relatively long, with a minimum follow-up period of 3 years and a median follow-up of 61 months. This factor may have allowed us to more accurately detect differences in survival between risk groups.

### Methods

### Patients

Between January 1997 and June 2004, 120 patients who had undergone complete surgical resection of lung adenocarcinoma, and had fresh-frozen tissue banked for genomic analysis, were entered into the study. Eligible patients had undergone surgical resection with curative intent and had adequate mediastinal lymph node staging. Patients who received preoperative chemotherapy were excluded from the study, so as not to confound development of a purely prognostic tool. Thirteen patients were excluded due to insufficient banked tissue, inadequate RNA quality, or weak expression of housekeeping genes. The primary endpoint was overall survival. Disease-free survival (DFS) was defined as the time from surgery until radiographic evidence of recurrent disease or time until the last documented physician follow-up visit in the absence of recurrent disease. Patients consented to tissue specimen collection prospectively, and the study was approved by the UCSF institutional review board (CHR# H8714-28880-01).

### Gene Selection

Of 217 genes identified from previously published microarray and PCR-based studies of prognosis in early stage lung cancer (see, for example, Beer et al. Nat Med 8:816 (2002); Potti et al. N Eng1 J Med 355:570 (2006); Wigle et al. Cancer Res 62:3005 (2002); Garber et al. Proc Natl Acad Sci U S A 98:13784 (2001); Miura et al. Cancer Res 62:3244 (2002); and Schneider et al. Br J Cancer 83:473 (2000)), 76 cancer-related genes were identified by content experts or literature review. 15 genes were excluded due to non-functioning primers or very weak expression in tumor tissue by RT-PCR in a pilot study. The remaining 61 genes are listed in

### Table 5.

### Sample preparation and analysis

All tissue was frozen in liquid nitrogen at the time of the operation. Tissue was macrodissected into 1cm³ sections which was ground in liquid nitrogen. RNA was extracted using a TriZol (Invitrogen, Carlsbad, CA) extraction protocol. Taqman RT-PCR was performed on cDNA in 384-well plates using Prism 7900HT machine (Applied Biosystems, Foster City, CA). The expression of each gene was assayed in triplicate. Samples were compared to commercially available pooled normal lung RNA (Clontech, Mountain View, CA), and normalized to 18S ribosomal rRNA (Applied Biosystems).

Approximately 1cm-cube blocks of tumor specimen were obtained from fresh lung resections and immediately snap-frozen in liquid nitrogen. Frozen tissues were stored at -80°C. Total RNA was extracted from 131 individual tumor specimens using a TriZol (Invitrogen) extraction protocol. Quality of RNA samples was verified using Agilent 2100 BioAnalyzer™ (Agilent), and RNA-concentrations of each sample were determined by Nanodrop ND-1000™ (Nanodrop). First-strand cDNA was synthesized off 3µg of total RNA templates using the iScript cDNA Synthesis Kit (BioRad), and the expression levels corresponding to each of 86 different gene transcripts were measured by real-time quantitative PCR using an ABI PRISM 7900 HT Sequence Detection System with automation accessory (Applied Biosystems). Each 20µL reaction, performed in triplicate, consisted of TaqMan Universal PCR Master Mix (Applied Biosystems), the appropriate Taqman Gene Expression Assay (Applied Biosystems), and 1.8µL cDNA template corresponding to 120ng of total RNA per reaction.

Ct values were obtained using the Sequence Detection System (SDS) 2.3 software (Applied Biosystems) and relative gene expression values were calculated as ΔΔCt, which yields the relative expression level of a target gene normalized to that of an endogenous reference gene relative to a calibrator sample (the reference for all samples). The calibrator sample used was cDNA synthesized from normal human lung total RNA supplied by Clontech (Catalog # 636524). To select an endogenous reference gene, expression levels in lung cancer (both tumor and normal tissues) of a number of commonly used reference genes published earlier were compared. Three genes-encoding the *18S* ribosomal RNA subunit, *POL2RA* RNA polymerase, and *TBP* TATA box protein-were selected and their expression levels were compared across dilutions of several cDNA specimens from both tumor and normal samples. *18S* rRNA exhibited the most stable expression across lung samples (tumor and normal), and therefore raw gene expression values were normalized to that of *18S* rRNA.

### Statistical Analysis

The salient features of our data structure are (i) a right censored survival endpoint (death) with modest event numbers (47 - after subsetting to exclude missings); (ii) a multitude (61) of predictors as constituted by the (log) gene expression values obtained from RT-QPCR (delta-delta Cts described elsewhere); and (iii) select clinical and demographic covariates. In view of these features and dimensions the primary data analytic tool we employed was L1 penalized Cox proportional hazards regression. This methodology extends the simultaneous coefficient shrinkage and predictor selection that is inherent in L1 penalization, where it has proven highly effective, to the survival data setting. Earlier extensions, largely motivated by microarray gene expression applications, were either computationally prohibitive or reliant on approximation. We used cross-validation to determine model size; i.e., number of selected genes. A risk score was then generated for each subject based on model coefficients. Resultant predicted risk scores were dichotomized (at the median) and corresponding Kaplan-Meier survival curves displayed and compared via the log-rank statistic. All analyses were conducted using the statistical package R (Version 2.3.1, 2006).

**Table 1**

| **Taqman Assay List** | | | | |
|---|---|---|---|---|
| number | Gene Symbol | Gene Name | NCBI Gene Reference | Assay ID |
| 2 | FZD7 | frizzled homolog 7 (Drosophila) | NM_003507.1,AB017365.1,BC0159 15.1 | Hs00275833_s1 |
| 6 | WIF1 | WNT inhibitory factor 1 | NM_007191.2,AF122922.1,BX6474 27.1,BC018037.1,AY358344.1 | Hs00183662_m1 |
| 7 | FUT3 | fucosyltran sferase 3 (galactoside 3(4)-L-fucosyltransferase, Lewis blood group included) | NM_000149.1,U27326.1,U27327.1, U27328.1,AF131913.1 | Hs00356857_m1 |
| 11 | NXF1 | nuclear RNA export factor 1 | NM_006362.3,AJ132712.1,U80073.1,AF112880.1,AF126246.1,AK0271 92.1,BC004904.2,CR749674.1,AB2 09915.1,BC028041.1 | Hs00234741_m1 |
| 15 | ASMTL | acetylserotonin O-methyltransferase-like | NM_004192.1,AK001090.1,BC0100 89.2,BC002508.2,AK090498.1 | Hs00414020_m1 |
| 16 | CTNNB1 | catenin (cadherin-associated protein), beta 1, 88kDa | NM_001904.2,Z19054.1,X87838.1, AB062292.1,BC058926.1 | Hs00170025_m1 |
| 17 | SH3BGR | SH3 domain binding glutamic acid-rich protein | NM_001001713.1,NM_007341.2,X9 3498.1,BC006371.2,BM474020.1 | Hs00201109_m1 |
| 18 | ARAF | v-raf murine sarcoma 3611 viral oncogene homolog | NM_001654.1,X04790.1,BC007514 2,BC002466.2,BT019864.1,AK130 043.1 | Hs00176427_m1 |
| 19 | TYROBP | TYRO protein tyrosine kinase binding | NM_198125.1,NM_003332.2,AF019 562.1,AJ010098.1,BC011175.1,CR 450342.1,CR542202.1,AY074782.1 ,8Q576278.1,BT009851.1 | Hs00182426_m1 |
| 20 | MGST2 | microsomal glutathione S-transferase 2 | NM_002413.3,U77604.1,AA122237.1,CR407640.1,AK130948.1,BC025 416.1 | Hs00182064_m1 |
| 21 | LRP3 | low density lipoprotein receptor-related protein 3 | NM_002333.1,AB009462.1,BC0074 08.2 | Hs00233925_m1 |
| 22 | PIK3CG | phosphoino sitide-3-kinase, catalytic, gamma polypeptide | NM_002649.2,X83368.1,AF327656.1,BX648341.1,BC035683.1 | Hs00176916_m1 |
| 23 | BMP7 | bone morphogen etic protein 7 (osteogenic protein 1) | NM_001719.1,M60316.1,X51801.1, BC008584.1,BC004248.1 | Hs00233477_m1 |
| 24 | TP5313 | tumor protein p53 inducible protein 3 | NM_147184.1,NM_004881.2,AF010 309.1,BC000474.2,BC018819.2,BT 007149.1,AK223382.1 | Hs00153280_m1 |
| 25 | BAG1 | BCL2-associated athanogene | NM_004323.3,Z35491.1,AF022224.1,U46917.1,AF116273.1,BC001936.1,BC014774.1,AK096038.1,AK222 749.1 | Hs00185390_m1 |
| 26 | MINA | MYC induced nuclear antigen | NM_032778.3,AK027299.1,AY3905 36.1,CR627479.1,AB083190.1,AY3 02110.2,AY456380.1,BC014928.1, AB083189.1 | Hs00262155_m1 |
| 27 | BCL2 | B-cell CLL/lymph oma 2 | NM_000633.2,M13994.1,M14745.1, X06487.1,BC027258.1 | Hs00608023_m1 |
| 28 | DNMT2 | DNA (cytosine-5-)-methyltrans ferase 2 | NM_004412.3,NM_176081.1,NM_1 76083.1,NM_176084.1,NM_176085.1,NM_176086.1,AJ223333.1,AF01 2128.1,AF045888.1,AF329944.1,AF 329940.1,AF329941.1,AF329942.1, AF329943.1,CR450316.1,BX53796 1.1,BC047733.1 | Hs00189402_m1 |
| 29 | SCUBE2 | signal peptide, CUB domain, EGF-like 2 | NM_020974.1,AK131552.1,AK1230 39.1 | Hs00221277_m1 |
| 30 | PRKCA | protein kinase C, alpha | NM_002737.2,X52479.1,AB209475.1 | Hs00176973_m1 |
| 31 | IL11 | interleukin 11 | NM_000641.2,X58377.1,M57765.1, BC012506.1 | Hs00174148_m1 |
| 32 | GLI1 | glioma-associated oncogene homolog 1 (zinc finger protein) | NM_005269.1,X07384.1,BC013000.2 | Hs00171790_m1 |
| 33 | GLI2 | GLI-Kruppel family member GLI2 | NM_030379.1,NM_030380.1,NM_0 30381.1,NM_005270.2,AB007295.1 ,AB007296.1,AB007297.1,AB00729 8.1,AB209354.1,DQ086814.1 | Hs00257977_m1 |
| 34 | BCL7A | B-cell CLL/lymph oma 7A | NM_001024808.1,NM_020993.3,X8 9984.1,BC094723.1 | Hs00277139_m1 |
| 35 | MUC1 | mucin 1, transmembrane | NM_001018016.1,NM_001018017.1,NM_001018021.1,NM_002456.4, M34088.1,M34089.1,J05581.1,X80 761.1,X52229.1,U60259.1,U60260.1,AF125525.1,AF348143.1,AY3275 82.1,AY327584.1,AY327585.1,AY3 27586.1,AY327587.1,AY3 | Hs00159357_m1 |
| 36 | NRP2 | neuropilin 2 | NM_018534.3,NM_201264.1,NM_2 01266.1,NM_201267.1,NM_201279.1,NM_003872.2,AF016098.1,AF02 2859.1,AF022860.1,BC009222.2,A F280544.1,AF280545.1,AF280546.1,BX537423.1,AK130198.1,AL8336 06.1 | Hs00187290_m1 |
| 37 | WDHD1 | WD repeat and HMG-box DNA binding protein 1 | NM_001008396.1,NM_007086.2,AJ 006266.1,AK001538.1,AK001585.1, AK001620.1,BC063041.1 | Hs00173172_m1 |
| 38 | ACSL6 | acyl-CoA synthetase long-chain family member 6 | NM_001009185.1,NM_015256.2,A B020644.1,AF099740.1,AF129166.1,BC026161.1,BC047453.1 | Hs00362960_m1 |
| 40 | MFHAS1 | malignant fibrous histiocytoma amplified sequence 1 | NM_004225.1,AB016816.1.BC0142 26.2 | Hs00180264_m1 |
| 42 | NMT1 | N-myristoyltransferase 1 | NM_021079.3,M86707.1,AF020500.1,AF043324.1,BC008312.2,BC008 579.1,BC006538.2,BC006569.2,BC 007258.2 | Hs00221506_m1 |
| 43 | UQCRC2 | ubiquinol-cytochromec reductase core protein II | NM_003366.2,J04973.1,BC000484.2,BC003136.1,AK094006.1 | Hs00268685_m1 |
| 44 | EPOR | erythropoiet in receptor | NM_000121.2,M34986.1,M60459.1, BC019092.2,AK074082.1 | Hs00181092_m1 |
| 45 | RARG | retinoic acid receptor, gamma | NM_000966.3,M57707.1,M38258.1, BC064524.1,BC072462.1,BC01909 8.2,BC093729.1,BC093727.1,BC09 8421.1 | Hs00171273_m1 |
| 46 | CAMK4 | calcium/calmodulin-dependent protein kinase IV | NM_001744.3.L17000.1,L24959.1, D30742.1,BC025687.1,BC016695.1 | Hs00174318_m1 |
| 47 | SH2D1A | SH2 domain protein 1A, Duncan's disease (lymphoproliferative syndrome) | NM_002351.1,AL023657.1,AF0729 30.1,AF073019.1,AF100539.1,AF10 0541.1,CR542031.1,CR542043.1,B C020732.1 | Hs00158978_m1 |
| 48 | OVOL2 | ovo-like 2 (Drosophila) | NM_021220.2,AL079276.1,AK0222 84.1,BC006148.1,BT007295.1 | Hs00221902_m1 |
| 49 | VEGF | vascular endothelial growth factor | NM_001025366.1,NM_001025367.1,NM_001025368.1,NM_00102536 9.1,NM_001025370.1,NM_003376.4,AB021221.1,AJ010438.1,M32977.1,M27281.1,X62568.1,AF022375.1 ,S85192.1,AF091352.1,AF214570.1 ,BC065522.1,AY263145.1, | Hs00900054_m1 |
| 50 | LAMB1 | laminin, beta 1 | NM_002291.1,BC026018.2,M61916.1,BX648251.1 | Hs00158620_m1 |
| 53 | WNT3A | wingless-type MMTV integration site family, member 3A | NM_033131.2,AB060284.1,AK0562 78.1 | Hs00263977_m1 |
| 54 | CDC6 | CDC6 cell division cycle 6 homolog (S.cerevisiae) | NM_001254.3,U77949.1,AF022109.1,BC025232.1 | Hs00154374_m1 |
| 55 | MYBL2 | v-myb myeloblastosis viral oncogene homolog (avian)-like 2 | NM_002466.2,X13293.1,BC007585.1,BC053555.1,BU178833.1,AK223 482.1 | Hs00231158_m1 |
| 56 | WNT10B | wingless-type MMTV integration site family, member 10B | NM_003394.2,U81787.1,X97057.1, AB070724.1,BC096353.1,BC09635 4.1,BC096355.1,BC096356.1 | Hs00559664_m1 |
| 57 | MMP11 | matrix metallopeptidase 11 (stromelysin 3) | NM_005940.3,X57766.1,CR602252.1,CR612686.1,CR626443.1,AK075 448.1,BC057788.1,AK129792.1,AK 125911.1 | Hs00171829_m1 |
| 58 | RND3 | Rho family GTPase 3 | NM_005168.3,X95282.1,S82240.1, CR542038.1,CR542070.1,X97758.1 ,BC012513.1,AF498969.1,BT00676 9.1 | Hs00170603_m1 |
| 59 | CTSL2 | cathepsin L2 | NM_001333.2,AB001928.1,Y14734.1,AF070448.1,BC067289.1,AY358641.1 | Hs00952036_m1 |
| 60 | CSTB | cystatin B (stefin B) | NM_000100.2.L03558.1,BC010532.1,BC003370.1,CR542148.1,BT0070 40.1 | Hs00164368_m1 |
| 61 | CD68 | CD68 antigen | NM_001251.1,S57235.1,BC015557.2,BT009923.1,AK222492.1,AK222 514.1 | Hs00154355_m1 |
| 64 | WNT2 | wingless-type MMTV integration site family member 2 | NM_003391.1,X07876.1,BC078170.1,BT019608.1,AK056742.1,BC029 854.1 | Hs00608224_m1 |
| 65 | TP53 | tumor protein p53 (Li-Fraumeni syndrome) | NM_000546.2,K03199.1,M14694.1, M14695.1,X02469.1,X60011.1,X60 012.1,X60013.1,X60014.1,X60015.1,X60016.1,X60017.1,X60018.1,X6 0019.1,X60020.1,X01405.1,AF3078 51.1,BC003596.1,BT019622.1,AB0 82923.1,AY429684. | Hs00153349_m1 |
| 66 | MKI67 | antigen identified by monoclonal antibody Ki-67 | NM_002417.2,X65550.1,X65551.1, AJ567756.1 | Hs00606991_m1 |
| 67 | KRAS | v-Ki-ras2 Kirsten rat sarcoma viral oncogene homolog | NM_004985.3,M54968.1,BC013572.2,AF493917.1,BT007153.1 | Hs00270666_m1 |
| 68 | STK6 | serine/threonine kinase 6 | NM_003600.2,AF011468.1,AL7110 75.1 | Hs00269212_m1 |
| 70 | BIRC5 | baculoviral IAP repeat-containing 5 (survivin) | NM_001012271.1,NM_001168.2,AF 077350.1,BC008718.2,AB028869.1, BC065497.1,CR541740.1,BC03414 8.1,AK223428.1 | Hs00153353_m1 |
| 71 | ERBB2 | v-erb-b2 erythroblastic leukemia viral oncogene homolog 2, neuro/glioblastoma derived oncogene homolog (avian) | NM_001005862.1,NM_004448.2,X0 3363.1,M11730.1,AK131568.1,BC0 80193.1 | Hs00170433_m1 |
| 72 | CCNB1 | cyclin B1 | NM_031966.2,M25753.1,BC006510.2,BT020128.1 | Hs00259126_m1 |
| 73 | CDK2AP1 | CDK2-associated protein 1 | NM_004642.2,AB006077.1,AF0064 84.1,BC034717.1 | Hs00428063_m1 |
| 74 | CCND1 | cyclin D1 (PRAD1: parathyroid adenomatosis 1) | NM_053056.1,M73554.1,M74092.1, M64349.1,X59798.1,BC000076.2,C R536538.1,CR542099.1,BT019844.1,BT019845.1,BC001501.2,BC0140 78.2,BC025302.1,BC023620.2 | Hs00277039_m1 |
| 75 | RHOH | ras homolog gene family, member H | NM_004310.2,Z35227.1,BG025818.1,BC014261.2,CD703012.1 | Hs00180265_m1 |
| 76 | CTSL | cathepsin L | NM_145918.1,NM_001912.2,X1245 1.1,AF217997.1,CR457053.1,AK05 5599.1,AK075100.1,BX537395.1,B X647101.1,BX647102.1,BX647413.1,BX647434.1,BX647435.1,BX6488 48.1,BX648849.1,BX649140.1,BC0 12612.1,AL832167.1 | Hs00377632_m1 |
| 77 | DUSP6 | dual specificity phosphatase 6 | NM_001946.2,AB013382.1,X93920.1,BC005047.1,BC003562.1,BC003 143.1,CR593797.1,BC037236.1,AK 091753.1,BT006895.1,DQ895701.1 | Hs00169257_m1 |
| 78 | MMD | monocyte to macrophage differentiati on-associated | NM_012329.2,X85750.1,BI546449.1,BC026324.1,AY424289.1 | Hs00202450_m1 |
| 79 | STAT1 | signal transducer and activator of transcription 1, 91kDa | NM_139266.1,NM_007315.2,M979 36.1,M97935.1,BC002704.2,CR618 286.1,CR749636.1,AK096686.1,BT 007241.1,AK225853.1 | Hs00234829_m1 |
| 80 | ERBB3 | v-erb-b2 erythroblastic leukemia viral oncogene homolog 3 (avian) | NM_001005915.1,NM_001982.2,M 29366.1,M34309.1,S61953.1,BC00 2706.2,CR621450.1,BC082992.1,B M837872.1,BT007226.1 | Hs00176538_m1 |
| 81 | LCK | lymphocyte -specific protein tyrosine kinase | NM_001042771.1,NM_005356.3,U0 7236.1,M36881.1,X13529.1,X05027.1,U23852.1,AF228313.1,CR59423 6.1,AJ865079.1,AK098027.1 | Hs00178427_m1 |
| 82 | TBP | TATA box binding protein | NM_003194.3,M55654.1,Z22828.1, X54993.1,M34960.1,CR456776.1,C R590323.1,CR612496.1,CR618783.1,BT019657.1,BC109054.1,BC109 053.1,BC110341.1 | Hs00427620_m1 |
| 83 | 18S | Eukaryotic 18S rRNA | X03205.1 | Hs99999901_s1 |
| 84 | POLR2A | polymerase (RNA) II (DNA directed) polypeptide A, 220kDa | NM_000937.2,X63564.1,BC067295.1 | Hs00172187_m1 |
| | housekee ping gene no longer using (dark grey=dupli cate) | | | |

**Table 2**

| Sample | VSDate | VitalSt atus | Followup Time | Sex | Smoking Status | Surgical Date | Cancer Stage | Ethnicity | | Age | MaxOfRelativeTiming | Tumor Size |
|---|---|---|---|---|---|---|---|---|---|---|---|---|
| 24 | 2/1/2002 | Dead | 34.90132 | Male | Past | 3/8/1999 | T1N1 (final stage was T2N1M0) | East Asian | | 73 | Neoadjuvant | 3 |
| 26 | 11/29/2005 | Dead | 80.625 | Female | Past | 3/15/1999 | T2N0 | Caucasian | | 61 | | 2.8 |
| 56 | 11/7/2001 | Dead | 29.07895 | Male | Past | 6/7/1999 | T2N1 (BAC in 1998) | East Asian | | 73 | | 3.5 |
| 57 | 11/14/2000 | Dead | 17.30263 | Female | Past | 6/7/1999 | T1N2 | Other | | 81 | Adjuvant | 3.0 |
| 80 | 5/21/2007 | Alive | 93.25658 | Male | Past | 8/16/1999 | T1N0M X | East Asian | | 90 | Unrelated to Current Cancer | 2.8 |
| 90 | 5/21/2007 | Alive | 92.23684 | Female | Current | 9/16/1999 | T2NXM X | African Americ an | | 61 | | 2 |
| 93 | 5/21/2007 | Alive | 92.33553 | Female | Never | 9/13/1999 | T1N0M X | Caucasian | | 72 | | 2.5 |
| 102 | 5/21/2007 | Alive | 91.875 | Female | | 9/27/1999 | TXN0M X (clinical correlation required for stage) | Hispanic | | 55 | Neoadjuvant | 3 |
| 112 | 5/21/2007 | Alive | 90.95395 | Female | Past | 10/25/1999 | T1N0M X | Caucasian | | 71 | | 2.5 |
| 113 | 5/21/2007 | Alive | 90.95395 | Female | Past | 10/25/1999 | T2N0 | African-American | | 64 | neoadjuvant | 4 |
| 128 | 11/30/2000 | Dead | 11.61184 | Male | Never | 12/13/1999 | not given | East Asian | | 77 | Neoadjuvant | |
| 130 | 5/21/2007 | Alive | 89.24342 | Female | Current | 12/16/1999 | T1/2N1 MX (at least) | Caucasian | 65 | 79 | Neoadjuvant | 1.8 |
| 139 | 5/21/2007 | Alive | 87.86184 | Female | Never | 1/27/2000 | T2N0 | Caucasian | | 82 | Neoadjuvant | 3.0 |
| 146 | 11/15/2001 | Dead | 21.01974 | Female | | 2/15/2000 | T2N1 | Caucasian | | 55 | Neoadjuvant | 5 |
| 222 | 12/16/2000 | Dead | 2.467105 | Female | Never | 10/2/2000 | T4N0M1 | Caucasian | 0 | 59 | Adjuvant | 1.3 |
| 231 | 5/25/2002 | Dead | 18.81579 | Male | Past | 10/30/2000 | M1 | Caucasian | | 58 | neoadjuvant | 8 |
| 235 | 8/14/2003 | Dead | 33.15789 | Female | Never | 11/9/2000 | T3N3M X | Caucasian | | 42 | neoadjuvant | 6 |
| 238 | 5/21/2007 | Alive | 78.05921 | Male | Current | 11/20/2000 | | African-American | | 57 | | 1.5 |
| 267 | 5/21/2007 | Alive | 74.60526 | Female | Current | 3/5/2001 | T2N0 | Other | | 82 | Neoadjuvant | 6 |
| 279 | 8/9/2002 | Dead | 16.25 | Male | Past | 4/2/2001 | T2N0 | Caucasian | | 71 | neoadjuvant | 6 |
| 284 | 12/23/2006 | Dead | 68.09211 | Female | Past | 4/23/2001 | T2N0 | Caucasian | | 58 | | 4 |
| 289 | 3/11/2003 | Dead | 22.07237 | Female | Past | 5/9/2001 | T1NXM X | Caucasian | | 77 | | 2 |
| 291 | 12/11/2003 | Dead | 31.08553 | Male | Current | 5/10/2001 | T2N0M X | Caucasian | 60 | 65 | | 1.9 |
| 294 | 10/23/2003 | Dead | 29.27632 | Male | Past | 5/16/2001 | T2N0 | Caucasian | | 78 | | 4.5 |
| 295 | 5/21/2007 | Alive | 72.20395 | Female | Past | 5/17/2001 | T1N0M X | Caucasian | | 82 | | 1.5 |
| 304 | 5/21/2007 | Alive | 71.71053 | Female | | 6/1/2001 | T2N2 | Caucasian | | 49 | Neoadjuvant | 5 |
| 309 | 5/21/2007 | Alive | 70.92105 | Female | Past | 6/25/2001 | T2N0 | Caucasian | | 72 | | 8 |
| 310 | 5/21/2007 | Alive | 70.92105 | Mate | Past | 6/25/2001 | T1N0 | African-American | | 66 | | 2.5 |
| 312 | 6/11/2005 | Dead | 47.53289 | Male | Current | 6/27/2001 | T2N0 | Caucasian | | 70 | neoadjuvant | 3.7 |
| 316 | 4/25/2005 | Dead | 45.59211 | Female | Past | 7/9/2001 | T2N0 | Caucasian | | 62 | Unrelated to Current Cancer | 6 |
| 327 | 5/21/2007 | Alive | 69.53947 | Fem ale | Past | 8/6/2001 | T2N2 | Caucasian | | 42 | neoadjuvant | 2 |
| 329 | 9/22/2004 | Dead | 37.36842 | Male | Current | 8/13/2001 | T1 | Caucasian | | 70 | | 2.5 |
| 333 | 11/12/2004 | Dead | 38.78289 | Female | Past | 8/21/2001 | T2N0 | Caucasian | | 70 | Unrelated to Current Cancer | 3.2 |
| 338 | 6/16/2005 | Dead | 44.93421 | Female | Never | 9/19/2001 | T3N2 | East Asian | 0 | 51 | neoadjuvant | 2.8 |
| 353 | 10/18/2002 | Dead | 11.77632 | Male | Current | 10/25/2001 | T2N2 | South east Asian | | 67 | neoadjuvant | 4.8 |
| 355 | 3/10/2007 | Dead | 64.375 | Male | Never | 10/30/2001 | T1N2M X | East Asian | 0 | 44 | adjuvant | 3 |
| 356 | 5/21/2007 | Alive | 66.7105 3 | Female | Never | 10/31/2001 | T1N0 | East Asian | | 65 | | 3 |
| 358 | 5/21/2007 | Alive | 66.44737 | Male | Past | 11/8/2001 | T2N1 | Caucasian | | 64 | neoadjuvant | 8 |
| 360 | 5/21/2007 | Alive | 66.25 | Female | Past | 11/14/2001 | T4N0 | Caucasian | | 72 | Unrelated to Current Cancer | 2 |
| 364 | 5/21/2007 | Alive | 66.01974 | Female | Past | 11/21/2001 | T2N0M X | Caucasian | | 69 | Unrelated to Current Cancer | 3.4 |
| 378 | 6/24/2003 | Dead | 17.20395 | Male | Current | 1/17/2002 | T1N0 | Caucasian | | 60 | | 1.5 |
| 381 | 3/11/2003 | Dead | 13.32237 | Male | Never | 1/30/2002 | T2N0 | Caucasian | 0 | 60 | | 12 |
| 382 | 5/21/2007 | Alive | 63.68421 | Female | Past | 1/31/2002 | T1N0 | Caucasian | | 79 | neoadjuvant | 2 |
| 408 | 10/12/2004 | Dead | 30.09868 | Female | Past | 4/11/2002 | T4N1 | Caucasian | | 73 | | 4 |
| 412 | 5/21/2007 | Alive | 61.01974 | Female | Past | 4/22/2002 | T1N0 | East Asian | | 78 | | 2.5 |
| 418 | 5/21/2007 | Alive | 60.32895 | Female | Current | 5/13/2002 | T2N0 | Caucasian | 80 | 62 | neoadjuvant | 3.2 |
| 420 | 8/30/2003 | Dead | 15.06579 | Female | Past | 5/29/2002 | T2N0 | Caucasian | | 76 | neoadjuvant | 4 |
| 424 | 5/21/2007 | Alive | 59.30921 | Female | Never | 6/13/2002 | T2N2 | Caucasian | 0 | 83 | Unrelated to Current Cancer | 5 |
| 426 | 5/2/2005 | Dead | 34.47368 | Male | Past | 6/19/2002 | T2N0 | Caucasian | | 82 | | 4 |
| 427 | 4/25/2004 | Dead | 22.23684 | Female | Current | 6/19/2002 | T4N0 | Caucasian | | 69 | | 3.5 |
| 428 | 5/21/2007 | Alive | 59.11184 | Male | Never | 6/19/2002 | T2N0 | South east Asian | | 65 | | 3.5 |
| 433 | 4/28/2004 | Dead | 21.94079 | Female | Past | 7/1/2002 | T2N0 | Caucasian | 50 | 70 | Unrelated to Current Cancer | 4 |
| 436 | 5/21/2007 | Alive | 58.19079 | Male | Current | 7/17/2002 | T3N0 | Caucasian | 5 0 | 65 | | 6.5 |
| 442 | 5/21/2007 | Alive | 57.10526 | Male | Past | 8/19/2002 | T1N0 | Hispanic | 20 | 70 | | 2.4 |
| 449 | 5/21/2007 | Alive | 56.31579 | Female | Past | 9/12/2002 | T1N0 | Decline to state | 5 | 64 | Unrelated to Current Cancer | 2.5 |
| 452 | 5/21/2007 | Alive | 55.88816 | Female | Past | 9/25/2002 | T4N2 | Caucasian | 15 | 53 | neoadjuvant | 4.9 |
| 454 | 5/21/2007 | Alive | 53.55263 | Female | Current | 12/5/2002 | T1N0 | Caucasian | 88 | 61 | Neoadjuvant | 2 |
| 470 | 5/21/2007 | Alive | 51.01974 | Male | Past | 2/20/2003 | T1N0 | Caucasian | 6 | 84 | neoadjuvant | 0.9 |
| 471 | 5/21/2007 | Alive | 50.65789 | Male | Past | 3/3/2003 | T4N0 | Caucasian | 18 | 50 | | 3.5 |
| 476 | 5/21/2007 | Alive | 50.13158 | Female | Past | 3/19/2003 | T2N0 | Caucasian | 25 | 72 | | 2.5 |
| 483 | 5/21/2007 | Alive | 49.67105 | Male | Past | 4/2/2003 | T1N0 | East Asian | 30 | 62 | Adjuvant | |
| 489 | 5/21/2007 | Alive | 48.78289 | Male | Never | 4/29/2003 | T1N0 | East Asian | 0 | 64 | | 1.3 |
| 494 | 6/20/2004 | Dead | 13.48684 | Female | Current | 5/7/2003 | T4N1M1 | Caucasian | 23 | 65 | | 2.5 |
| 496 | 5/21/2007 | Alive | 48.28947 | Female | Past | 5/14/2003 | T2N0 | Caucasian | 20 | 85 | | 4 |
| 497 | 5/21/2007 | Alive | 48.28947 | Male | Current | 5/14/2003 | T2NX | Caucasian | 58 | 77 | Adjuvant | 3.5 |
| 506 | 5/21/2007 | Alive | 47.79605 | Male | Past | 5/29/2003 | T1NX | Caucasian | 100 | 81 | | 2.9 |
| 511 | 5/21/2007 | Alive | 47.40132 | Female | Never | 6/10/2003 | T2N0 | South Asian | 0 | 61 | neoadjuvant | 3.5 |
| 513 | 5/3/2006 | Dead | 34.73684 | Female | Current | 6/12/2003 | T2N1 | Caucasian | 50 | 66 | Adjuvant | 6 |
| 516 | 5/21/2007 | Alive | 47.13816 | Female | Never | 6/18/2003 | T2N1 | Caucasian | 0 | 58 | Adjuvant | 3.5 |
| 520 | 8/30/2006 | Dead | 37.73026 | Male | Current | 7/10/2003 | T1N0M X | Caucasian | 80 | 61 | | 2.5 |
| 525 | 5/21/2007 | Alive | 45.95395 | Female | Past | 7/24/2003 | T1N0 vs M1 | Caucasian | 4 5 | 67 | | 2.5 |
| 526 | 5/21/2007 | Alive | 45.82237 | Female | Past | 7/28/2003 | T1N0 | Caucasian | 22 | 74 | | 1.8 |
| 527 | 5/21/2007 | Alive | 45.82237 | Female | Past | 7/28/2003 | T2NXM X | Caucasian | | 69 | | 4.5 |
| 535 | 5/9/2005 | Dead | 20.69079 | Female | Current | 8/19/2003 | T2N0 | Caucasian | | 84 | | 3.5 |
| 544 | 5/21/2007 | Alive | 44.21053 | Female | Past | 9/15/2003 | T2N0 | Middle Easter n | 5 | 69 | neoadjuvant | 4 |
| 556 | 5/21/2007 | Alive | 43.51974 | Male | Never | 10/6/2003 | T1N0 | South east Asian | | 67 | | 3.0 |
| 559 | 5/21/2007 | Alive | 43.22368 | Male | Past | 10/15/2003 | T1N0 | Caucasian | | 76 | | 2 |
| 565 | 3/5/2005 | Dead | 15.98684 | Male | Past | 11/5/2003 | T4NXM X | Caucasian | | 73 | Unrelated to Current Cancer | 2.6 |
| 575 | 5/21/2007 | Alive | 41.84211 | Female | Past | 11/26/2003 | T1N0 | Caucasian | 60 | 73 | | 1.5 |
| 583 | 5/21/2007 | Alive | 40.98684 | Female | Past | 12/22/2003 | T1N0 | Caucasian | 15 | 62 | Neoadjuvant | 2.5 |
| 585 | 10/7/2007 | Dead | 45.3289 5 | Male | Past | 12/29/2003 | T2N2 | Caucasian | 50 | 79 | Neoadjuvant | 1.4 |
| 607 | 2/7/2006 | Dead | 22.73026 | Female | Past | 3/18/2004 | T1N0 | Caucasian | | 64 | Unrelated to Current Cancer | 2.5 |
| 611 | 5/3/2006 | Dead | 24.70395 | Female | Current | 4/12/2004 | T2N0 | Caucasian | | 67 | Adjuvant | 4 |
| 613 | 11/25/2004 | Dead | 7.171053 | Male | Past | 4/21/2004 | T4N0 | Caucasian | | 76 | | 2.2 |
| 614 | 5/21/2007 | Alive | 37.00658 | Female | Past | 4/21/2004 | T2N0 | Caucasian | | 78 | | 3.2 |
| 621 | 4/16/2007 | Dead | 35.23026 | Female | | 5/10/2004 | M1 | Caucasian | | 88 | | 6 |
| 626 | 5/21/2007 | Alive | 36.08553 | Female | Current | 5/19/2004 | T1N0 | Caucasian | | 67 | | 2.5 |
| 628 | 4/7/2005 | Dead | 10.49342 | Female | Current | 5/23/2004 | T2N0 | Caucasian | | 62 | Adjuvant | 6 |
| 629 | 5/21/2007 | Alive | 35.92105 | Female | Past | 5/24/2004 | T1N1 | Caucasian | | 79 | | 2.5 |
| 680 | 12/11/2005 | Dead | 13.68421 | Male | Past | 10/21/2004 | T2N0 | Caucasian | | 75 | | 3.1 |
| 701 | 10/2/2005 | Dead | 8.815789 | Female | Past | 1/7/2005 | T1N0 | Caucasian | 25 | 84 | | 2.5 |
| 753 | 6/21/2005 | Dead | 0.263158 | Male | Past | 6/13/2005 | T2N0 | Caucasian | 20 | 81 | Neoadjuvant | 9 |
| 9705 | 11/14/2000 | Dead | 39.63816 | Female | Never | 7/28/1997 | T3N0 | South east Asian | 0 | 74 | | 13 |
| 9739 | 5/21/2007 | Alive | 113.75 | Female | Never | 12/1/1997 | T2N0 | Caucasian | 0 | 85 | | 3.5 |
| 9747 | 5/21/2007 | Alive | 113.2895 | Female | Past | 12/15/1997 | T1N0 | African Americ an | 4 | 72 | | 2.5 |
| 9801 | 9/16/2006 | Dead | 104.2105 | Female | Never | 1/13/1998 | T1N0 | Caucasian | 0 | 86 | | 2.5 |
| 9803 | 6/3/2002 | Dead | 52.43421 | Female | Current | 1/21/1998 | T2N0 | Caucasian | | 83 | | 2.5 |
| 9843 | 2/27/1999 | Dead | 9.605263 | Male | Past | 5/11/1998 | T2N2 | Caucasian | 120 | 74 | adjuvant | 4.5 |
| 9855 | 7/1/2006 | Dead | 96.80921 | Male | Never | 6/10/1998 | T2N2 | Caucasian | | 51 | Neoadjuvant | 3.5 |
| 9875 | 7/15/2002 | Dead | 47.13816 | Female | Current | 8/12/1998 | T2N1 | Caucasian | | 58 | | 2.5 |
| 9890 | 5/21/2007 | Alive | 103.75 | Female | Past | 10/1/1998 | T2N0 | Caucasian | | 72 | neoadjuvant | 3.4 |
| 9891 | 5/21/2007 | Alive | 103.5855 | Male | Past | 10/6/1998 | T2N1 | Caucasian | | 65 | Neoadjuvant | |
| 9896 | 5/31/2000 | Dead | 19.40789 | Female | Current | 10/19/1998 | T1/2N0 (depend s if RLL primary or met) | Caucasian | 70 | 68 | | 1.5 |
| 9908 | 5/21/2007 | Alive | 102.1711 | Male | Current | 11/18/1998 | T2N0 | East Asian | | 51 | | 5 |
| 9913 | 4/1/1999 | Dead | 4.013158 | Male | Current | 11/30/1998 | T1N1 | East Asian | | 70 | | 1.5 |

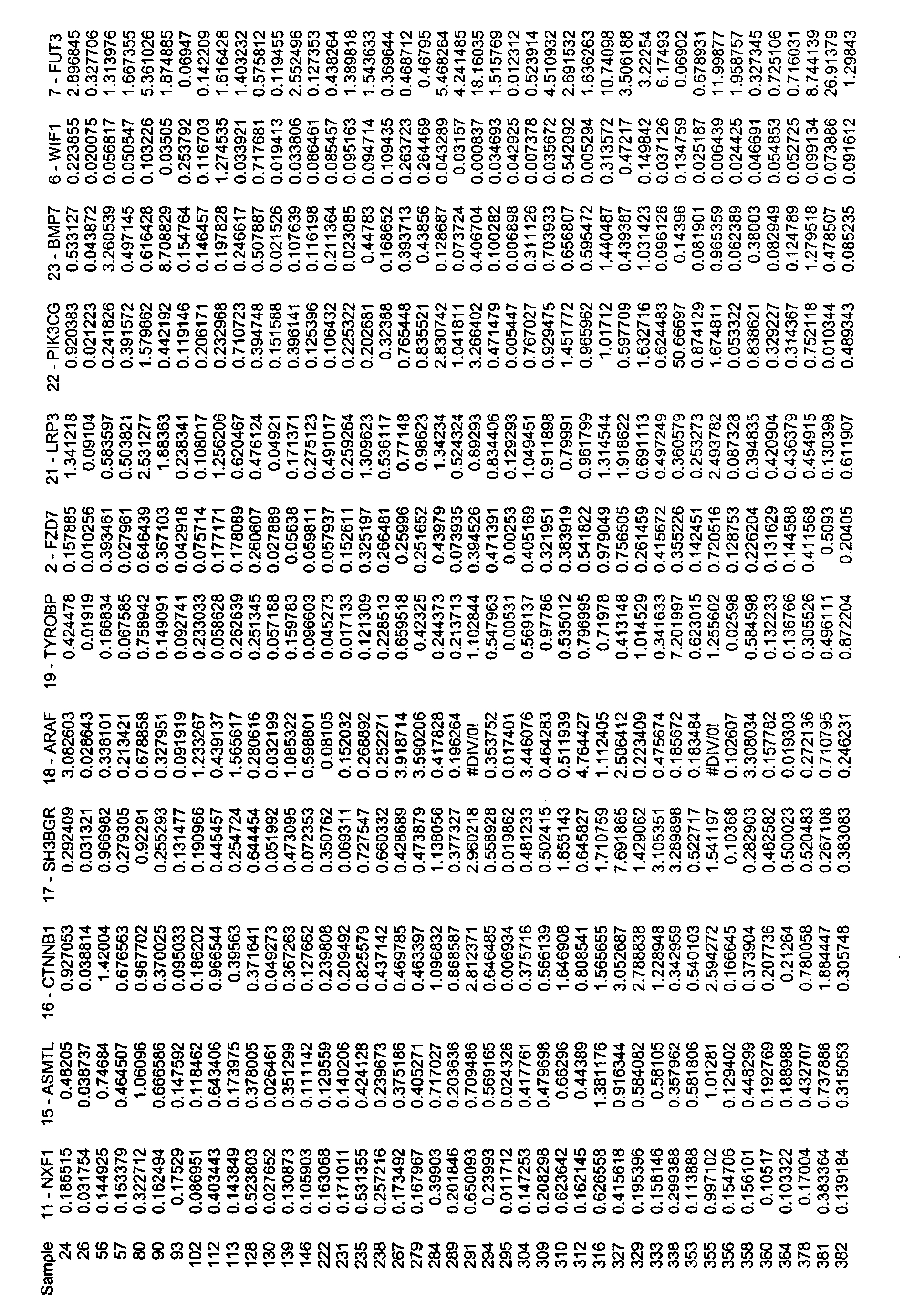

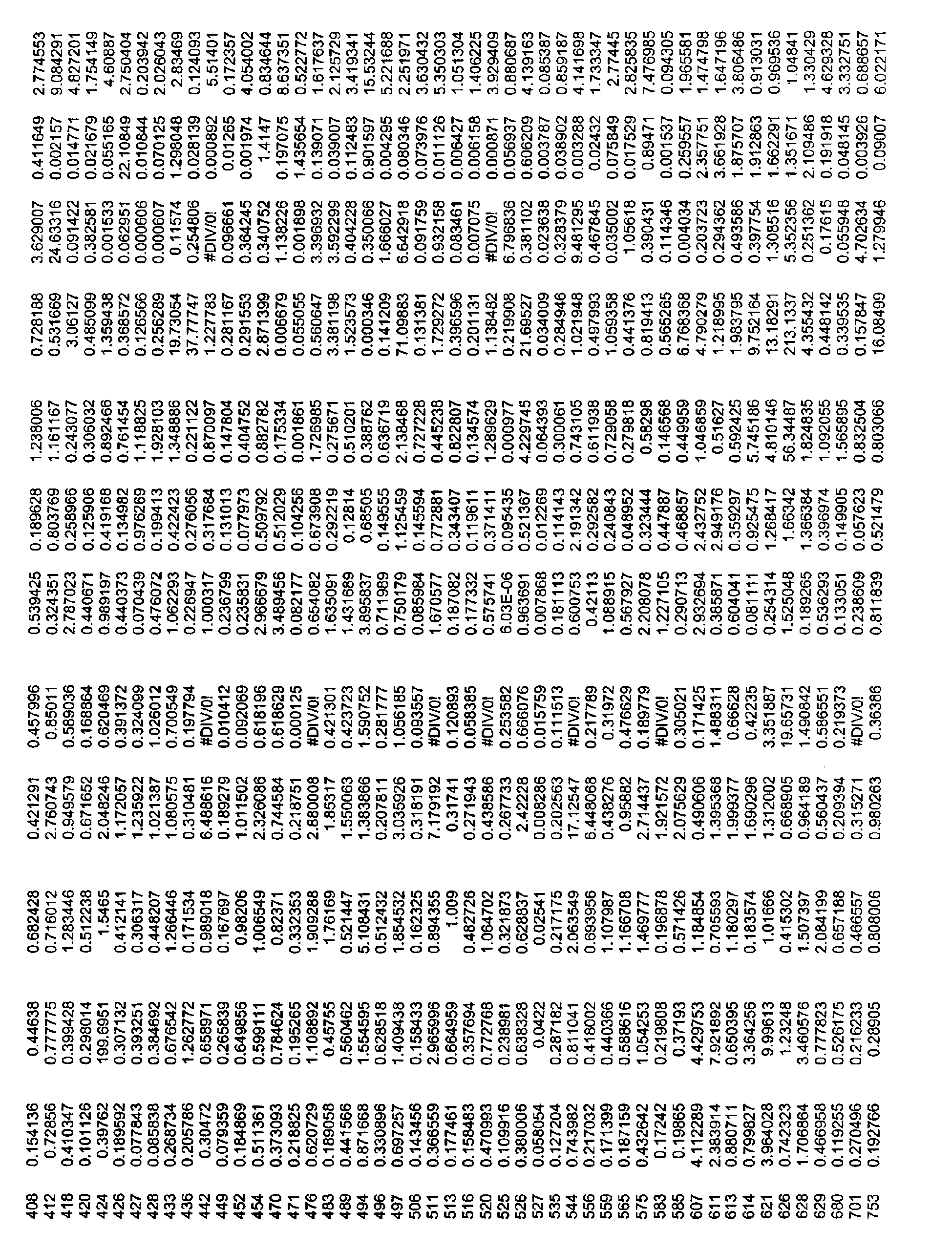

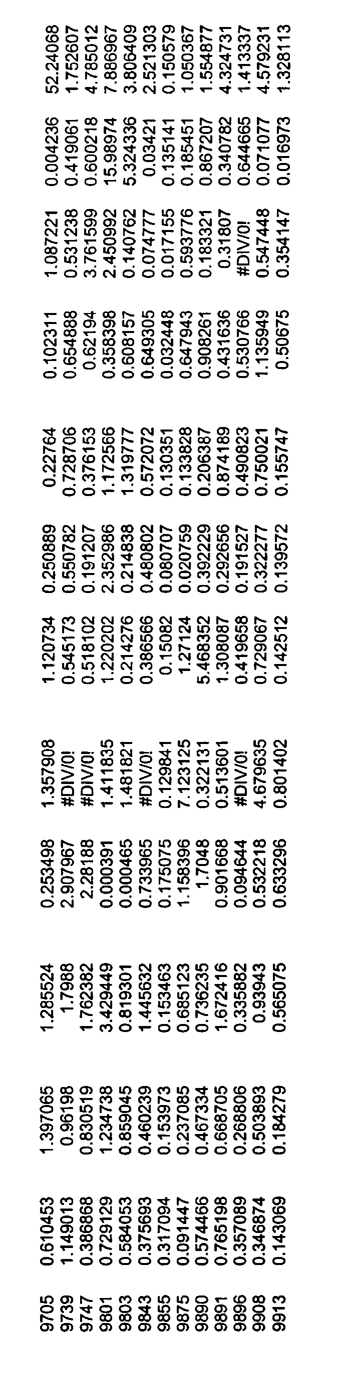

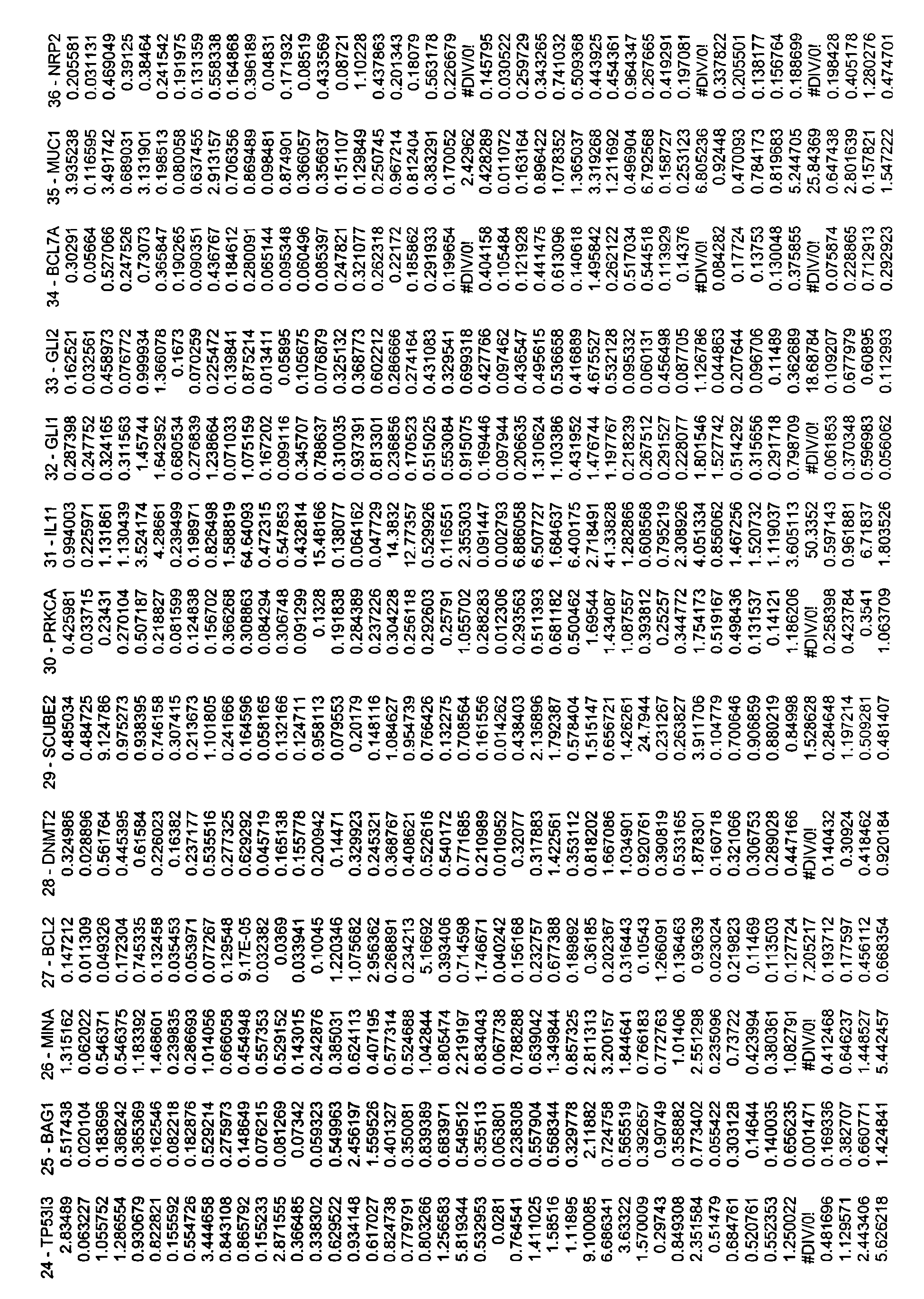

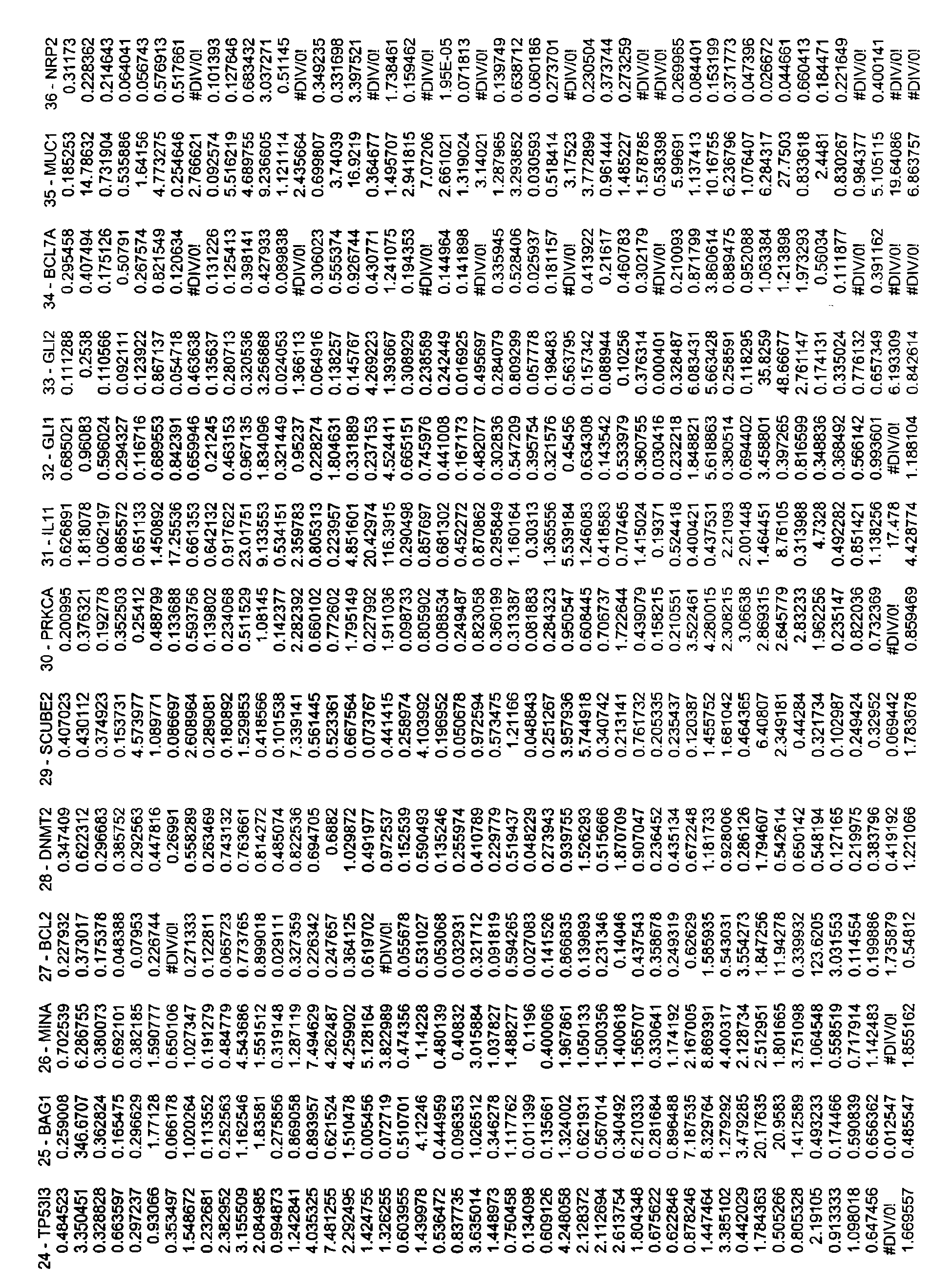

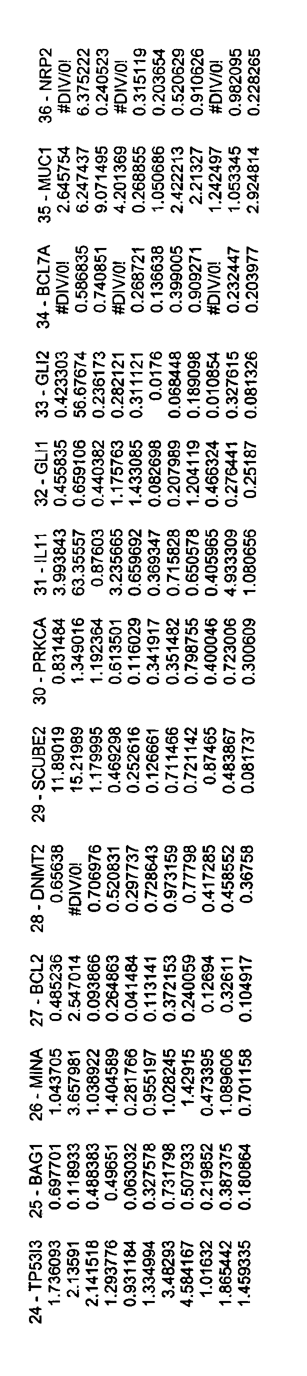

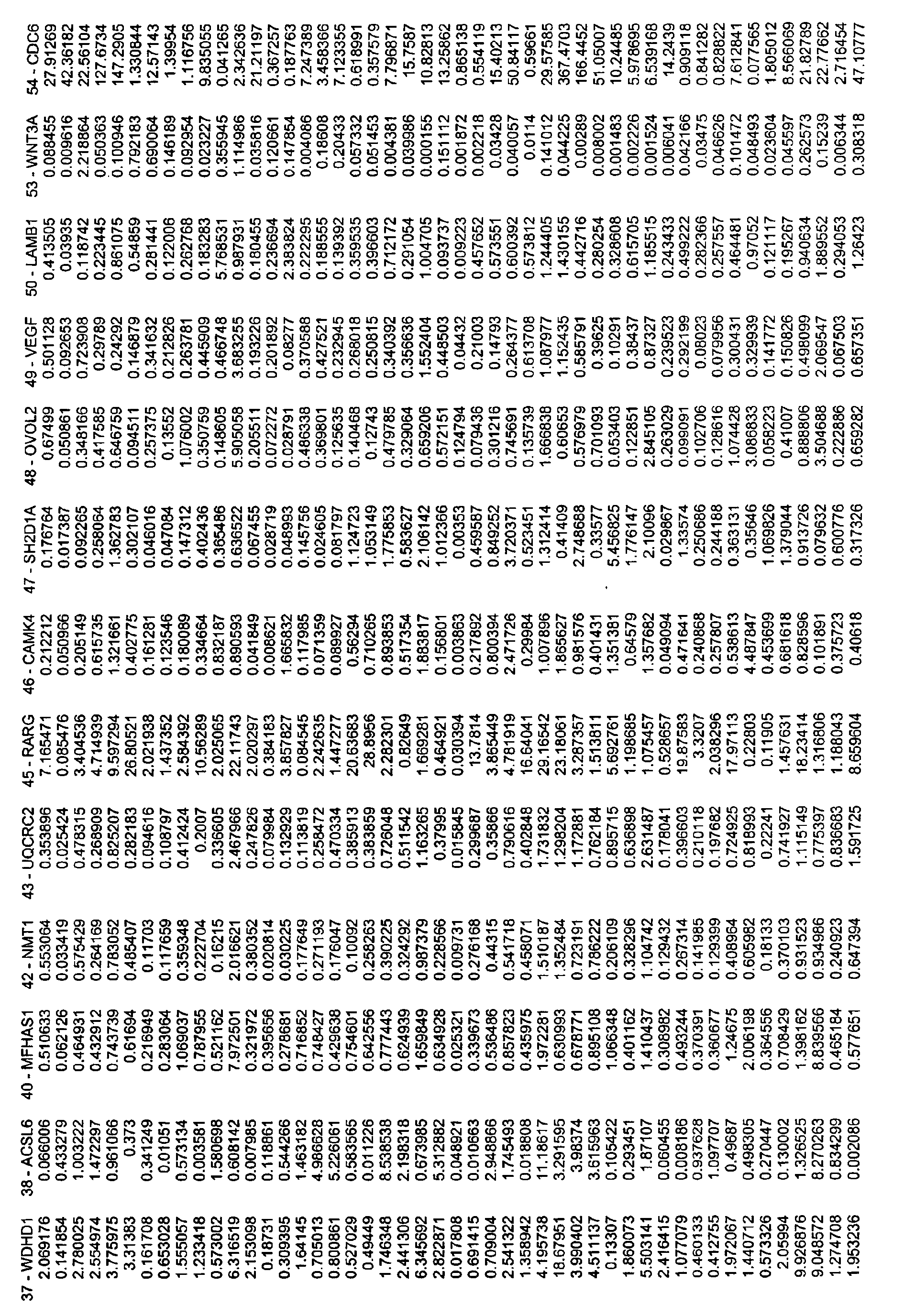

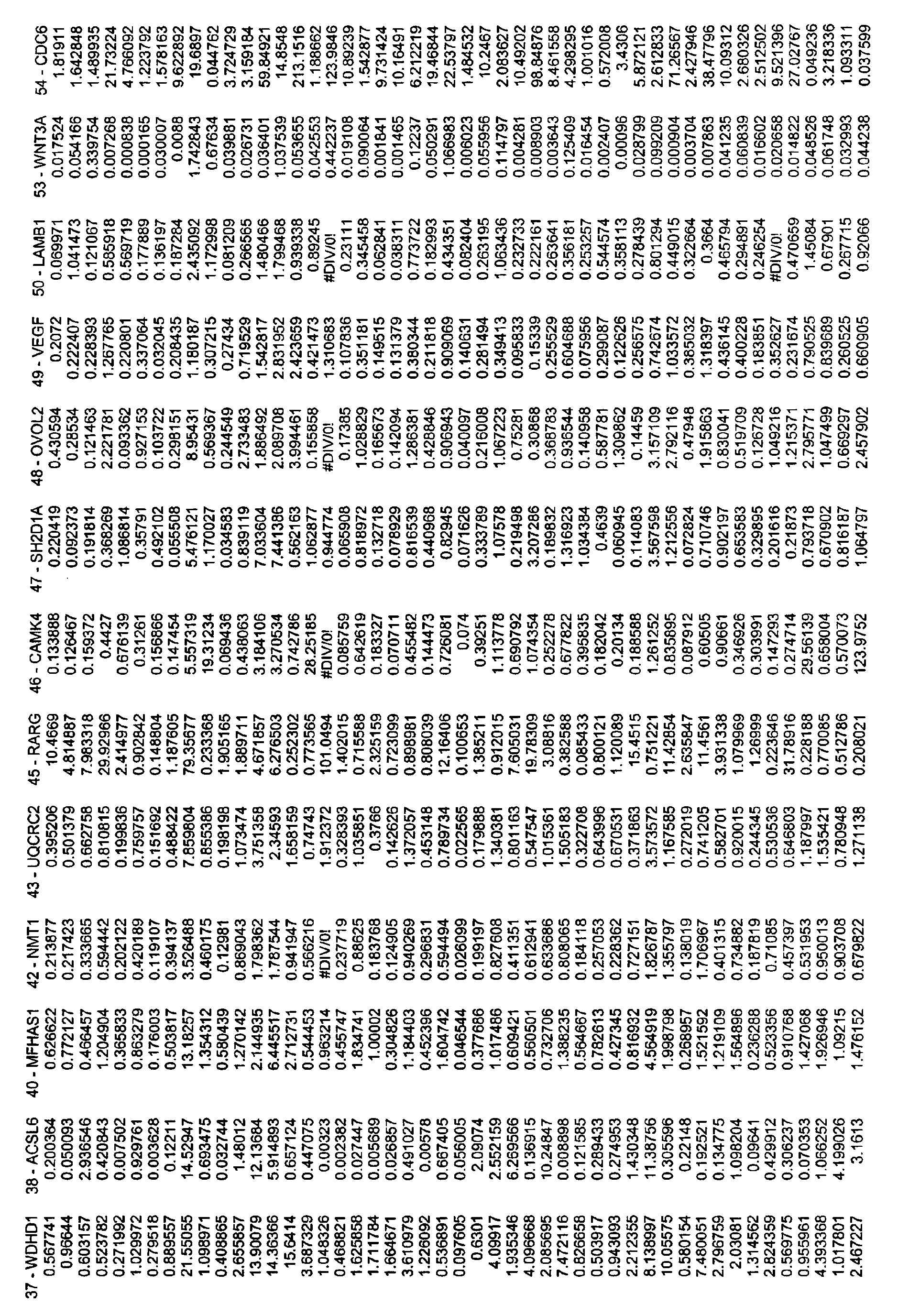

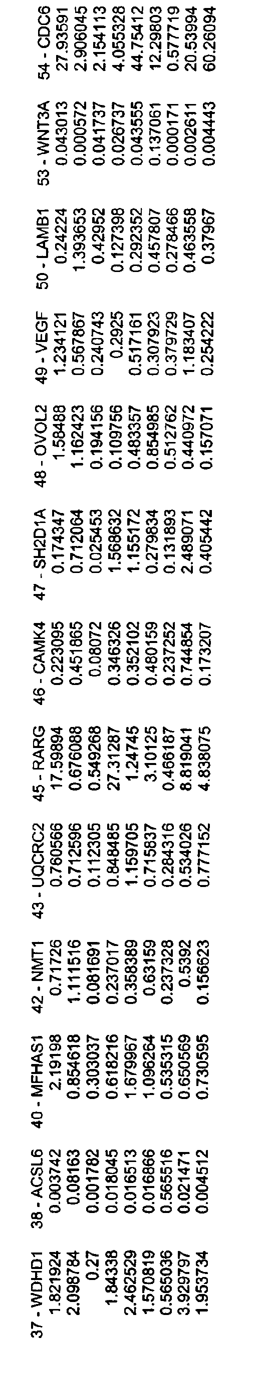

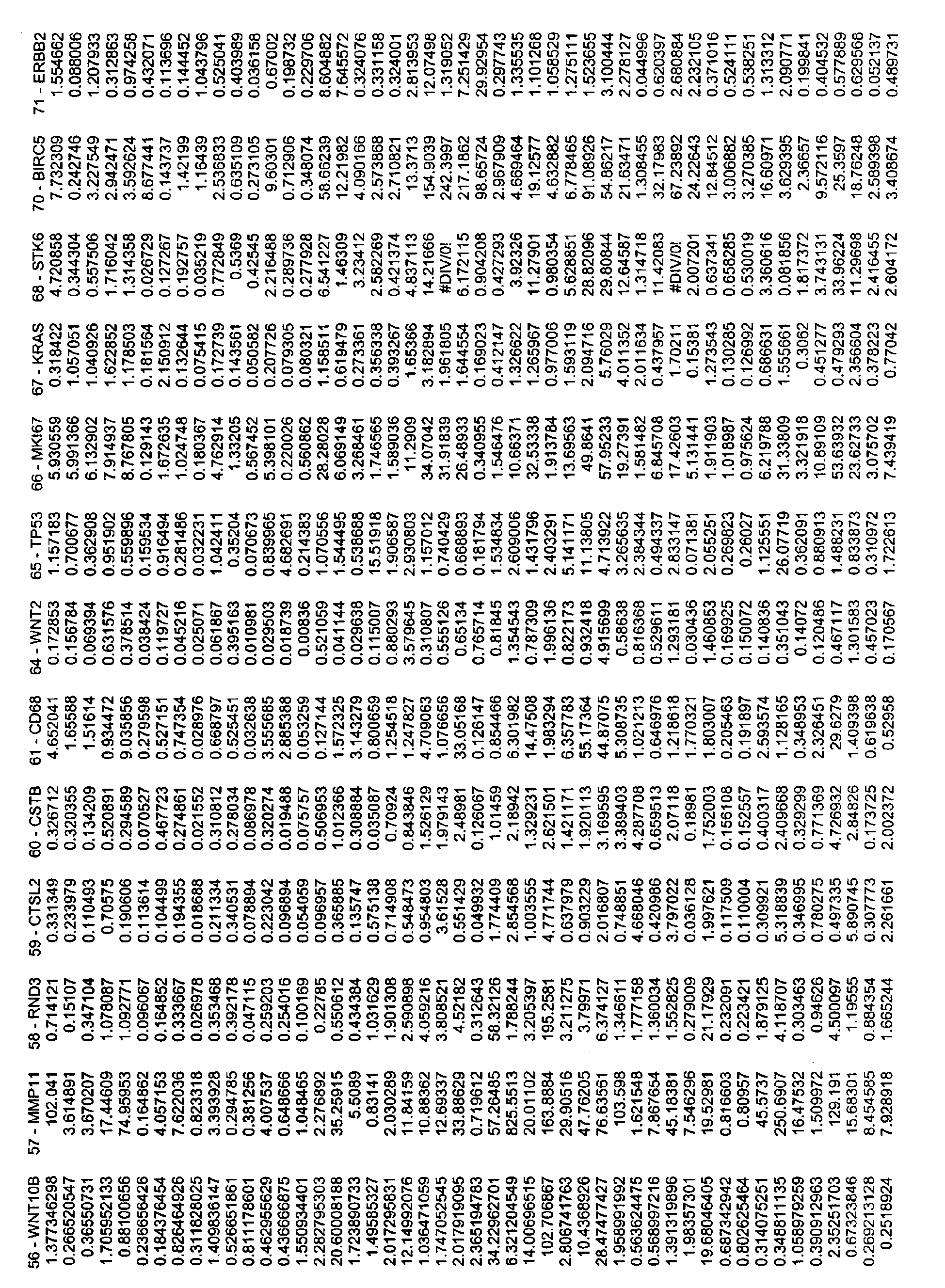

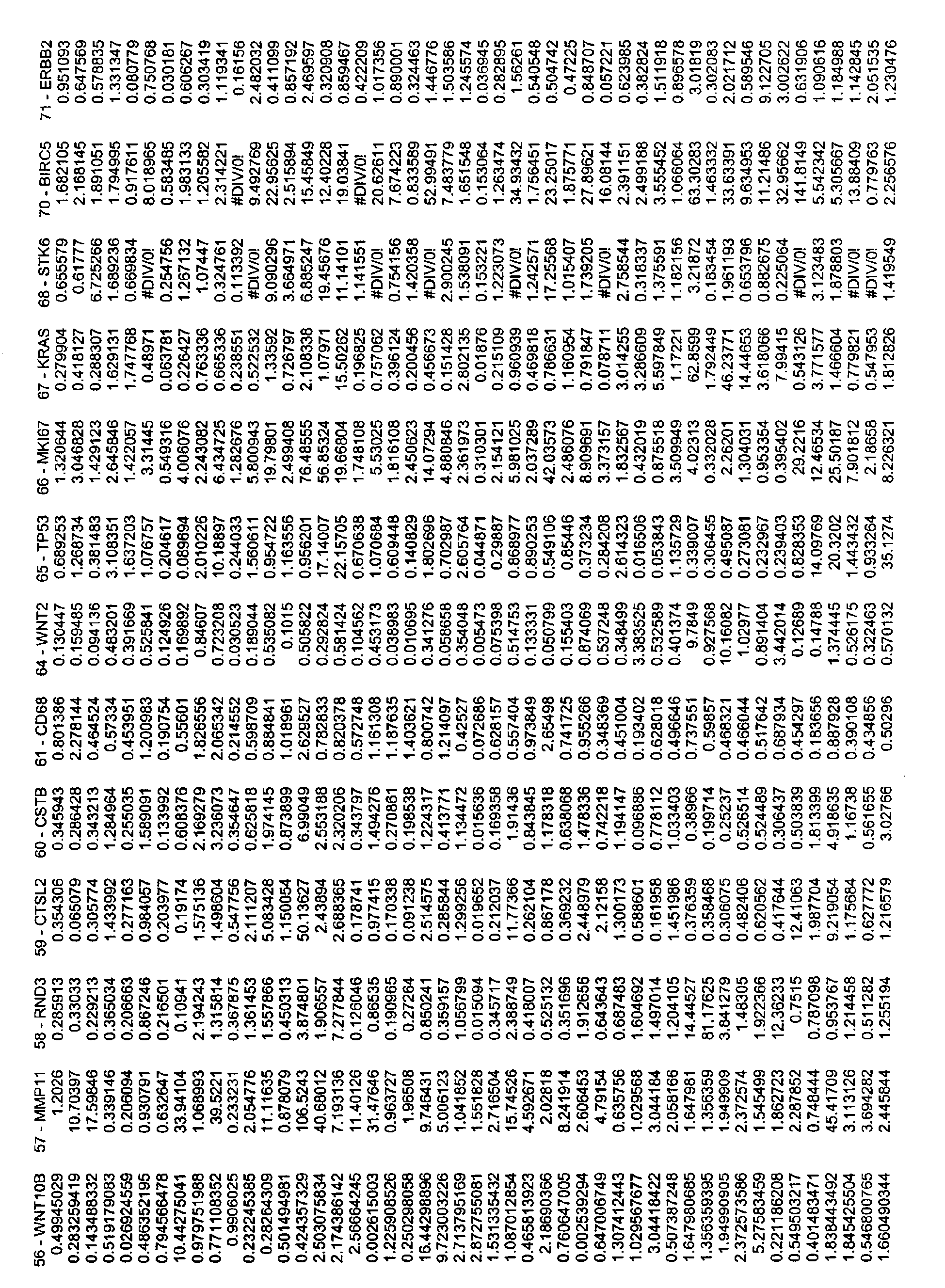

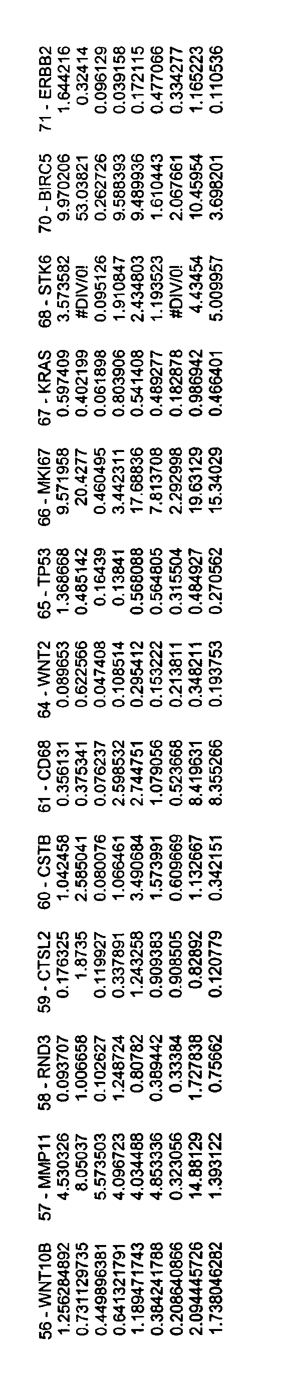

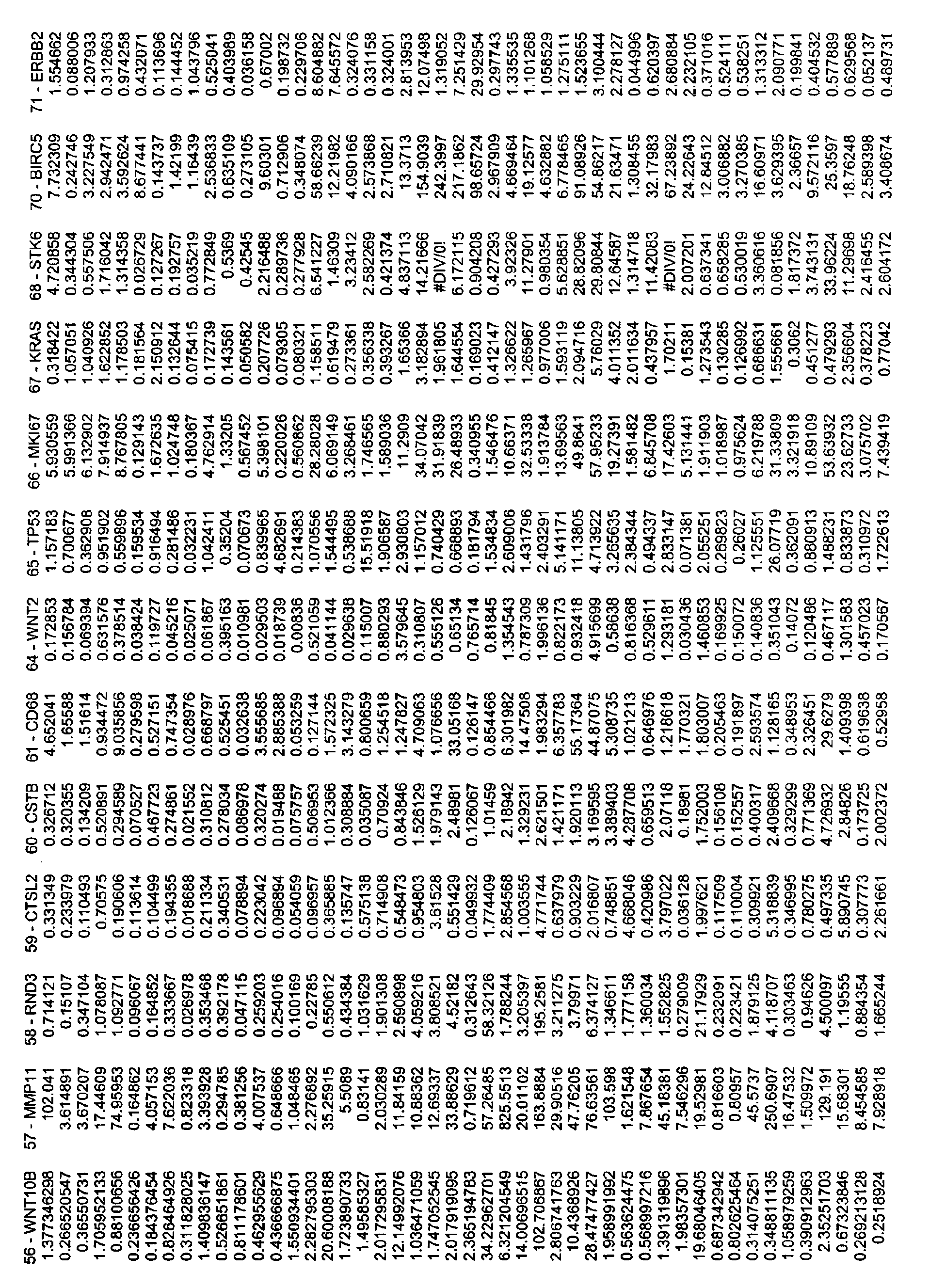

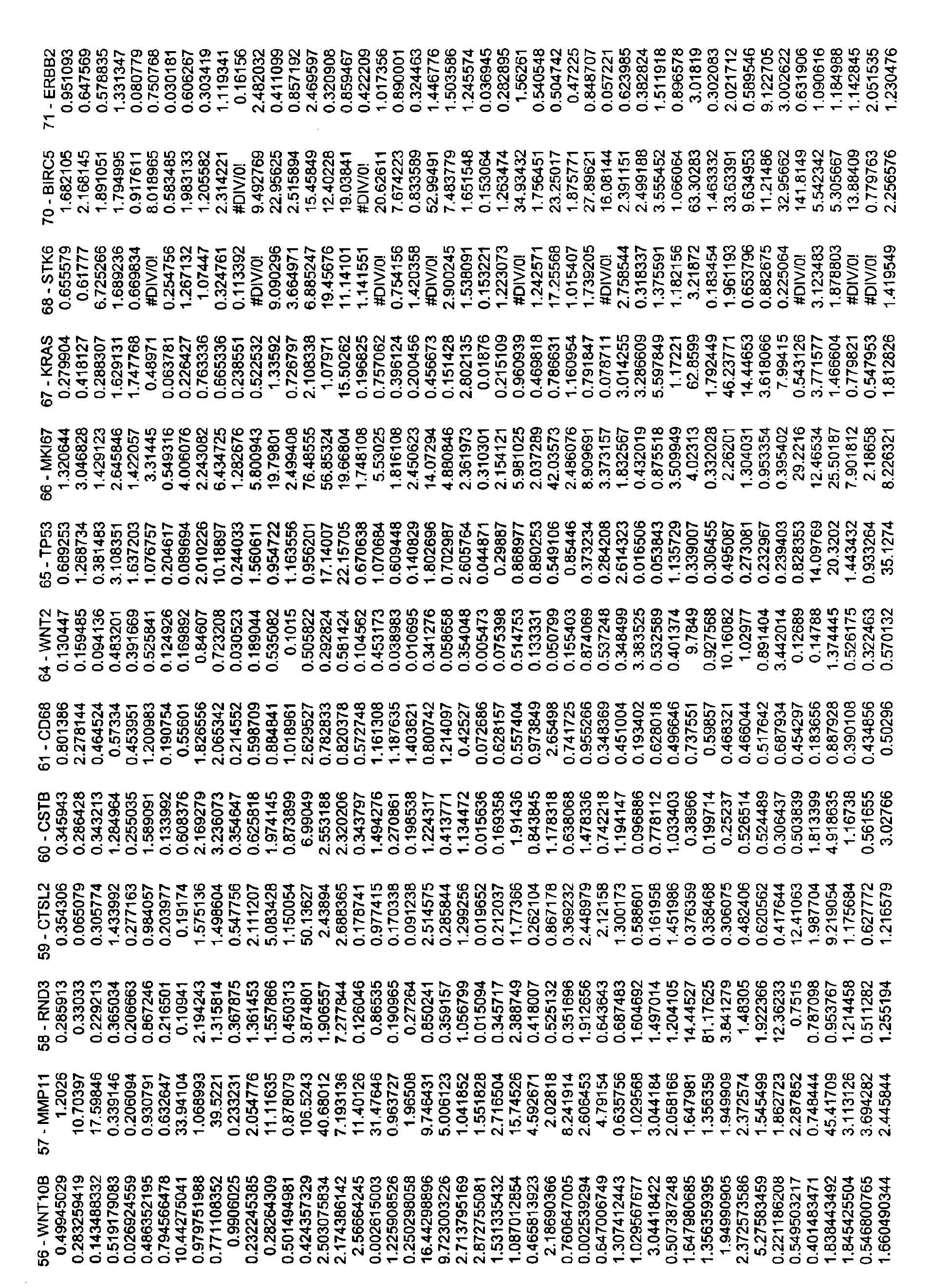

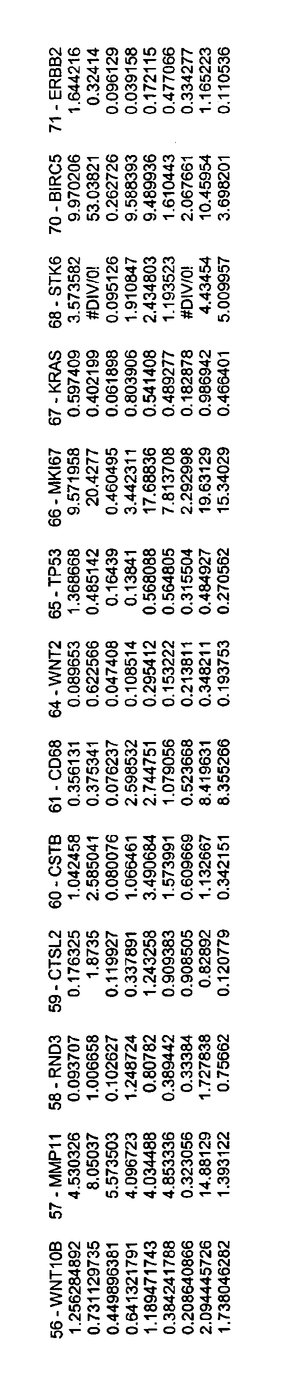

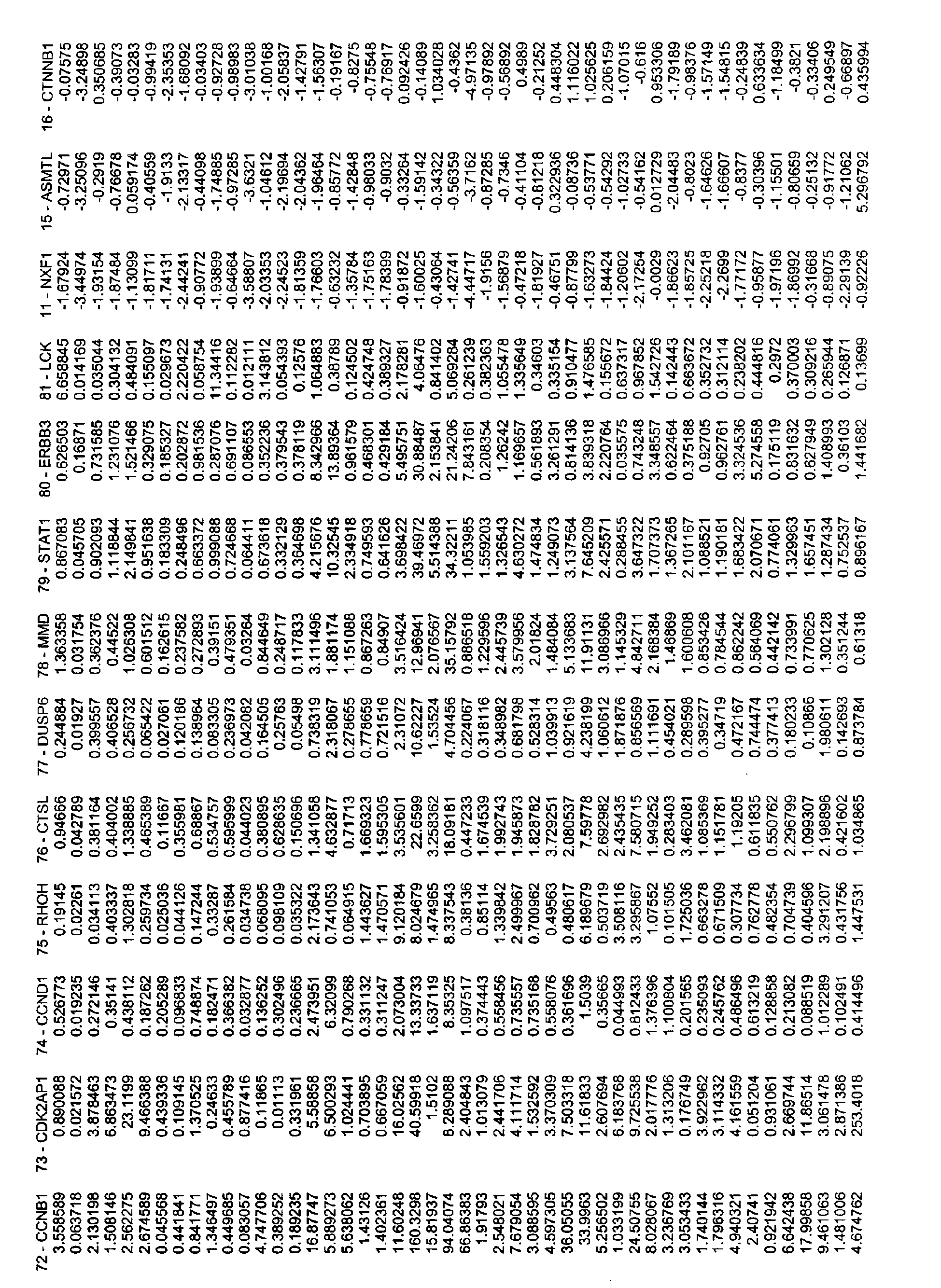

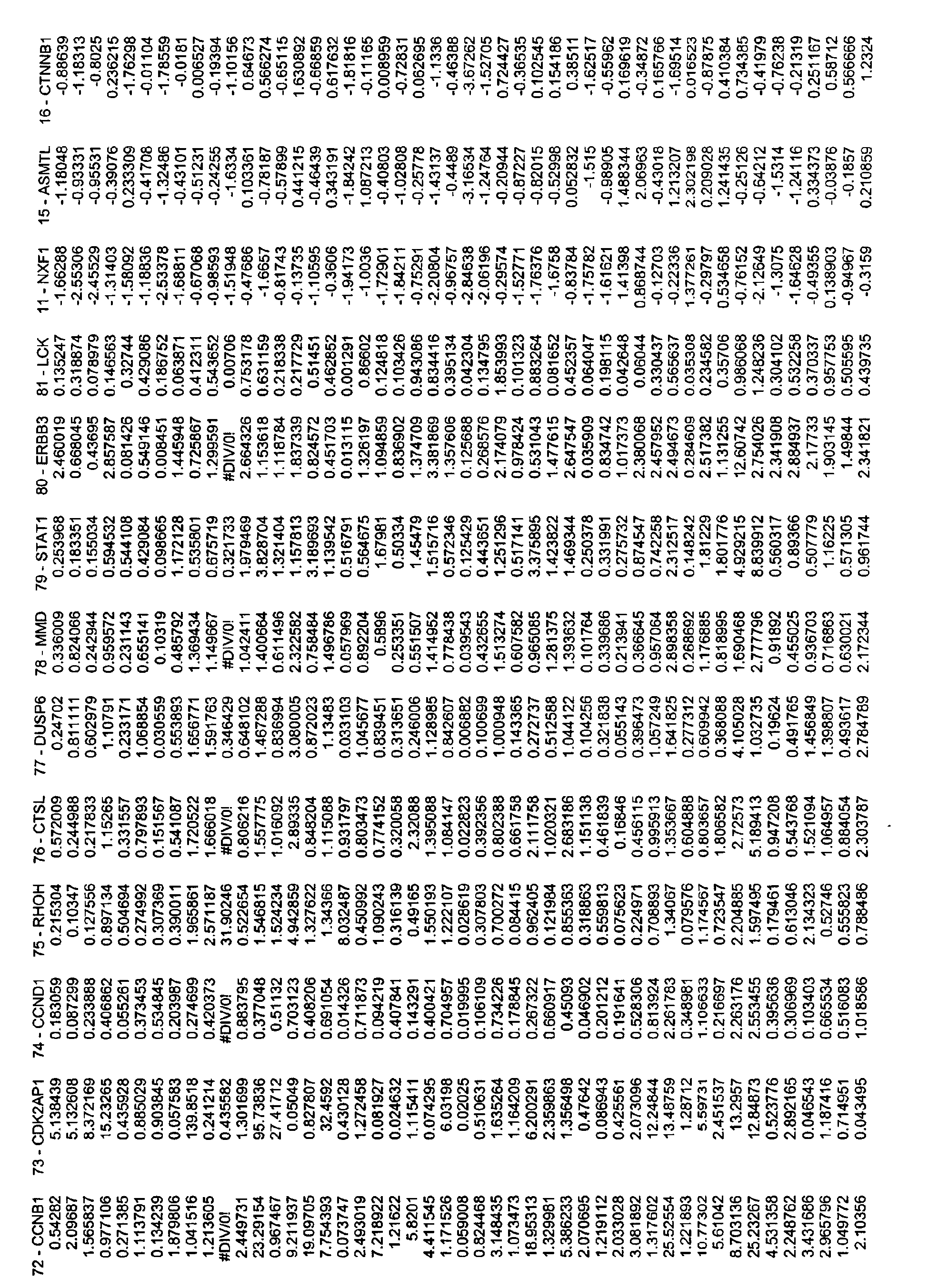

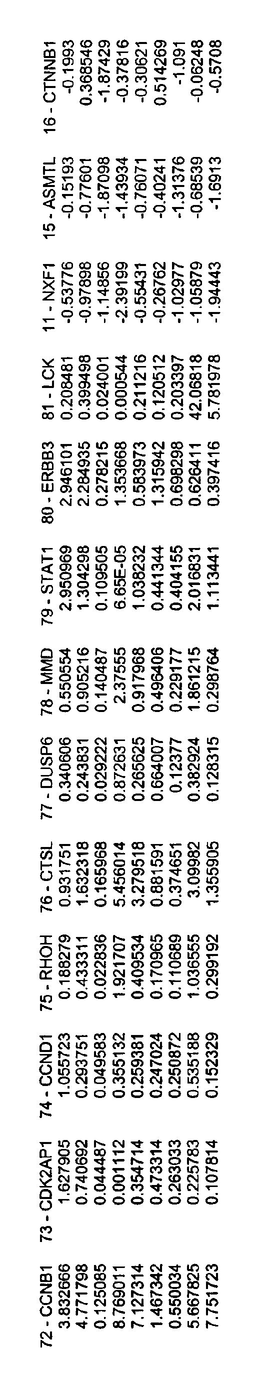

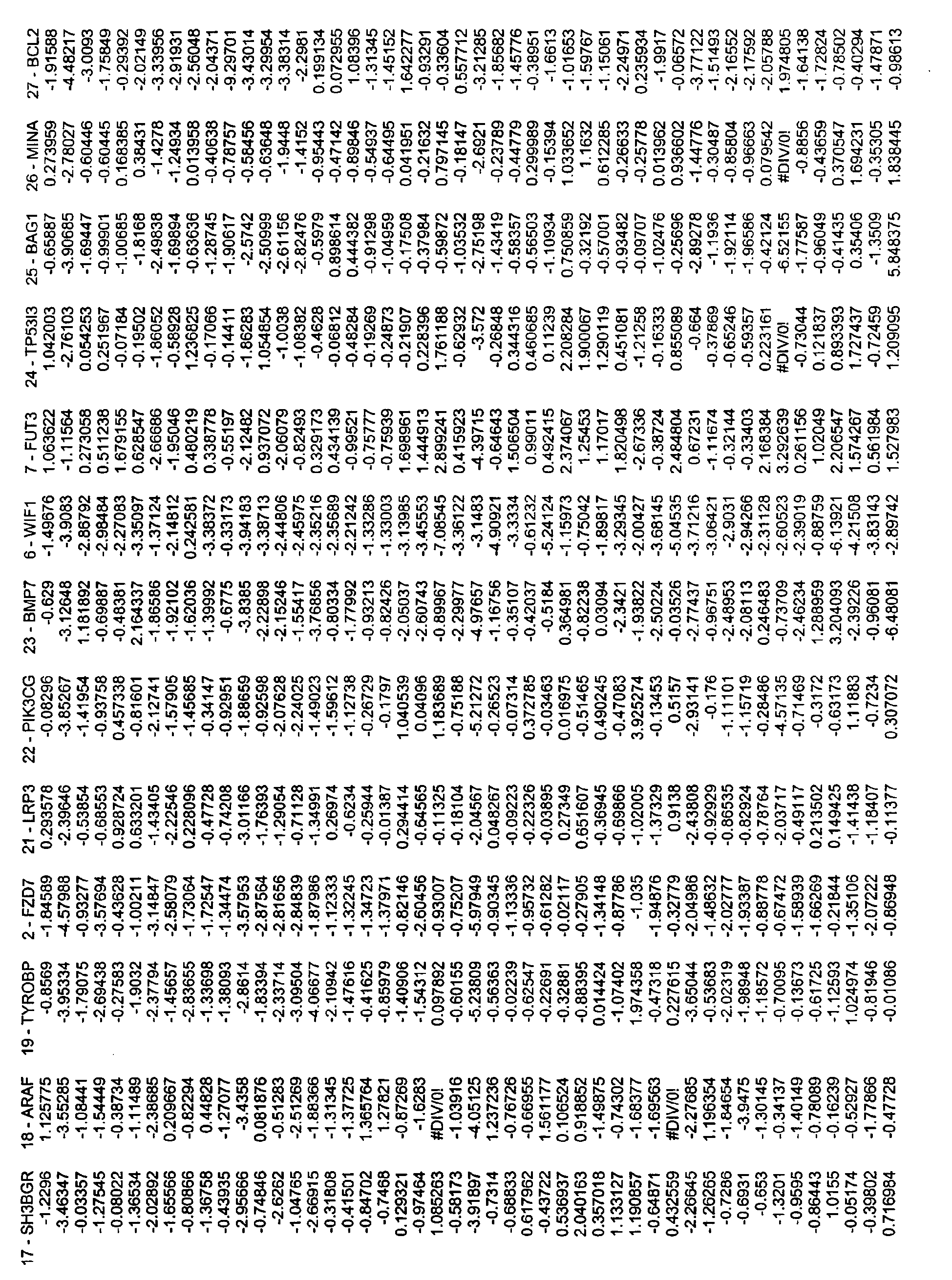

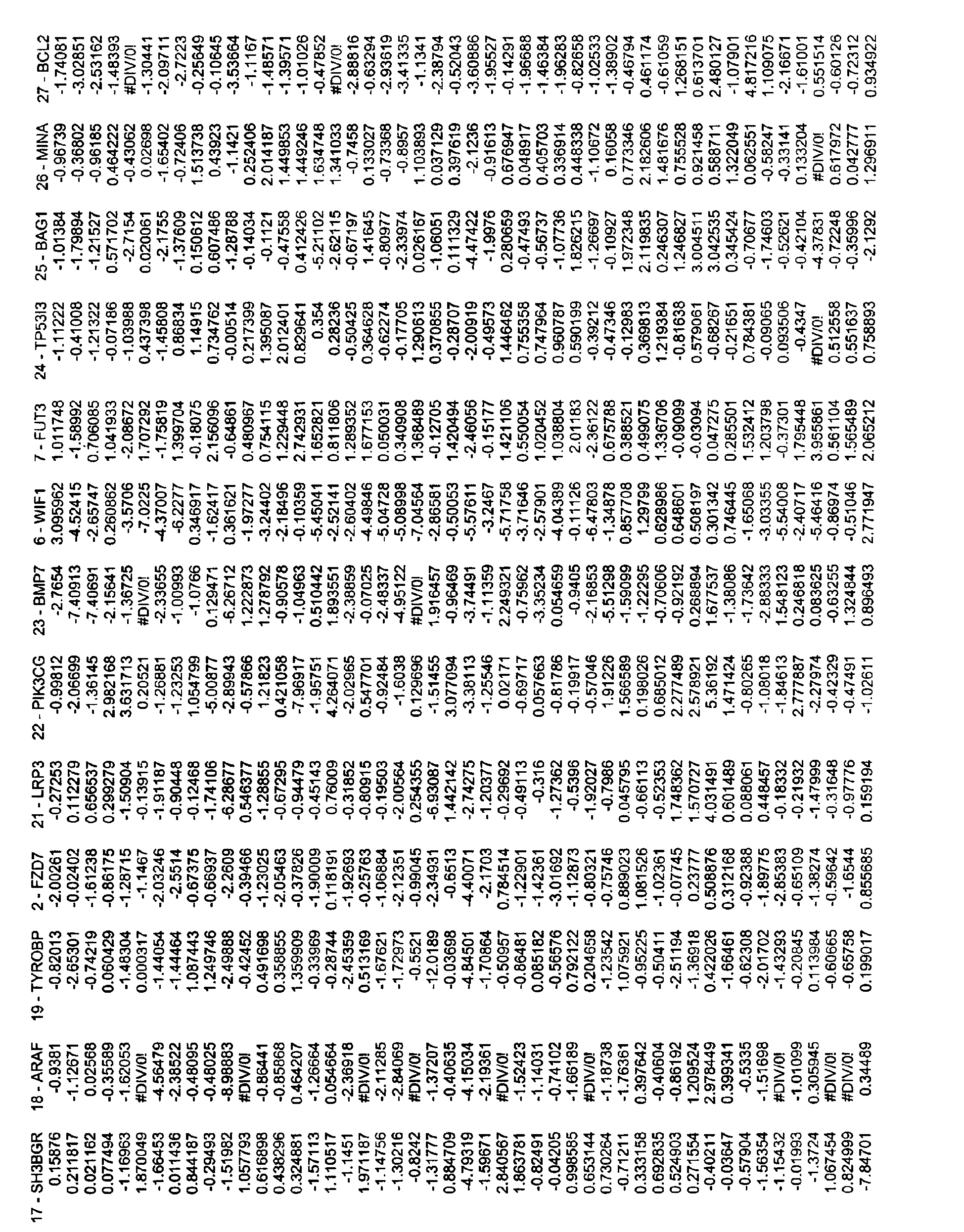

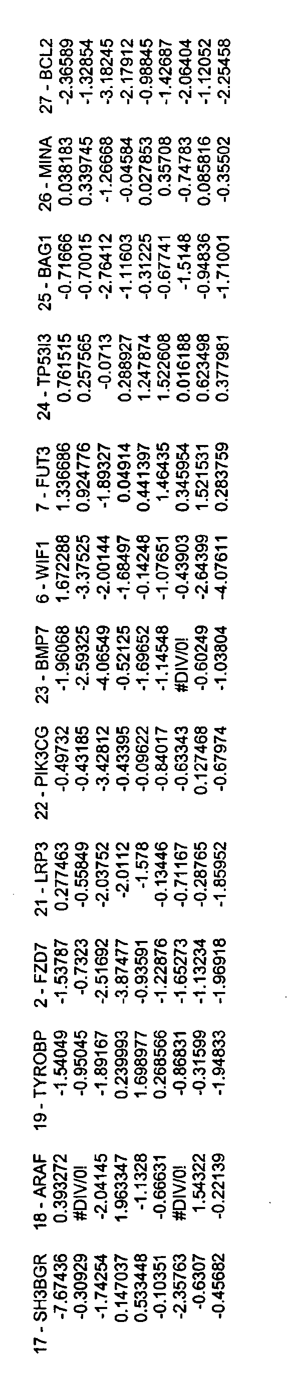

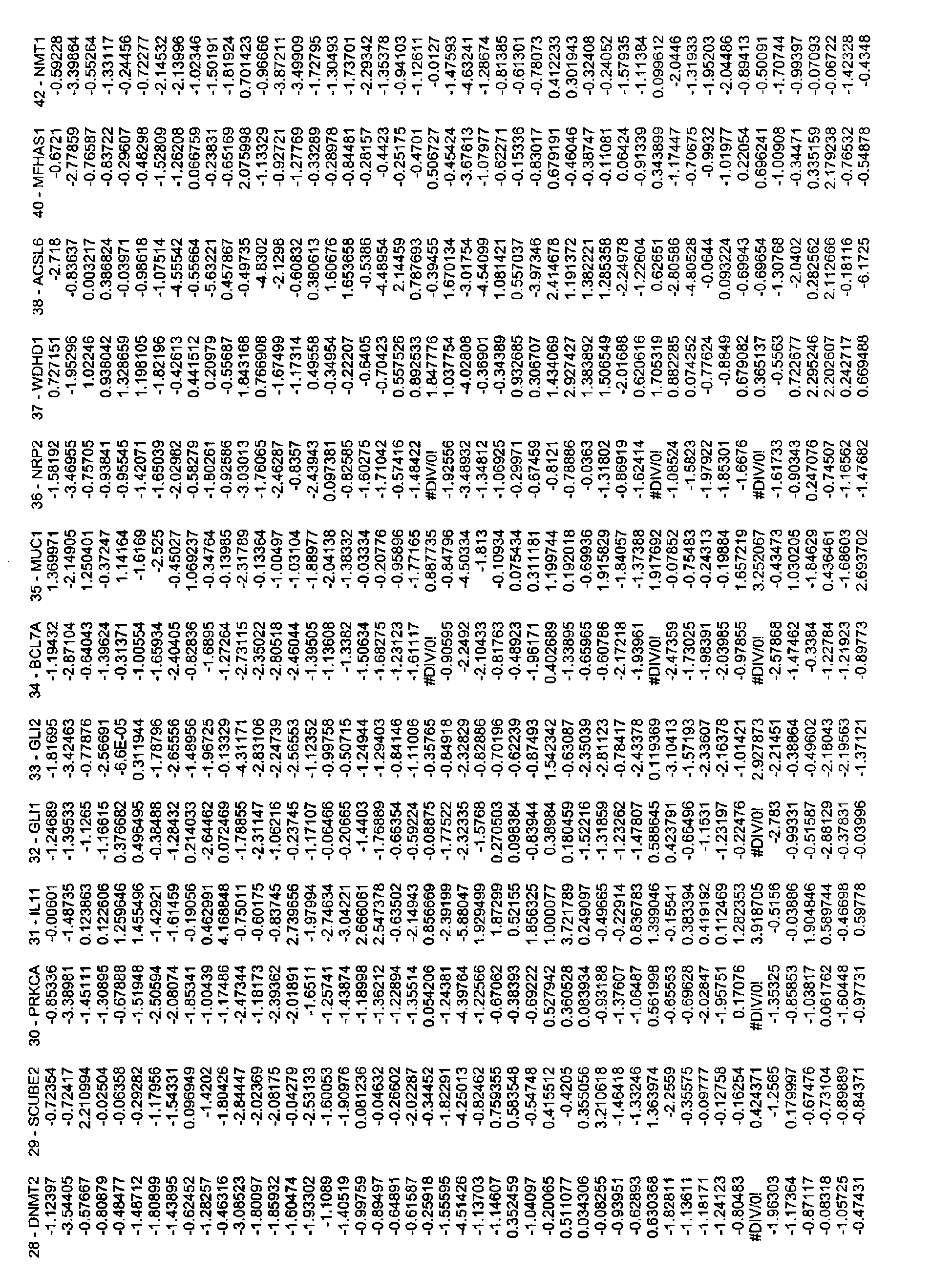

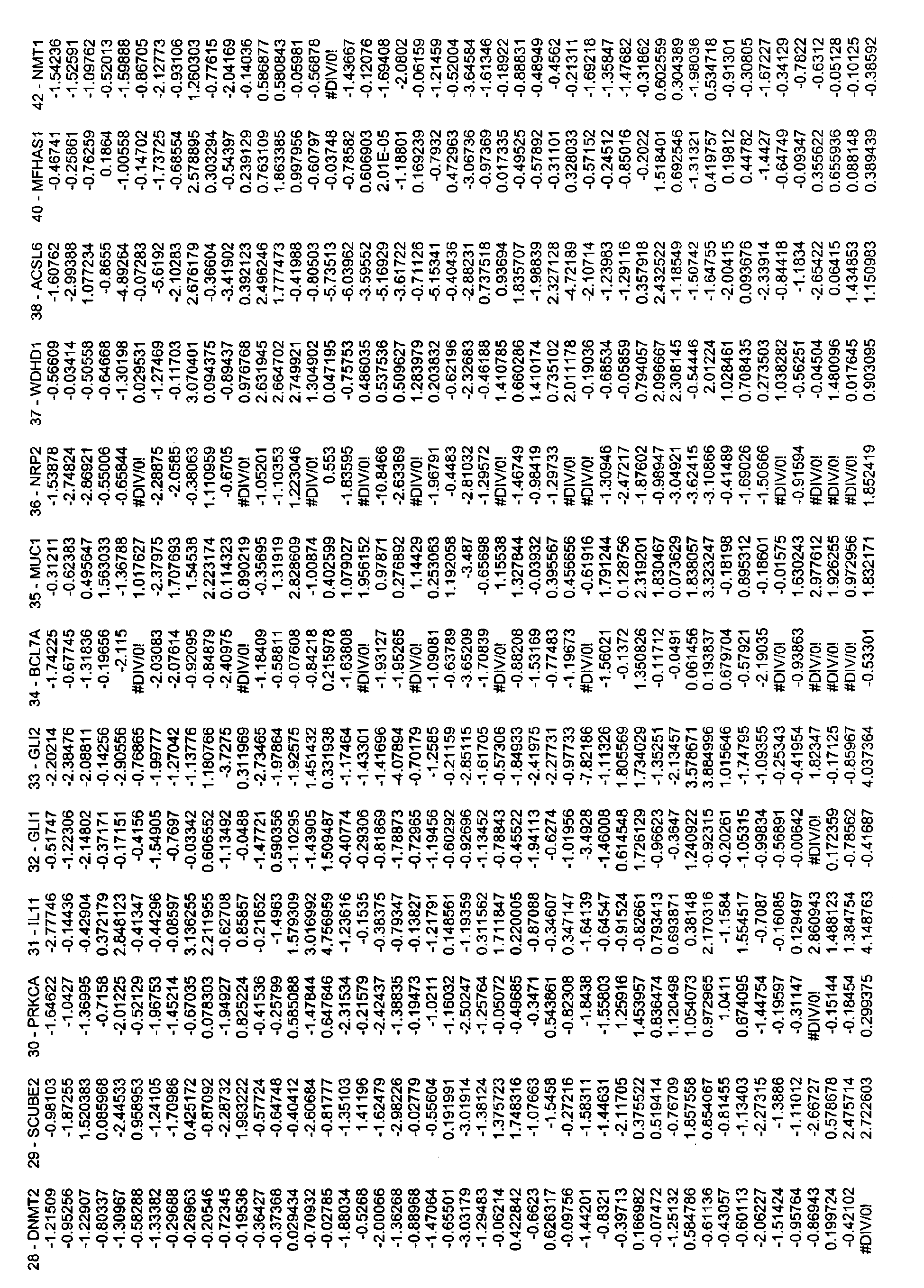

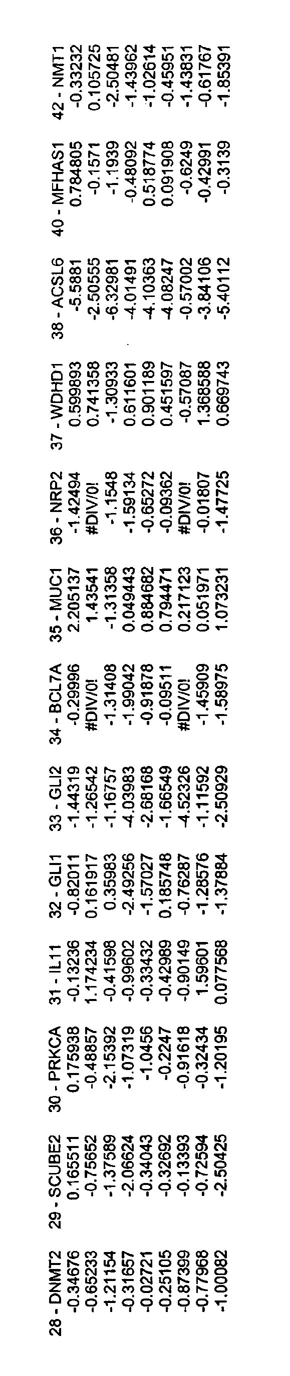

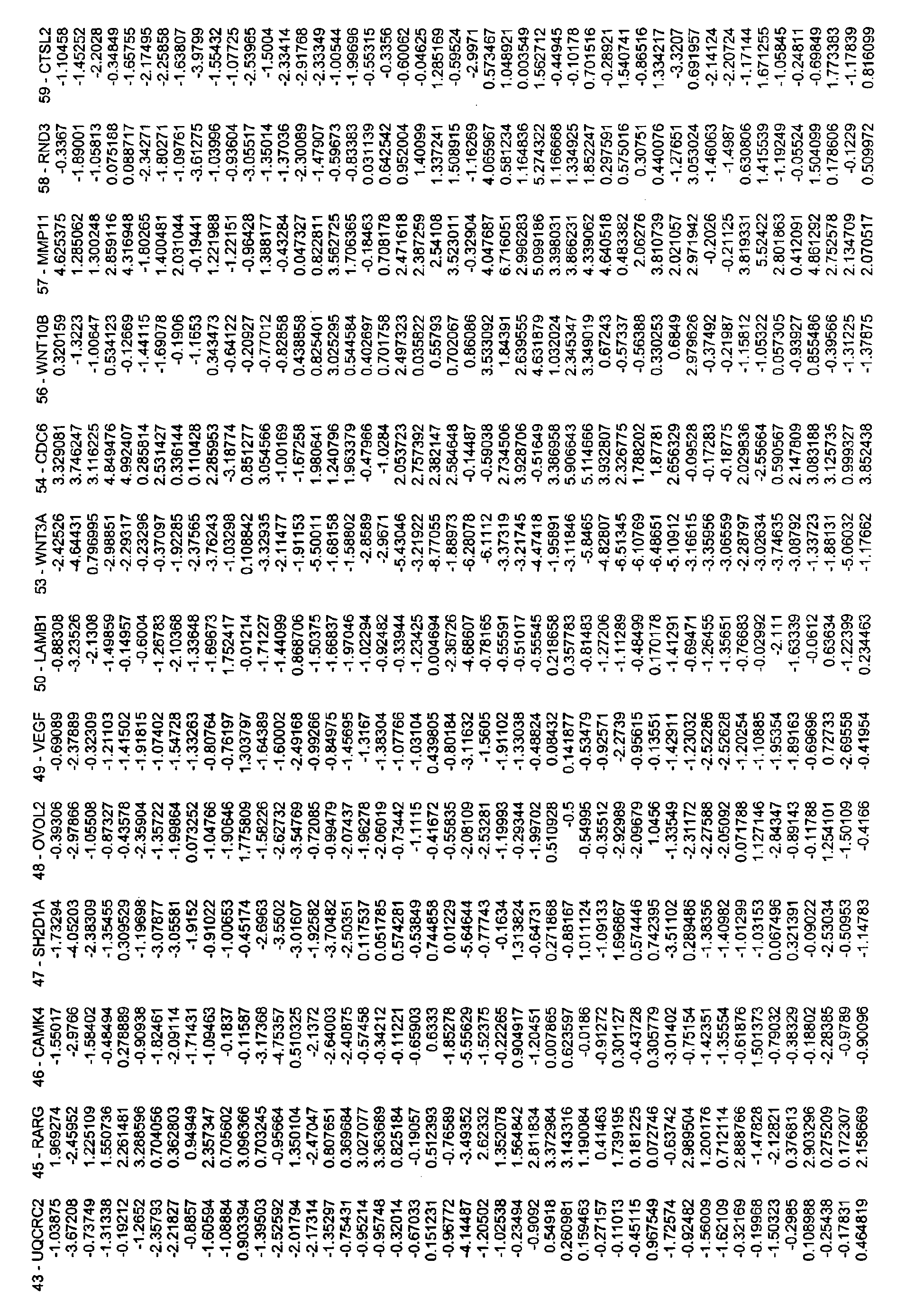

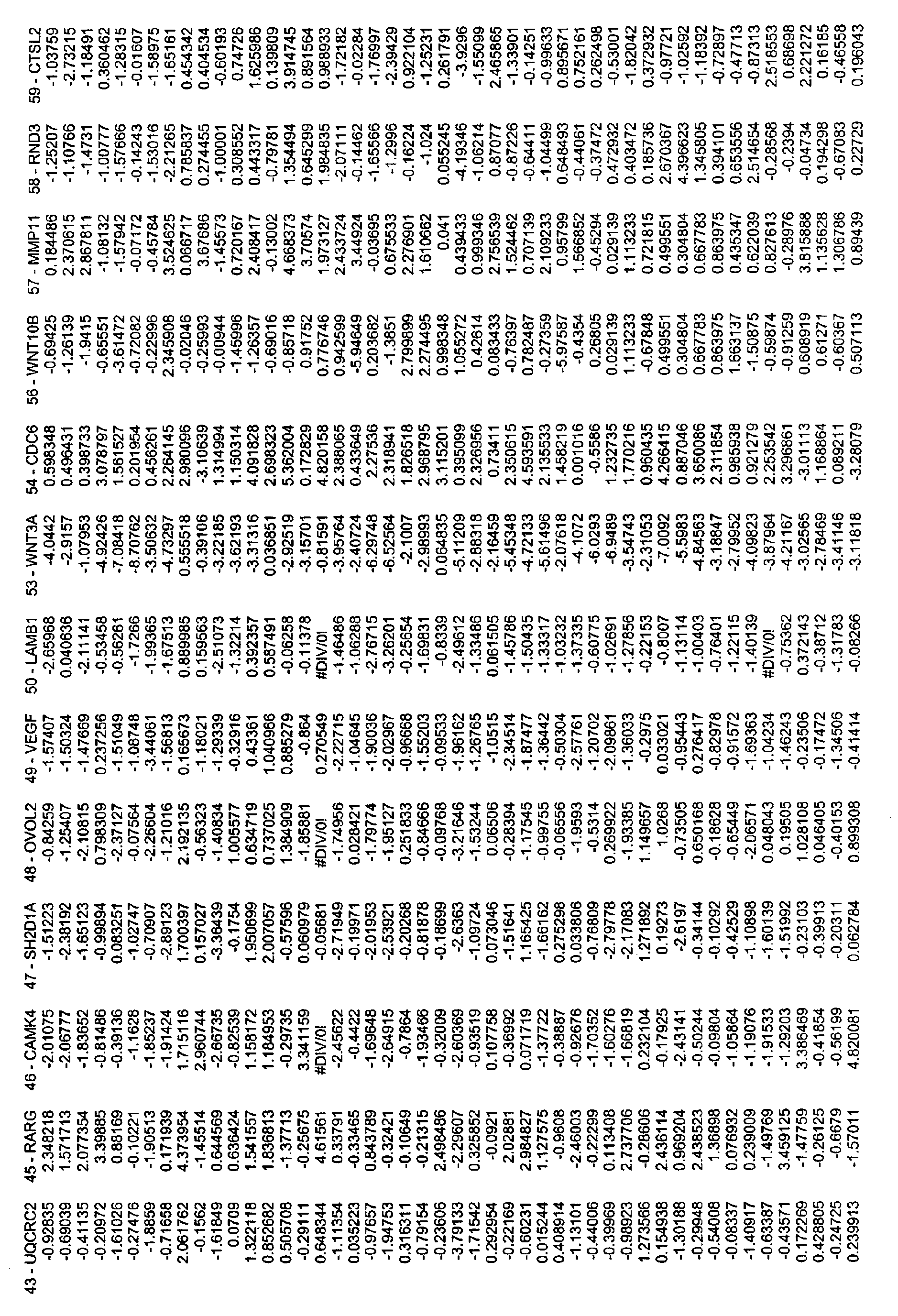

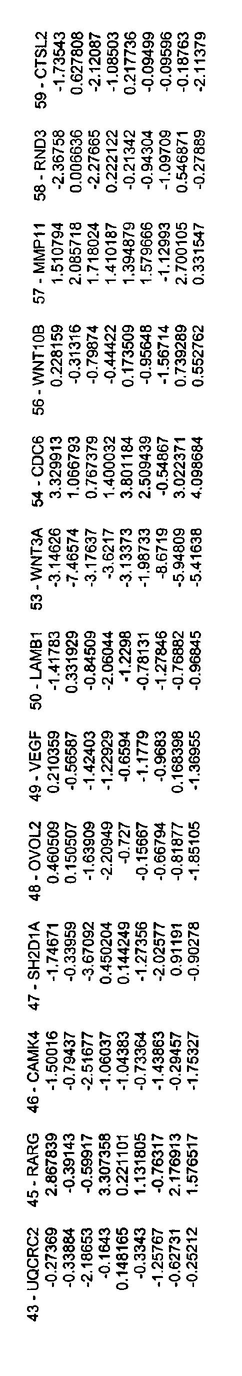

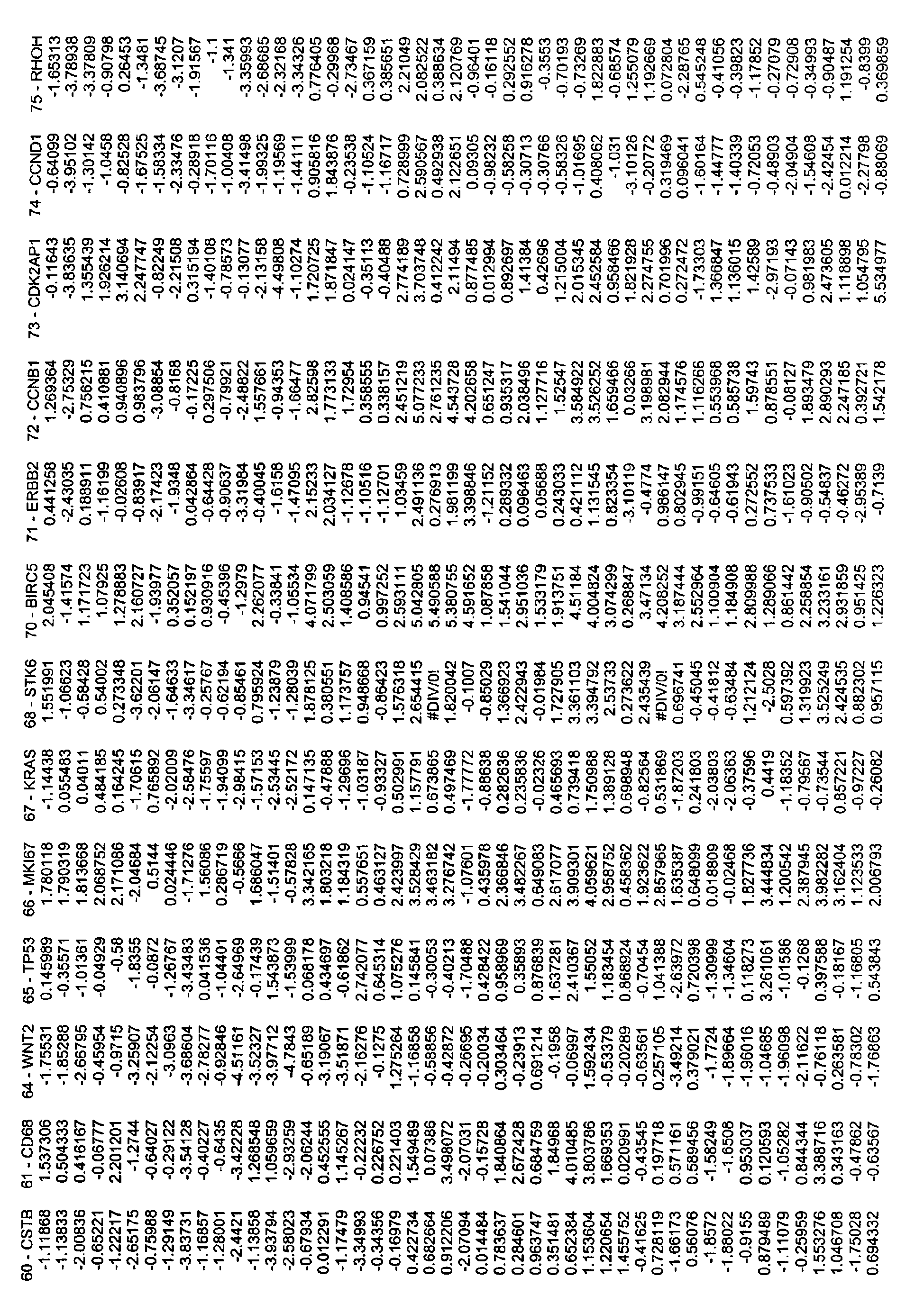

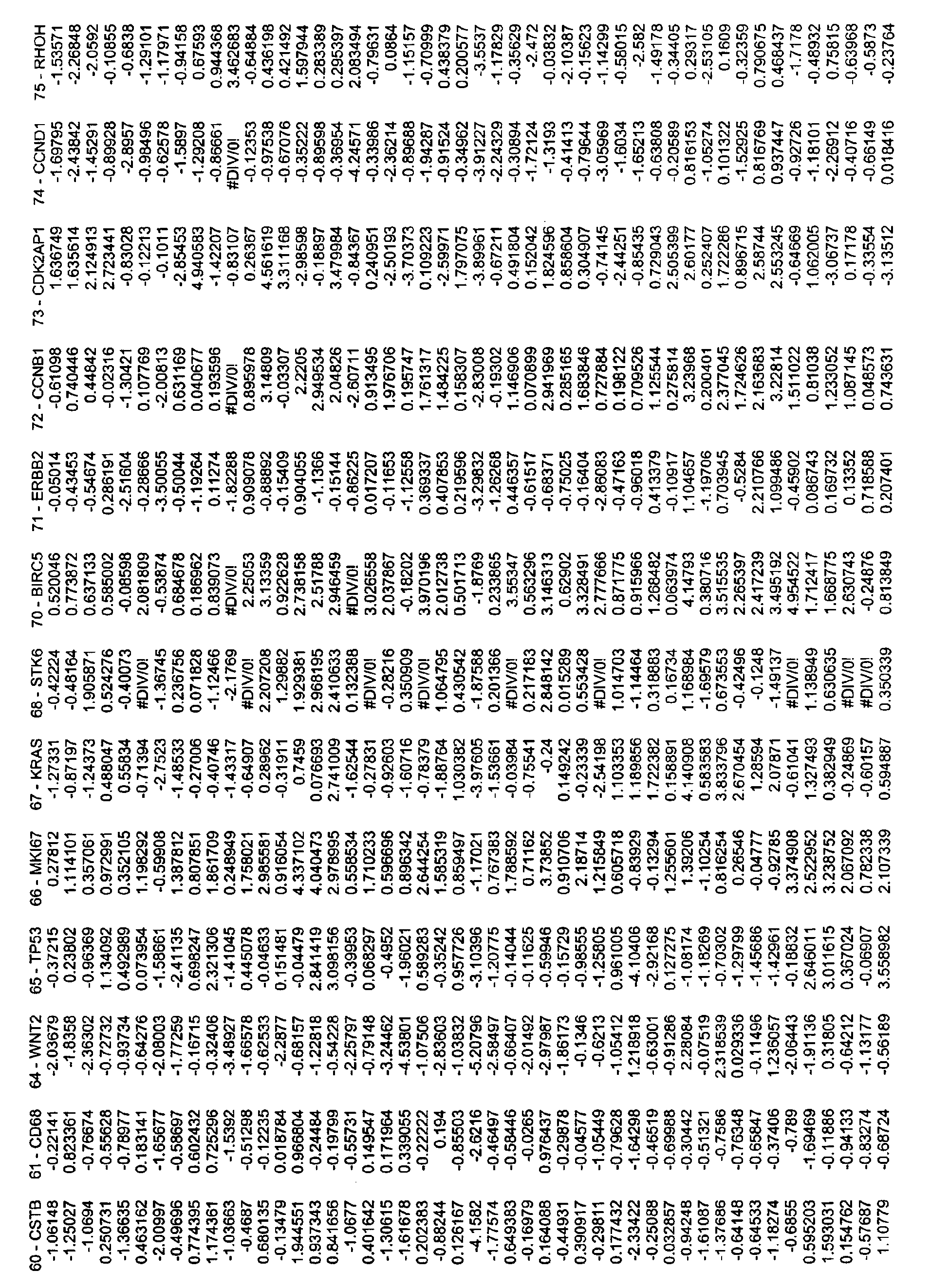

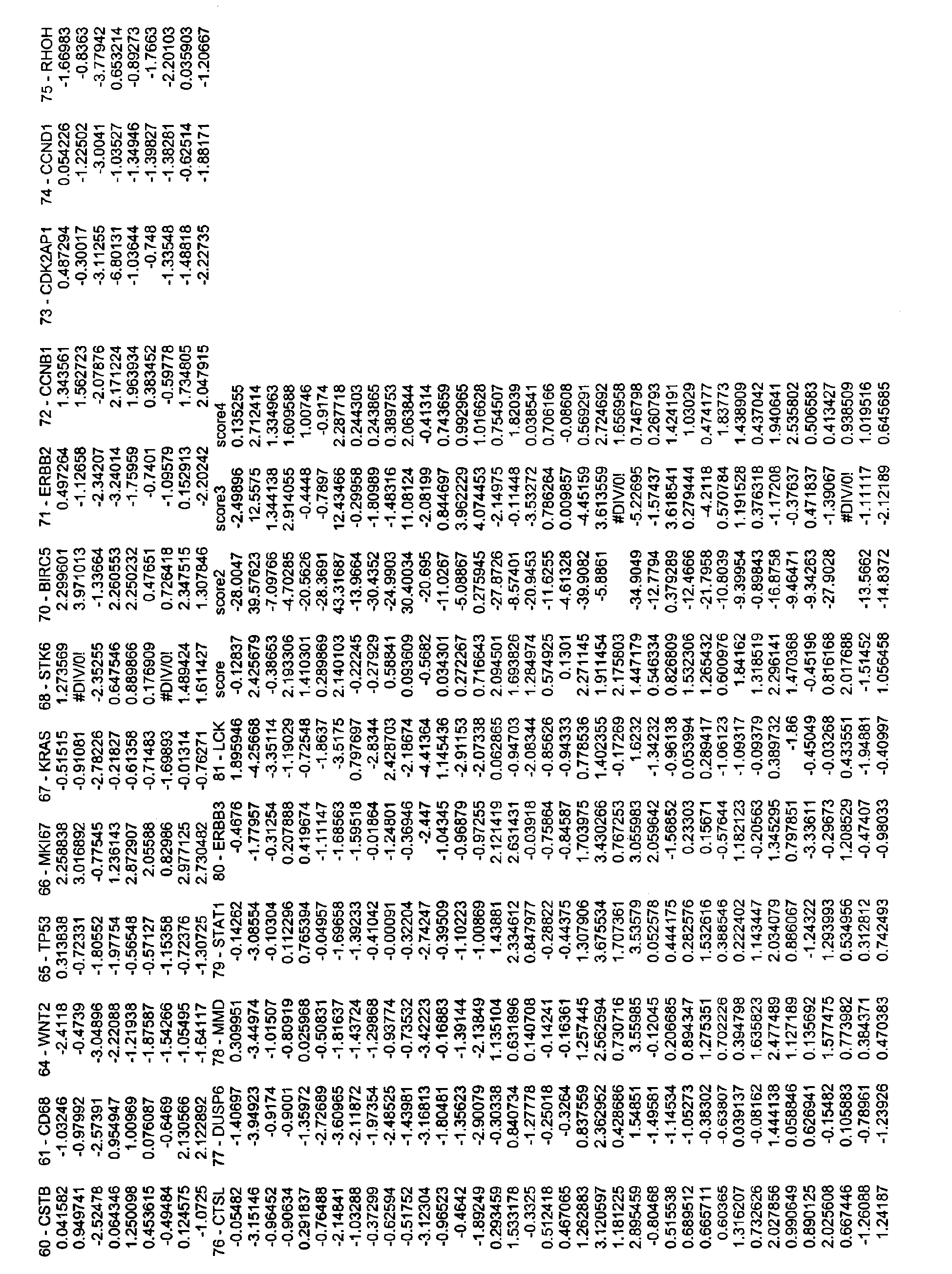

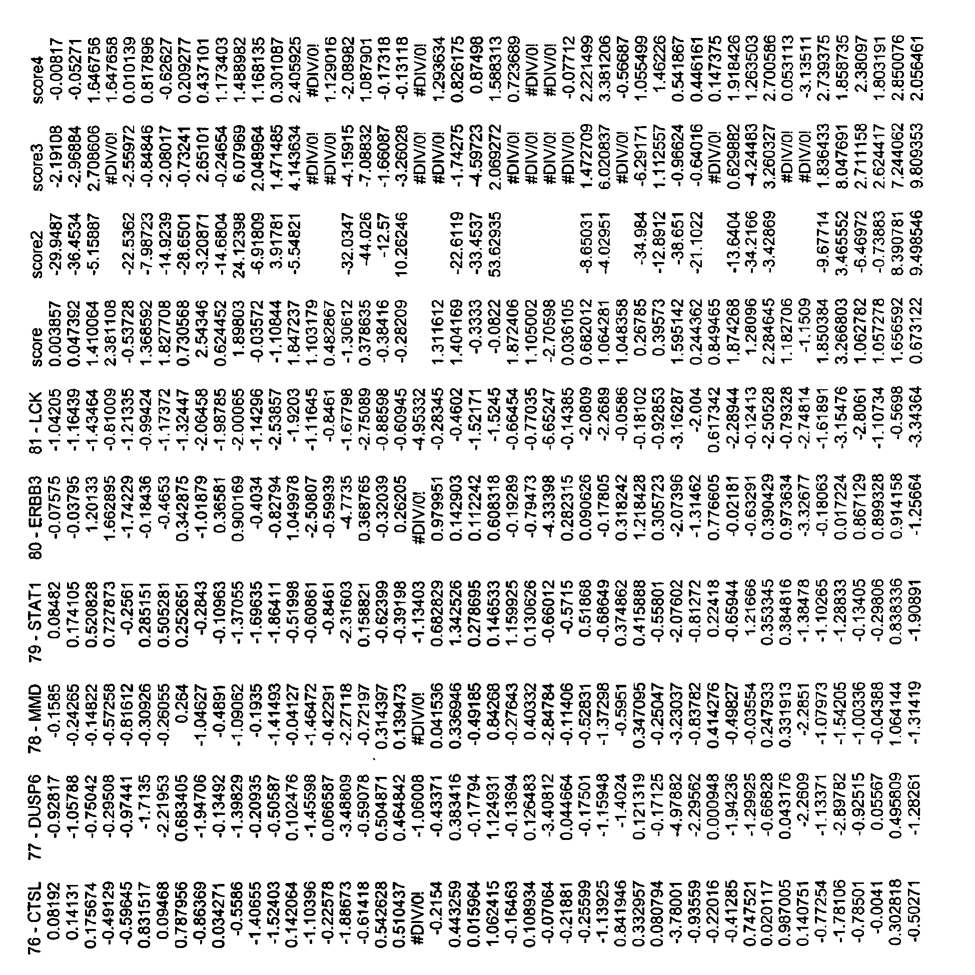

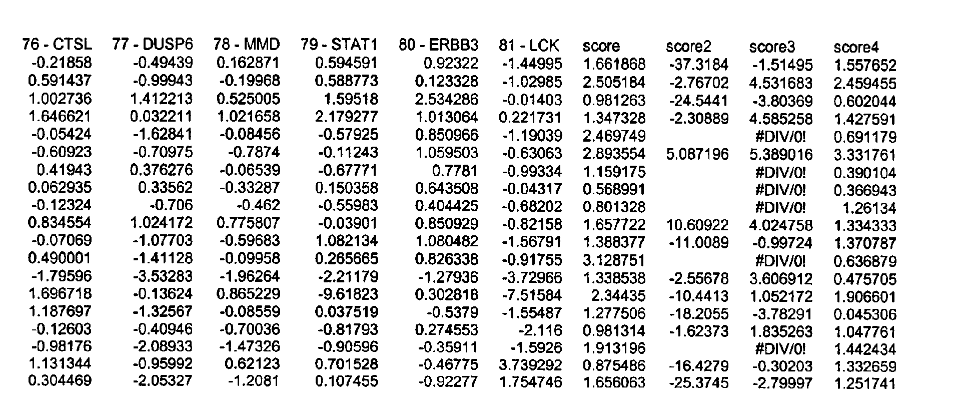

**Table 3: Patient characteristics, test group (n=107)**

| | | |
|---|---|---|
| Age, years (mean, SD) | | 68±11 |

| Sex (n,[%]) | | |
|---|---|---|
| | Women | 65 (61%) |
| | Men | 42 (39) |

| Smoking Status | | |
|---|---|---|
| | Current | 26 (25%) |
| | Past | 61 (57%) |
| | Never | 20 (19%) |

| Histology | | |
|---|---|---|
| | Pure BAC* | 14 (13%) |
| | Mixed Adenocarcinoma and BAC* | 29 (27%) |

| Race/Ethnicity | | |
|---|---|---|
| | Caucasian | 77 (72%) |
| | Asian | 19 (18%) |
| | African-American | 5 (5%) |
| | Hispanic | 2 (2%) |
| | Other | 4 (4%) |

| Tumor grade | | |
|---|---|---|
| | Well differentiated | 32 (30%) |
| | Moderately differentiated | 46 (43%) |
| | Poorly differentiated | 28 (26%) |
| Tumor size (mean in cm, SD) | | 3.5±2.0 |
| | | |

| Cancer Stage | | |
|---|---|---|
| | I | 70 (65%) |
| | II | 12 (11%) |
| | IIIa | 12 (11%) |
| | IIIb/IV | 13 (12%) |
| Followup time, months (median [range]) | | 61 (36-114) |
| Surviving patients | | |

| | | |
|---|---|---|
| *BAC= bronchioloalveolar carcinoma | | |

**Table 4: Adjusted Cox regression analysis¹**

| | HR | 95% Cl | p value |
|---|---|---|---|
| Risk score² | 6.72 | 1.56-28.91 | 0.01 |
| Stage³ | 1.32 | 1.05-1.65 | 0.02 |
| Tumor size | 1.04 | 0.91-1.19 | 0.56 |
| Age | 1.01 | 0.98-1.03 | 0.54 |

| | | | |
|---|---|---|---|
| ¹HR= Hazard ratio; Cl= confidence interval ²Risk score analyzed as a continuous variable ³HR for stage is stage I patients compared to all other patients | | | |

| **Gene Symbol** | **Gene Name** | **NCBI Gene Reference** |
|---|---|---|
| FZD7 | frizzled homolog 7 | NM_003507.1,AB017365.1,BC015915.1 |
| WIF1 | WNT inhibitory factor 1 | NM_007191.2,AF122922.1,BX647427.1,BC018037.1,AY358344.1 |
| FUT3 | fucosyltransferase 3 | NM_000149.1,U27326.1,U27327.1,U27328.1,AF131913.1 |
| NXF1 | nuclear RNA export factor 1 | NM_006362.3,AJ132712.1,U80073.1,AF112880.1,AF126246.1,AK 027192.1,BC004904.2,CR749674.1,AB209915.1,BC023041.1 |
| ASMTL | acetylserotonin O-methyltransferase-like | NM_004192.1,AK001090.1,BC010089.2,BC002508.2,AK090498.1 |
| CTNNB1 | catenin, beta 1, 88kDa | NM_001904.2,Z19054.1,X87838.1,A8062292.1,BC058926.1 |
| SH3BGR | SH3 domain binding glutamic acid-rich protein | NM_001001713.1,NM_007341.2,X93498.1,BC006371.2,BM474020.1 |
| ARAF | v-raf murine sarcoma 3611 viral oncogene homolog | NM_001654.1,X04790.1,BC007514.2,BC002466.2,BT019864.1,AK 130043.1 |
| TYROBP | TYRO protein tyrosine kinase binding protein | NM_198125.1,NM_003332.2,AF019562.1,AJ010098.1,BC011175.1 ,CR450342.1 ,CR542202.1,AY074782.1,BQ576278.1,BT009851.1 |
| LRP3 | low density lipoprotein receptor-related protein 3 | NM_002333.1,AB009462.1,BC00T408.2 |
| PIK3CG | phosphoinositide-3-kinase, catalytic, gamma polypeptide | NM_002649.2,X83368.1,AF327656.1,BX648341.1,BC035683.1 |
| BMP7 | bone morphogenetic protein 7 | NM_001719.1,M60316.1,X51801.1,BC008584.1,BC004248.1 |
| TP53I3 | tumor protein p53 inducible protein 3 | NM_147184.1,NM_004881.2,AF010309.1,BC000474.2,BC018819.2,BT007149.1,AK223382.1 |
| BAG1 | BOL2-associated athanogene | NM_004323.3,Z35491.1,AF022224.1,U46917.1,AF116273.1,BC00 1936.1,BC014774.1,AK096038.1,AK222749.1 |
| MINA | MYC induced nuclear antigen | NM_032778.3,AK027299.1,AY390536.1,CR627479.1,AB083190.1, AY302110.2,AY456380.1,BC014928.1,ABD83189.1 |
| BCL2 | B-cell CLL/lymphoma 2 | NM_000633.2,M13994.1,M14745.1,X06487.1,BC027258.1 |
| DNMT2 | DNA-methyltransferase 2 | NM_004412.3,NM_176081.1,NM_176083.1,NM_176084.1,NM_176 085.1,NM_176086.1,AJ223333.1,AF012128.1,AF045888.1,AF3299 44.1,AF329940.1,AF329941.1,AF329942.1,AF329943.1,CR450316.1,BX537961.1,BC047733.1 |
| SCUBE2 | signal peptide, CUB domain, EGF-like 2 | NM_020974.1,AK131552.1,AK123039.1 |
| PRKCA | protein kinase C. alpha | NM_002737.2,X52479.1,AB209475.1 |
| IL11 | interleukin 11 | NM_000641.2,X58377.1,M57765.1,BC012506.1 |
| GLI1 | glioma-associated oncogene homolog 1 | NM_005269.1,X07384.1,BC013000.2 |
| GLI2 | GLI-Kruppel family member GLI2 | NM_030379.1,NM_030380.1,NM_030381.1,NM_005270.2,AB0072 95.1,AB007296.1,AB007297.1,AB007298.1,AB209354.1,DQ08681 4.1 |
| BCL7A | B-cell CLL/lymphoma 7A | NM_001024808.1,NM_020993.3,X89984.1,BC094723.1 |
| MUC1 | mucin 1, transmembrane | NM_001018016.1,NM_001018017 1,NM_001018021.1,NM_00245 6.4,M34088.1,M34089.1,J05581.1,X807611.1,X52229.1,U60259.1,U 60260.1,AF125525.1,AF348143.1,AY327582.1,AY327584.1,AY327 585.1,AY327586.1,AY327587.1,AY3 |
| NRP2 | neuropilin 2 | NM_018534.3,NM_201264.1,NM_201266.1,NM_201267.1,NM_201 279.1,NM_003872.2,AF016098.1,AF022859.1,AF022860.1,BC009 222.2,AF280544.1,AF280545.1,AF280546.1,BX537423.1,AK13019 8.1,AL833606.1 |
| | | NM 001008396.1,NM007086.2,AJ006266.1,AK001538.1,AK0015 |

## Claims

1. A method of providing a prognosis for lung cancer in a subject, the method comprising the steps of:
(a) contacting a biological sample from the subject with reagents that each specifically bind to one member of a panel of mRNA biomarkers comprising the eight biomarkers ctnnb1, wnt3a, tp53, kras, erbb3, mucl, erbb2, and dusp6, and
(b) calculating a model score for said subject using the expression levels of the eight biomarkers and model coefficients, thereby identifying said subject as high-risk or low-risk based on the model score.

2. The method of claim 1, wherein:
(a) the reagent is a nucleic acid;
(b) the reagent is an oligonucleotide; or
(c) the reagent is a PCR primer set.

3. The method of claim 1, wherein the lung cancer is lung adenocarcinoma.

4. The method of claim 3, wherein:
(a) the lung adenocarcinoma is stage I;
(b) the lung adenocarcinoma is stage II;
(c) the lung adenocarcinoma is stage III; or
(d) the lung adenocarcinoma is stage IV.

5. The method of claim 1, wherein:
(a) the sample is from a surgically resected tumor; or
(b) the sample is from lung tissue or a lung tumor biopsy.

6. The method of claim 1, wherein the prognosis provides a high or low risk assessment for long term mortality.

7. A method of providing a prognosis of a subject having a lung cancer, the method comprising quantifying in a biological sample derived from said subject the mRNA expression levels of at least four biomarkers comprising rnd3, wnt3a, erbb3, and lck; and calculating a model score for said subject using said expression levels and model coefficients, thereby identifying said subject as high-risk or low-risk based on the model score.

8. The method of claim 7, wherein said quantifying comprises quantitative rtPCR.

9. The method of claim 7 or 8, wherein said biological sample is:
(a) a blood sample;
(b) a tumor biopsy; or
(c) a lung biopsy.

10. Use of quantification of expression levels of mRNAs encoding rnd3, wnt3a, erbb3, and lck as a prognostic indicator of mortality due to lung cancer.

11. Use of quantification of expression levels of mRNAs encoding ctnnbl, wnt3a, tp53, kras, erbb3, mucl, erbb2, and dusp6 as a prognostic indicator of mortality due to lung cancer.

## Patentansprüche

1. Verfahren zur Bereitstellung einer Prognose für Lungenkrebs bei einem Subjekt, wobei das Verfahren die Schritte:
(a) Inkontaktbringen einer biologischen Probe von dem Subjekt mit Reagentien, von denen jedes spezifisch an ein Mitglied einer Gruppe von mRNA-Biomarkern bindet, welche die acht Biomarker ctnnbl, wnt3a, tp53, kras, erbb3, mucl, erbb2 und dusp6 umfasst, und
(b) Errechnen eines Modell-Scores für das Subjekt unter Verwendung der Expressionslevel der acht Biomarker und von Modellkoeffizienten, dadurch Identifizieren auf der Basis des Modell-Scores, das das Subjekt ein hohes Risiko oder ein niedriges Risiko hat, umfasst.

2. Verfahren nach Anspruch 1, wobei
(a) das Reagens eine Nucleinsäure ist;
(b) das Reagens ein Oligonucleotid ist oder
(c) das Reagens ein PCR-Primer-Satz ist.

3. Verfahren nach Anspruch 1, wobei der Lungenkrebs Lungenadenokarzinom ist.

4. Verfahren nach Anspruch 3, wobei:
(a) das Lungenadenokarzinom Stadium I ist;
(b) das Lungenadenokarzinom Stadium II ist;
(c) das Lungenadenokarzinom Stadium III ist oder
(d) das Lungenadenokarzinom Stadium IV ist.

5. Verfahren nach Anspruch 1, wobei:
(a) die Probe von einem chirurgisch resezierten Tumor ist oder
(b) die Probe von Lungengewebe oder eine Lungentumorbiopsie ist.

6. Verfahren nach Anspruch 1, wobei die Prognose eine Beurteilung hohes oder niedriges Risiko für Langzeitmortalität bereitstellt.

7. Verfahren zur Bereitstellung einer Prognose eines Subjektes, das einen Lungenkrebs hat, wobei das Verfahren Quantifizieren in einer biologischen Probe, die von dem Subjekt stammt, die mRNA-Expressionslevel von wenigstens vier Biomarkem, die rnd3, wnt3a, erbb3 und lck umfassen, und Errechnen eines Modell-Scores für das Subjekt unter Verwendung der Expressionslevel und von Modellkoeffizienten, dadurch Identifizieren auf der Basis des Modell-Scores, dass das Subjekt ein hohes Risiko oder niedriges Risiko hat.

8. Verfahren nach Anspruch 7, wobei das Quantifizieren quantitative rtPCR umfasst.

9. Verfahren nach Anspruch 7 oder 8, wobei die biologische Probe ist:
(a) eine Blutprobe;
(b) eine Tumorbiopsie oder
(c) eine Lungenbiopsie.

10. Verwendung einer Quantifizierung von Expressionsleveln von mRNAs, die md3, wnt3a, erbb3 und lck codieren, als prognostischer Indikator für Mortalität infolge von Lungenkrebs.

11. Verwendung einer Quantifizierung von Expressionsleveln von mRNAs, die ctnnb1, wnt3a, tp53, kras, erbb3, mucl, erbb2 und dusp6 codieren, als prognostischer Indikator für Mortalität infolge von Lungenkrebs.

## Revendications

1. Procédé permettant de fournir un pronostic pour un cancer du poumon chez un sujet, lequel procédé comporte les étapes suivantes :
a) mettre un échantillon biologique provenant du sujet en contact avec des réactifs dont chacun se lie spécifiquement à un élément d'un ensemble de biomarqueurs d'ARNm comprenant les huit biomarqueurs ctnnbl, wnt3a, tp53, kras, erbb3, mucl, erbb2 et dusp6,
b) et calculer un score selon modèle pour ledit sujet, en utilisant les niveaux d'expression des huit bio-marqueurs et des coefficients de modèle, ce qui permet, en se basant sur ce score selon modèle, d'identifier le sujet comme étant à haut risque ou à faible risque.

2. Procédé conforme à la revendication 1, dans lequel
a) le réactif est un acide nucléique,
b) le réactif est un oligonucléotide,
c) ou le réactif est un jeu d'amorces pour PCR.

3. Procédé conforme à la revendication 1, dans lequel le cancer du poumon est un adénocarcinome pulmonaire.

4. Procédé conforme à la revendication 3, dans lequel
a) l'adénocarcinome pulmonaire est de stade I,
b) l'adénocarcinome pulmonaire est de stade II,
c) l'adénocarcinome pulmonaire est de stade III,
d) ou l'adénocarcinome pulmonaire est de stade IV.

5. Procédé conforme à la revendication 1, dans lequel
a) l'échantillon provient d'une tumeur chirurgicalement réséquée,
b) ou l'échantillon provient du tissu pulmonaire ou d'une biopsie de tumeur pulmonaire.

6. Procédé conforme à la revendication 1, dans lequel le pronostic fournit une évaluation du risque, haut ou faible, de mortalité à long terme.

7. Procédé permettant de fournir un pronostic pour un cancer du poumon chez un sujet, lequel procédé comporte le fait de déterminer quantitativement, dans un échantillon biologique provenant du sujet, les niveaux d'expression des ARNm d'au moins quatre biomarqueurs comprenant les biomarqueurs rnd3, wnt3a, erbb3 et lck, et le fait de calculer un score selon modèle pour ledit sujet, en utilisant lesdits niveaux d'expression et des coefficients de modèle, ce qui permet, en se basant sur ce score selon modèle, d'identifier le sujet comme étant à haut risque ou à faible risque.

8. Procédé conforme à la revendication 7, dans lequel ladite opération de détermination quantitative comprend une opération de PCR quantitative en temps réel.

9. Procédé conforme à la revendication 7 ou 8, dans lequel ledit échantillon biologique est
a) un échantillon de sang,
b) une biopsie de tumeur,
c) ou une biopsie de poumon.

10. Utilisation de la détermination quantitative des niveaux d'expression des ARNm qui codent rnd3, wnt3a, erbb3 et lck en tant qu'indicateur de pronostic de mortalité due à un cancer du poumon.

11. Utilisation de la détermination quantitative des niveaux d'expression des ARNm qui codent ctnnbl, wnt3a, tp53, kras, erbb3, mucl, erbb2 et dusp6 en tant qu'indicateur de pronostic de mortalité due à un cancer du poumon.
